# EUROPEAN PATENT SPECIFICATION

(11) **EP 1 652 923 B1**
(45) Date of publication and mention of the grant of the patent: **12.10.2011**
(21) Application number: 04771625.3
(22) Date of filing: 06.08.2004
(51) Int. Cl.: C12N 15/12, C12Q 1/68, C07K 14/82, A61K 31/711, C07K 16/32, C12N 5/10, G01N 33/15, G01N 33/50

(54) **GENE OVEREXPRESSED IN CANCER**
BEI KREBS ÜBEREXPRIMIERTES GEN
GENE SUREXPRIME DANS LE CANCER

(30) Priority: 08.08.2003 JP 2003290704
(43) Date of publication of application: 03.05.2006
(60) Divisional application: 10178204.3
(73) Proprietor: Perseus Proteomics Inc., Tokyo 153-0041 (JP); CHUGAI SEIYAKU KABUSHIKI KAISHA, Tokyo, 115-8543 (JP); Aburatani, Hiroyuki, Musashino-shi, Tokyo 180-0003 (JP)
(72) Inventor: ABURATANI, Hiroyuki, Musashino-shi, Tokyo 180-0003 (JP); HIPPO, Yoshitaka, Komae-shi, Tokyo 201-004 (JP); TANIGUCHI, Hirokazu, Tokyo 154-0003 (JP); CHEN, Yong xin, Tokyo 157-0067 (JP); ISHIKAWA, Shumpei, Tokyo 113-0032 (JP); FUKUMOTO, Shin-ichi, Tokyo 155-0031 (JP); SHIMAMURA, Takahiro, Tokyo 151-0072 (JP); KAMIMURA, Naoko, Tokyo 171-0021 (JP); GUO, Ying qiu, Tokyo 157-0067 (JP); YAMAMOTO, Shogo, Tokyo 140-0015 (JP); ITO, Yukio, c/o Perseus Proteomics Inc., Tokyo 153-0041 (JP); ITO, Hirotaka, c/o Chugai Seiyaku K. K., Gotenba-shi, Shizuoka 412-8513 (JP); OHTOMO, Toshihiko, c/o Chugai Seiyaku K. K., Gotenba-shi, Shizuoka 412-8513 (JP)
(74) Representative: Vossius & Partner
(86) International application number: PCT/JP2004/011650
(87) International publication number: WO 2005/014818

(56) References cited:
- EP-A- 1 293 569
- WO-A-02/26982
- WO-A-2004/087874
- WO-A2-02/46415
- WO-A2-03/029424
- WO-A2-2004/040000
- US-A1- 2003 003 538

## Description

### Technical Field

The present invention relates to an in vitro method for diagnosing cancer making use of a cancer-associated gene, a protein encoded by the gene, and an antibody recognizing this protein.

### Background Art

Heretofore, a number of genes have been found whose expression level changes as being associated with malignant transformation of cells and antigens that can be used as a cancer marker, and numerous studies on such genes have been carried out. However, it is still difficult to specifically detest or treat a specific cancer. Therefore, in this technical field, identification of another cancer-associated gene or protein that can be used in diagnosis and treatment of cancer has been demanded.

There are prior art documents related to the present invention: EP 1033401; US 2002022248; US 2002042096; US 200208150; US 6337195; US 6362321; WO 9738098; WO 9920764; WO 9929729; WO 0006698; WO 0012702; WO 0034477; WO 0036107; WO 0037643; WO-0055174; WO 0055320; WO 0055351; WO 0055633; WO 0058473; WO 0073509; WO 0100828; WO 0109317; WO 0121653; WO 0122920; WO 0151513; WO 0151628; WO 0154733; WO 0155355; WO 0157058; WO 0159111; WO 0160860; WO 0164835; WO 0164886; WO 0166719; WO 0170976; WO 0173027; WO 0175177; WO 0177168; WO 0192578; WO 0194629; WO 0200677; WO 0200889; WO 0200939; WO 0204514; WO 0210217; WO 0212280; WO 0220598; WO 0229086; WO 0229103; WO 0258534: WO 0260317; WO 0264797 and WO 0226982. WO 0226982 discloses sequences concerning human secreted proteins (SECP). An object of the present invention is to provide a gene and a protein that can be used as an agent for diagnosing and treating cancer.

### Disclosure of the invention

The present inventors have found that the expression of specific genes is elevated in a cancer tissue, and have completed the present invention. The present invention provides an in vitro method of diagnosing cancer as defined in the claims. Such a method comprises the step of detecting in a sample collected from a subject a protein having the amino acid sequence as set forth in SEQ ID NO: 66 contained in the sample.

The present patent describes a protein having the amino acid sequence represented by SEQ ID NO. : 66. Such a protein or a fragment thereof is useful as an antigen for producing an antibody or is useful in the method according to the present invention for diagnosing cancer.

Also described is an antibody recognizing the above-mentioned protein or a fragment thereof or an antigen-binding fragment thereof. The patent also describes a cell producing such an antibody.

In still another aspect, described is a polynucleotide having a nucleotide sequence represented by

SEQ ID NO: or a nucleotide sequence complementary thereto, and a polynucleotide that can hybridize under high stringent conditions to any of these polynucleotides.

Further described is a polynucleotide having a sequence comprising at least 12 consecutive nucleotides of a nucleotide sequence represented by SEQ ID NO: 2 ; or a nucleotide sequence complementary thereto, and an oligonucleotide with a length of at least 12 nucleotides that can hybridize under high stringent conditions to a polynucleotide having a nucleotide sequence represented by SEQ ID NO: 2.

Such a polynucleotide is useful for diagnosis of cancer, production of a protein, and as a primer, an antisense and siRNA for inhibiting gene expression.

In still another aspect, the present invention provides a method of diagnosing cancer by detecting C20orf102 protein as represented by SEQ ID NO: 66.

It is preferred that the cancer is lung cancer, liver cancer or pancreatic cancer. In the method of the present invention, it is preferred that C20orf102 protein secreted outside a cell is detected. In addition, it is preferred that the method of the present invention is carried out by using an antibody recognizing C20orf102 protein. Preferable, in the method of the present invention, C20orf102 protein in blood, serum or plasma or C20orf102 protein secreted from a cell is detected.

In another aspect, the present invention provides a method of diagnosing cancer comprising the steps of:
(a) collecting a sample from a subject; and
(b) detecting C20orf102 protein contained in the collected sample.

### Brief Description of the Drawings

Fig. 1 shows the results of an expression analysis of a cancer-associated gene TEG1.
Fig. 73 shows the expression of C20orf102 gene in lung squamous cell carcinoma.
Fig. 74 shows the detection of a C20orf102 protein molecule in a variety of cancer cell lines and a culture supernatant thereof using an anti-C20orf102 antibody.
Fig. 75 shows the results of an expression analysis of C20orf102 protein in lung adenocarcinoma tissue using an anti-C20orf102 antibody.

### Detailed Description of the Intention

The present invention provides an in vitro method for diagnosing cancer utilizing a specific gene whose expression is elevated in a cancer tissue and a protein encoded by the gene.

### Protein

The patent describes a protein encoded by a cancer-associated gene represented by SEQ ID NO: 2 or a fragment thereof. The corresponding encoded protein is a protein having the amino acid sequence represented by SEQ ID NO : 66

This protein or the fragment thereof is useful for diagnosing cancer or as an antigen for producing an antibody. The protein or the fragment thereof may be a variant in which one or more amino acid residues are substituted, added or deleted from the above-mentioned sequence, as long as it has a desired immunogenicity. It is preferred that such a variant has an amino acid sequence with an identity of at least 80%, preferably 90% or more, more preferably 95% or more with the above-mentioned amino acid sequence.

The identity of amino acid sequence is calculated by dividing the number of identical residues by the total number of residues in two sequences to be compared and multiplying by 100. Several computer programs for determining the identity of sequences using standard parameters are available, for example, Gapped BLAST or PSI-BLAST (Altschul, et al. (1997) Nucleic Acids Res. 25: 3389-3402), BLAST (Altschul, et al. (1990) J. Mol. Biol. 215 : 403-410) and Smith-Waterman (Smith, et al. (1981) J. Mol. Biol. 147: 195-197).

It has been already known that a protein having an amino acid sequence which has been modified by deletion, addition and/or substitution with another amino acid of one or more amino acid residues in a certain amino acid sequence retains the original biological activity (Mark, D. F. et al., Proc. Natl. Acad. Sci. USA (1984) 81, 5662-5666, Zoller, M. J. & Smith, M. Nucleic Acids Research (1982) 10, 6487-6500, Wang, A. et al., Science 224, 1431-1433, Dalbadie-McFarland, G. et al., Proc. Natl. Acad. Sci. USA (1982) 79, 6409-6413).

It is preferred that an amino acid residue to be mutated is replaced with another amino acid residue in which the classes of the property of the amino acid side chain is conserved. Examples of the property of an amino acid side chain include a hydrophobic amino acid (A, I, L, M, F, P, W, Y, V), a hydrophilic amino acid (R, D, N, C, E, Q, G, H, K, S, T), an amino acid having an aliphatic side chain (G, A, V, L, I, P), an amino acid having a hydroxyl group-containing side chain (S, T, Y), an amino acid having a sulfur atom-containing side chain (C, M), an amino acid having a carboxylic acid and amide-containing side chain (D, N, E, Q), an amino acid having a base-containing side chain (R, K, H), and an amino acid having an aromatic containing side chain (H, F, Y, W). The parenthetic letters indicate the one-letter codes of amino acids.

A protein that is functionally equivalent to a given protein can be prepared by a method known to those skilled in the art, for example, by introducing an appropriate mutation into the amino acid using a site-directed mutagenesis method (Gotoh, T. et al. (1995), Gene 152, 271-275; Zoller, M J. and Smith, M. (1983), Methods Enzymol. 100, 468-500; Kramer, W. et al. (1984), Nucleic Acids Res. 12, 9441-9456, Kramer W, and Fritz H J (1987) Methods. Enzymol. 154, 350-367, Kunkel, T A (1985), Proc. Natl. Acad. Sci. USA. 82, 488-492, Kunkel (1988), Methods Enzymol. 85, 2763-2766).

The protein can vary in its amino acid sequence, molecular weight, isoelectric point, and presence or absence of a sugar chain or the form of a sugar chain depending on a cell or a host used for producing the protein or a purification method as described latter. For example, when the protein is expressed in a prokaryotic cell such as E. coli, a methionine residue is added at the N-terminus of the amino acid sequence of the original protein.

The described protein can be prepared as a recombinant protein or as a naturally occurring protein by a method known to those skilled in the art. In the case of a recombinant protein, it can be prepared as follows. DNA encoding the protein is introduced into an appropriate expression vector, and the vector is introduced into an appropriate host cell. Then, the resulting transformant is collected, and an extract is obtained. Then the protein is purified by chromatography such as ion exchange, reverse phase, or gel filtration chromatography, or by affinity chromatography with a column on which antibodies against the protein are immobilized, or combination of one or more of these columns.

Further, when the protein is expressed in a host cell (such as an animal cell or E. coli), as a fusion protein with glutathione S-transferase protein or as a recombinant protein supplemented with multiple histidine residues, the expressed recombinant protein can be purified using a glutathione column or a nickel column, respectively. After purifying the fusion protein, it is also possible to remove regions other than the intended protein from the fusion protein by cutting with thrombin, factor-Xa or the like as needed.

In the case of a natural protein, it can be isolated by a method well known to those skilled in the art, for example, by purifying an extract of tissue or cells expressing the protein of the present invention with an affinity column having antibodies that binds to the protein which will be described later. The antibody may be a polyclonal antibody or a monoclonal antibody.

Also a fragment of the protein can be used in producing an antibody against the protein or screening a compound binding to the protein.

When the fragment is used as an immunogen, it is composed of at least 7 amino acids or more, preferably 8 amino acids or more, and more preferably 9 amino acids or more.

Such a fragment can be produced by a genetic engineering technique, a well-known peptide synthesizing method, or by cleaving the described protein, with a' suitable peptidase. The peptide synthesis may be carried out by, for example, a solid phase synthesis method or a liquid phase synthesis method.

Moreover, described is a vector into which the DNA as described herein is inserted. The vector may be useful for maintaining the DNA in a host cell or expressing the protein.

When E. coli is used as a host, it is preferred that the vector has an "ori" to be amplified in E. coli and a gene for selecting the transformed E. coli (e.g., a drug resistance gene that can be selected by a drug such as ampicillin, tetracycline, kanamycin, or chloramphenicol). in order to amplify and prepare the vector in a large amount in E. coli (such as JM109, DHSα, HB101 or XL1Blue).

Examples of the vector include M13-series vectors, pUC-series vectors, pBR322, pBluescript, pCR-Script and the like. In addition, when it is intended to be used for subcloning and excision of the cDNA, pGEM-T, pDIRECT, pT7 and the like may also be used in addition to the above-mentioned vectors.

When the vector is used to produce the protein described herein, an expression vector is especially useful. When the expression vector is intended to be expressed in E. coli, the vector should have the above-mentioned characteristic for amplification of the vector in E. coli. Additionally, when E. coli such as JM109, DH5α, HB101 or XL1-Blue is used as a host cell, it is essential that the vector should have a promoter that can efficiently drive the expression in E. coli, for example, lacZ promoter (Ward et al., Nature (1989) 341: 544-546; FASEB J. (1992) 6: 2422-2427), araB promoter (Better et al., Science (1988) 240: 1041-1043), or T7 promoter, Examples of such a vector include other than the above-mentioned vectors, pGEX-5X-1 (manufactured by Pharmacia), "QIAexpress system" (manufactured by QIAGEN), pEGFP, pET (for this vector, BL21 expressing T7 RNA polymerase is preferably used as a host) and the like.

Further, the vector may contain a signal sequence for protein secretion. When the protein is secreted into the periplasm of E. coli, pelB signal sequence (Lei S.P. et al., J. Bacteriol. (1987) 169, 4379) may be used as a signal sequence for protein secretion. The introduction of the vector into a host cell can be carried out by, for example, a calcium chloride method or an electroporation method.

Examples of the vector used to produce the protein described herein include expression vectors other than those from E. coli, for example, expression vectors derived from mammals (e.g., pcDNA3 (manufactured by Invitrogen), pEGF-BOS (Nucleic Acids. Res. 1990, 18 (17), p5322), pEF, and pCDM8); expression vectors derived from insect cells (e.g., "Bac-to-BAC baculovirus expression system" (manufactured by GIBCO BRL), pBacPAK8): expression vectors derived from plants (e.g., pMH1 and pMH2) ; expression vectors derived from animal viruses (e.g., pHSV, pMV, and pAdexLcw); expression vectors derived from retroviruses (e.g., pZIPneo); expression vectors derived from yeast (e.g., "Pichia Expression Kit" (manufactured by Invitrogen), pNV11 and SP-Q01); and expression vectors derived from Bacillus subtilis (e.g., pPL608 and pKTH50).

When it is intended to express the protein in an animal cell, such as a CHO cell, COS cell, or NIH3T3 cell, it is essential that the vector should include a promoter necessary for expression in such a cell, for example, SV40 promoter (Mulligan et al., Nature (1979) 271, 108), MMLV-LTR promoter, the EF1α promoter (Mizushima et al., Nucleic Acids Res. (1990) 18, 5322), and CMV promoter. It is more preferred that the vector additionally has a gene for selecting the transformed cell (e.g., a drug resistance gene that allows for selection with a drug such as neomycin or G418). Examples of the vector having such a characteristic include pMAM, pDR2, pBK-RSV, pBK-CMV, pOPRSV, pOP13 and the like.

Further, when it is intended to stably express the gene and to amplify the copy number of genes in a cell, a vector (such as pCHOI) having a DHFR gene that compensates for the deficiency may be introduced into a CHO cell deficient in nucleic acid synthetic pathways, and amplified with methotrexate (MTX). Further, when it is intended to transiently express the gene, a COS cell having a gene expressing SV40 T antigen on the chromosome may be transformed with a vector (such as pCD) having SV40 replication origin. The replication origin may include those derived from polyoma virus, adenovirus, bovine papillomavirus (BPV) and the like. Further, in order to amplify the gene copy number in a host cell system, the expression vector can include as a selection marker, the aminoglycoside transferase (APH) gene, the thymidine kinase (TK) gene, the E. coli xanthine guanine phosphoribosyl transferase (Ecogpt) gene, the dihydrofolate reductase (dhfr) gene or the like.

Further described is a host cell into which the described vector is introduced. The host cell is not particularly limited but may include, for example, E. coli, various animal cells and the like. The host cell may be used, for example, as a production system for producing and expressing the protein described herein. There are in vitro and in vivo production systems available for producing the protein. Examples of the in vitro production system include a production system using a eukaryotic cell and a production system using a prokaryotic cell.

When the eukaryotic cell is used, for example, an animal cell, a plant cell or a fungal cell can be used as a host. The animal cells known in the art include mammalian cells, such as CHO (J. Exp. Med. (1995) 108, 945), COS, 3T3. myeloma, baby hamster kidney (BHK), HeLa and-Vero; amphibian cells such as Xenopus laevis oocytes (Valle, et al., Nature (1981) 291, 338-340); and insect cells such as sf9, sf21 and Tn5. CHO cells preferably used include those deficient in the DHFR gene such as dhfr-CHO (Proc. Natl. Acad. Sci. USA (1980) 77, 4216-4220) and CHO K-1 (Proc. Natl. Acad. Sci. USA (1968) 60, 1275). Among the animal cells, the CHO cell is particularly preferred for mass expression. Introduction of the vector into the host cell can be carried out by, for example, a calcium phosphate method, a DEAE-dextran method, a method using cationic liposome DOTAP (manufactured by Boehringer Mannheim), an electroporation method, lipofection or the like.

A plant cell derived from Nicotiana tabacum is known as a protein production system and may be subjected to callus culture. Fungal cells known in the art include yeast such as the Saccharomyces genus, for example, Saccharomyces cerevisiae and filamentous bacteria such as Aspergillus genus, for example, Aspergillus niger.

For prokaryotic cells, various production systems utilizing a bacterial cell are available. Examples of the bacterial cell include E. coli such as JM109, DH5α and HB101, and the like. In addition, Bacillus subtilis is also available.

Such a cell is transformed by a desired DNA, and the resulting transformed cell is cultured in vitro to obtain the protein. Cultivation can be carried out according to a known method. The culture medium used for animal cells include, for example, DMEM, MEM, RPMI1640, or IMDM. A serum supplement such as fetal calf serum (FCS) may be used in combination with the medium, or serum free culture may be carried out. The pH of the culture medium during the cultivation ranges preferably from about 6 to 8. Cultivation is generally carried out at about 30 to 40°C, for 15 to 200 hours, and the culture medium may be replaced, aerated, or stirred as needed.

On the other hand, examples of the in vivo protein production system include a production system using an animal and a production system using a plant. A desired DNA is introduced into such an animal or a plant, and the protein is allowed to be produced in the animal or the plant, and then collected. The term "host" in the present invention encompasses such an animal and a plant.

For animals, various production systems utilizing a mammal or an insect are available. The mammal includes goats, pigs, sheep, mice and cattle (Vicki Glaser, SPECTRUM Biotechnology Applications, 1993). In addition, the mammal may also include a transgenic animal.

For example, a desired DNA is prepared as a fusion gene with a gene encoding a protein such as goat β-casein that is specifically produced into milk. Next, the DNA fragment containing this fusion gene is injected into a goat's embryo, which is then implanted in a female goat. A desired protein can be obtained from milk produced by a transgenic goat which is born from the goat that had received the embryo or offspring thereof. To increase the amount of milk containing the protein produced by the transgenic goat, an appropriate hormone may be administered to the transgenic goat (Ebert, R.M. et al., Bio/Technology (1994) 12, 699-702)..

Further, a silkworm may be used as a host insect. The silkworm is infected with a baculovirus into which DNA encoding a desired protein has been inserted. The desired protein can be obtained from the body fluid of the silkworm (Susumu, M. et al., Nature (1985) 315, 592-594).

Further, tobacco may be used as a plant host. DNA encoding a desired protein is inserted into a plant expression vector such as pMON 530, and this vector is introduced into bacteria such as Agrobacterium tumefaciens. Then, tobacco such as Nicotiana tabacum is infected with the bacteria and the desired protein can be obtained from the leaves of the tobacco (Julian, K. -C. Ma et al., Eur. J. Immunol. (1994) 24, 131-138).

The protein described herein and obtained as above can be isolated from the inside or the outside of the host cell (such as medium) and purified to a substantially pure homogenous protein. Any method commonly used for isolation and purification of protein may be used for purifying the protein. For example, chromatography column, filtration, ultrafiltration, salting out, solvent precipitation, solvent extraction, distillation, immunoprecipitation, SDS-polyacrylamide gel electrophoresis, isoelectric focusing, dialysis, recrystallization and the like may be suitably selected and combined, whereby the protein can be isolated and purified.

Examples of the chromatography include affinity chromatography, ion exchange chromatography, hydrophobic chromatography, gel filtration, reversed-phase chromatography, adsorption chromatography and the like (Strategies for Protein Purification and Characterization: A Laboratory Course Manual. Ed Daniel R. Marshak et al., Cold Spring Harbor Laboratory Press, 1996). Such chromatography can be carried out by liquid chromatography such as HPLC or FPLC. A protein highly purified by using such a purification method is also encompassed by the present invention.

The protein can be optionally modified or a peptide portion can be removed by treating the protein with an appropriate protein-modifying enzyme before or after the purification of the protein. Such a protein-modifying enzyme includes, for example, trypsin, chymotrypsin, lysylendopeptidase, protein kinase, glucosidase and the like.

As shown in Examples described below, PCR primers were designed based on the gene sequences of cancer-associated gene represented by SEQ ID NO: 2 (see Table 1) and the expression level of the cancer-associated gene in human tissue was quantified by quantitative PCR using cDNA obtained from human normal tissue and cancer tissue. It was found that the expression of the cancer-associated genes as described herein is elevated in certain human cancer tissue.

Inter alia the expression of a gene having the nucleotide sequence represented by SEQ ID NO: 2. was found to be elevated in lung cancer. Thus, a protein encoded by the gene having the nucleotide sequence represented by SEQ ID NO:2 or a fragment thereof is useful for diagnosing lung cancer.

### Antibody

Also described is an antibody recognizing a protein encoded by the cancer-associated gene having the nucleotide sequence represented by SEQ ID NO: 2 or a fragment of the protein, or an antigen-binding fragment of the antibody. Further disclosed is:
a composition for diagnosing cancer comprising the antibody or the binding fragment thereof. The antibody can recognize a protein having an amino acid sequence represented by SEQ ID NO : 66 or a fragment thereof. Also described is a cell producing such an antibody.

The term "recognize" means that an antibody binds to the above-mentioned protein encoded by a cancer-associated gene or a fragment thereof under specific conditions with higher affinity than it binds to another polypeptide.

The described antibody may include monoclonal antibodies, polyclonal antibodies, antibodies having an ability of specifically binding to an antigen determinant, variants and derivatives of antibodies such as T-cell receptor fragments.

The type of the antibody is not particularly limited, but may include a mouse antibody, a human antibody, a rat antibody, a rabbit antibody, a sheep antibody, a camel antibody or the like. Also included are a genetically recombinant antibody which has been artificially modified for the purpose of lowering heterologous antigenicity against human such as a chimeric antibody, a humanized antibody or the like. The genetically recombinant antibody can be produced by a known method. The chimeric antibody is an antibody comprising antibody heavy chain and light chain variable regions of a non-human mammal such as a mouse, and the antibody heavy chain and light chain constant regions of a human. It can be obtained by ligating DNA encoding the variable region of a mouse antibody to DNA encoding the constant region of a human antibody, which is then introduced into an expression vector and introduced into a host for production of the antibody. A humanized antibody, also referred to as a reshaped human antibody, is obtained by transplanting a complementarity determining region (CDR) of an antibody of a non-human mammal such as a mouse, into the complementarity determining region of a human antibody. A general technique of genetic recombination is also known. Specifically, a DNA sequence designed to ligate a CDR of a mouse antibody to the framework region (FR) of a human antibody is synthesized by a PCR method using several oligonucleotides constructed to have overlapping portions at their ends. Then, the resulting DNA is ligated to DNA encoding a human antibody constant region, which is introduced into an expression vector, and the vector is introduced into a host to produce an antibody, whereby a humanized antibody can be obtained (see European Patent Application No. EP239400, and International Patent Application No. WO 96/02576). The human antibody FR that is ligated via the CDR is selected to allow the complementarity determining region to form a favorable antigen-binding site. As necessary, an amino acid in the framework region of an antibody variable region may be substituted such that the complementarity determining region of a reshaped human antibody forms an appropriate antigen-binding site (Sato, K. et al., Cancer Res. 1993, 53, 851-856).

Methods for obtaining a human antibody are also known. For example, a desired human antibody having an antigen-binding activity can be obtained by sensitizing a human lymphocyte with a desired antigen or a cell expressing a desired antigen in vitro, and fusing the sensitized lymphocyte with a human myeloma cell such as U266 (see JP-B-1-59878). Alternatively, a desired human antibody can also be obtained by using a desired antigen to immunize a transgenic animal having the entire repertoire of human antibody genes (see International Patent Application Nos. WO 93/12227, WO 92/03918, WO 94/02602, WO 94/25585, WO 96/34096 and WO 96/33735). Further, techniques to obtain a human antibody by panning with a human antibody library are known in the art. For example, the variable region of a human antibody is expressed as a single chain antibody (scFv) on the surface of a phage using a phage display method, and a phage binding to the antigen can be selected. By analyzing the gene of the selected phage, the DNA sequence encoding the variable region of a human antibody binding to the antigen can be determined. If the DNA sequence of scFv that binds to the antigen is identified, an appropriate expression vector containing the sequence is constructed, and a human antibody can be obtained. These methods are already well known and described in WO 92/01047, WO 92/20791, WO 93/06213, WO 93/11236, WO 93/19172, WO 95/01438 and WO 95/15388.

Moreover, the described antibody may also be a low molecular weight antibody such as an antibody fragment or a modified antibody as long as it can bind to an antigen. Specific examples of the antibody fragment include Fab, Fab' , F(ab')2, Fv, Diabody and the like. To obtain such an antibody fragment, a gene encoding such an antibody fragment is constructed, the gene is introduced into an expression vector, and then the gene may be expressed in a suitable host cell (see, for example, Co, M.S. et al., J. Immunol. (1994) 152, 2968-2976; Better, M. and Horwitz, A.H., Methods Enzymol. (1989) 178, 476-496; Pluckthun, A. and Skerra, A., Methods Enzymol. (1989) 178, 497-515; Lamoyi, E. , Methods Enzymol. (1986) 121, 652-663; Rousseaux, J. et al., Methods Enzymol. (1986) 121, 663-669; and Bird, R. E. and Walker, B. W., Trends Biotechnol. (1991) 9, 132-137).

The modified antibody may also include an antibody bound to any of a variety of molecules such as polyethylene glycol (PEG). In addition, a radioisotope, a chemotherapeutic agent, a cytotoxic substance such as a bacteria-derived toxin may be attached to the antibody. A radiolabeled antibody is particularly useful. Such a modified antibody can be obtained by subjecting the obtained antibody to chemical modification. The method of modifying an antibody has been already established in this field.

Further, it is possible to use an antibody whose sugar chain is modified for the purpose of enhancing the cytotoxic activity or the like. Techniques to modify a sugar chain of an antibody have been already known (e.g., WO 00/61739, WO 02/31140, etc.)

Further, the present invention also encompasses the use of a multispecific antibody having specificity for at least two different antigens. While such a molecule is generally binds to two antigens (i.e., bispecific antibody), the term "multispecific antibody" in the present invention encompasses an antibody having specificity for two or more (such as three) antigens. The multispecific antibody can be a full length antibody or a fragment of such an antibody (e.g. F(ab')₂ bispecific antibody).

Methods for producing the multispecific antibody are known in the art. Production of a full length bispecific antibody comprises co-expression of two immunoglobulin heavy chain-light chain pairs in which the two chains have different specificities (Millstein et al., Nature, 305: 537-539 (1983)). Because of the random assortment of immunoglobulin heavy chain and light chain, plural hybridomas (quadromas) co-expressing the heavy and light chains are obtained as a mixture of hybridomas respectively expressing different antibody molecules. Thus, it is necessary to select those producing a correct bispecific antibody. The selection can be carried out by affinity chromatography. In another method, antibody variable domains with the desired binding specificities are fused to immunoglobulin constant domain sequences. The constant domain sequence preferably comprises at least part of the hinge, CH2, and CH3 regions of the immunoglobulin heavy chain constant domain. It is preferred to contain the heavy chain CH1 region necessary for binding with the light chain. DNA encoding the immunoglobulin heavy chain fusions and, if desired, DNA encoding the immunoglobulin light chains, are inserted into separate expression vectors, and are transfected into a suitable host organism. Insertion of the respective genes into separate expression vectors will allow for adjustment of the expression ratios of the respective chains. This method is convenient when unequal ratios of the respective chains will provide an increase in the yield of the obtained antibody. It is, however, possible to insert the genes encoding plural chains into one vector.

In a preferred embodiment, a bispecific antibody is composed of a hybrid immunoglobulin heavy chain with a first binding specificity in one arm, and a hybrid immunoglobulin heavy chain-light chain pair with a second binding specificity in the other arm. By allowing an immunoglobulin light chain to be present in only one arm in this way, the isolation of the bispecific antibody from other immunoglobulins can be readily carried out. As for the isolation method, see WO. 94/04690. As for the production method of the bispecific antibody, see the method of Suresh et al. (Methods in Enzymology, 121: 210 (1986)). To decrease the number of homodimers in the final product obtained from the recombinant cell culture and increase the ratio of the heterodimers, one antibody molecule including CH3 of an antibody constant domain may be modified, where one or more amino acids with a small side chain present on the surface and binding to the other molecule are replaced with an amino acid with a larger side chain (e.g. tyrosine or tryptophan), and an amino acid with a large side chain in the region corresponding to the other antibody molecule is replaced with a smaller one (e.g. alanine or threonine), whereby a cavity corresponding to the larger side chain in the first antibody molecule is created (WO 96/27011).

The bispecific antibody also include a heteroconjugate antibody in which one antibody is coupled to avidin and the other is coupled to biotin or the like (U.S. Pat. No. 4,676,980, WO 91/00360, WO 92/200373, and EP 03089). A cross-linking agent to be used in the production of such a heteroconjugate antibody is well known, and is disclosed in, for instance, U.S. Pat. No. 4,676,980.

A method for producing the bispecific antibody from an antibody fragment has also been reported. For example, it can be produced by utilizing a chemical bond. First, F(ab')₂ fragments are produced and are reduced in the presence of sodium arsenite, a dithiol complexing agent, in order to prevent disulfide formation within the same molecule. Then, the F(ab')₂ fragments are converted to thionitrobenzoate (TNB) derivatives. Then, one of the F(ab')₂-TNB derivatives is reduced again to the Fab'-thiol with mercaptoethylamine, and the F(ab')₂-TNB derivatives are mixed with an equimolar amount of the Fab' -thiol, whereby a bispecific antibody is produced.

Various methods for producing and isolating bispecific antibodies directly from the recombinant cell culture have also been reported. For example, a method for producing bispecific antibodies using leucine zippers has been reported (Kostelny et al., J. Immunol., 148 (5): 1547-1553 (1992)). First, the leucine zipper peptides from the Fos and Jun proteins were linked to the Fab' portions of different antibodies by gene fusion. The antibody homodimers were reduced at the hinge region to form monomers and then re-oxidized to form antibody heterodimers. Another method is available in which a heavy-chain variable domain (VH) is connected to a light-chain variable domain (VL) via a linker which is too short to allow pairing between these two domains to form a pair of complementary other VL and VH domains, whereby two antigen-binding sites are created (Hollinger et al., Proc. Natl. Acad. Sci. USA 90: 6444-6448 (1993)). In addition, dimers obtained from a single-chain Fv (sFv) have also been reported (Gruger et al., J. Immunol., 152: 5368 (1994)). Further, trispecific antibodies instead of bispecific antibodies have also been reported (Tutt et al. J. Immunol. 147: 60 (1991)).

The described "antibody" also encompasses antibodies described above.

The described antibody and the antibody fragment can be produced by any suitable manner such as in vivo, cultured cells, in vitro translation reaction or a recombinant DNA expression system.

Techniques for producing monoclonal antibodies and hybridomas are well known in the art (Campbell, "monoclonal Antibody Technology: Laboratory Techniques in Biochemistry and Molecular Biology", Elsevier Science Publishers, Amsterdam, The Netherlands, 1984; St. Groth, et al., J. Immunol. Methods 35: 1-21, 1980). The above-mentioned protein encoded by a cancer-associated gene or a fragment thereof may be used as an antigen to immunize any animal (mouse, rabbit, or the like) which is known to produce antibodies by subcutaneous or intraperitoneal injection. An adjuvant may be used in the immunization, and the use of such an adjuvant is well known in the art.

Polyclonal antibodies can be obtained by isolating antisera containing antibodies from an immunized animal, and detecting the presence of an antibody with the desired specificity by using a well known method in the art such as an ELISA assay, Western blot analysis, or radioimmunoassay.

Monoclonal antibodies can be obtained by excising spleen cells from an immunized animal, fusing the spleen cells with myeloma cells, and producing hybridoma cells that produces monoclonal antibodies. A hybridoma cell that produces an antibody recognizing a desired protein or a fragment thereof is selected using a method well known in the art such as an ELISA assay, Western blot analysis, or radioimmunoassay. A hybridoma secreting the desired antibody is cloned and cultured under appropriate conditions, the secreted antibody is recovered, and purified by a method well known in the art, such as ion exchange column or affinity chromatography. Alternatively, a human monoclonal antibody may be produced by using a XenoMouse strain (see Green J. Immunol. Methods 231: 11-23, 1999; Wells, Eek, Chem Biol 2000 Aug; 7 (8): R185-6).

The DNA encoding the monoclonal antibody can be readily isolated and sequenced using a conventional method (e.g., by using an oligonucleotide probe capable of binding specifically to genes encoding the heavy chain and the light chain of the monoclonal antibody). The hybridoma cells serve as a preferred source of such DNA. Once isolated, the DNA is inserted into an expression vector, which is then transfected into a host cell such as an E. coli cell, simian COS cell, Chinese Hamster Ovary (CHO) cell, or myeloma cell that does not otherwise produce immunoglobulin protein, whereby a monoclonal antibody is produced in the recombinant host cell. In another embodiment, an antibody or antibody fragment can be isolated from an antibody phage library generated using the techniques described in McCafferty et al. (Nature 348: 552-554 (1990).

The above-mentioned antibody can be detectably labeled. Examples of the label include radioisotopes, affinity labels (such as biotin and avidin), enzymatic labels (such as horseradish peroxidase and alkaline phosphatase), fluorescent labels (such as FITC or rhodamine), paramagnetic atoms and the like. Methods for accomplishing such labeling are well known in the art. The above-mentioned antibodies may be immobilized on a solid support. Examples of the solid support include plastic, agarose, sepharose, polyacrylamide, latex beads and the like. Techniques for immobilizing antibodies on such a solid support are well known in the art.

As described in the Examples below, the cancer-associated gene described herein shows elevated expression in as specific cancer tissue, therefore the described antibody is useful as a diagnostic marker for cancer. The expression of the protein encoded by the cancer-associated gene can be detected in a tissue or cell using the described antibody in a method such as Western blotting, the ELISA method or histological staining. A sample (such as biopsy sample or blood sample) derived from tissue of a subject is brought into contact with a composition containing the descried antibody under conditions so as to form an immune complex.

The presence or the amount of the protein encoded by the cancer-associated gene in the sample can be determined by determining whether the sample binds to the antibody. In this way, diagnosis of cancer, monitoring of progress or cure of cancer, and prediction of prognosis may be carried out. The diagnostic composition described herein may be provided as a kit for detecting the presence of the above-mentioned protein encoded by the cancer-associated gene in a sample. Such a kit may contain, in addition to the above-mentioned antibody, a washing reagent , a reagent that can detect the presence of the bound antibody such as a labeled secondary antibody, a chromophore that can react with the labeled antibody, an enzyme, and an antibody-binding reagent, and a usage guideline.

Further, the antibody against the protein encoded by the disclosed cancer-associated gene has some specificity for a specific cancer cell, therefore it may be used as a diagnostic agent for cancer.

Preferably, the composition containing the described antibody is used in diagnosis of lung cancer, pancreatic cancer and liver cancer.

### Polynucleotide

Described is also a polynucleotide having a nucleotide sequence represented by SEQ ID NO: 2 or a nucleotide sequence complementary thereto or a polynucleotide that can hybridize under high stringent conditions to this polynucleotide.

Also described is a composition containing a polynucleotide having a sequence comprising at least 12 consecutive nucleotides of the nucleotide sequence represented by SEQ ID NO: 2 or a nucleotide sequence complementary thereto or an oligonucleotide with a length of at least 12 nucleotides that can hybridize under high stringent conditions to a polynucleotide having the nucleotide sequence represented by SEQ ID NO : 2

Such a polynucleotide is useful for diagnosis of cancer, The cancer is preferably selected from lung cancer, pancreatic cancer bowel cancer and liver cancer.

The expression of the described cancer-associated gene represented by SEQ ID NO: 2 is elevated in a specific human cancer tissue as shown in the following Examples. Therefore, the described composition may be used as an agent of antisense oligonucleotide, ribozyme, siRNA or the like for silencing the expression of the cancer-associated gene, and as a probe or a primer for detecting the cancer-associated gene. It can also be used for producing the described protein.

The polynucleotide or the oligonucleotide contained in the described composition may be single stranded or double stranded, and may be DNA, RNA, or a mixture thereof or a derivative of PNA or the like. Such a polynucleotide or oligonucleotide may be chemically modified at the internucleoside linkage, the base moiety and/or the sugar moiety, or may have a modifier at the 5' end and/or 3' end. Examples of the modified internucleoside linkage include phosphorothioate, phosphorodithioate, phosphoramidethioate, phosphoramidate, phosphorodiamidate, methylphosphonate, alkylphosphotriester, formacetal and the like. Examples of the modified base moiety include 5-fluorouracil, 5-bromouracil, 5-chlorouracil, 5-iodouracil, hypoxanthine, xanthine, 4-acetylcytosine, 5-(carboxyhydroxylethyl) uracil and the like. Examples of the modified sugar moiety include 2' -O-alkyl, 2' -O-alkyl-O-alkyl or 2' -fluoro modification and the like. In addition, a sugar other than ribose may also be used, for example, arabinose, 2-fluoroarabinose, xylulose and hexose.

The disclosed polynucleotide includes a. polynucleotide having a nucleotide sequence represented by SEQ ID NO: 2' or a nucleotide sequence complementary thereto or a polynucleotide that can hybridize under high stringent conditions to this polynucleotide. A polynucleotide that can hybridize under stringent conditions generally has a high identity. The term "high identity" as used herein means that it has an identity of 70% or more, preferably 80% or more, more preferably 90% or more with a nucleotide sequence represented by any of SEQ ID NOs: 1 to 65.

The identity of a nucleotide sequence can be determined by using the algorithm BLAST by Karlin and Altschul (Proc. Natl. Acad. Sci. USA, 90: 5873-5877, 1993). The programs called BLASTN and BLASTX have been developed based on the above algorithm (Altschul et al. , J. Mol. Biol. , 215: 403-410 1990). In the case of analyzing a nucleotide sequence by BLASTN based on BLAST, the parameters can be set, for example, score = 100 and wordlength = 12. BLAST and Gapped BLAST programs may be used with its default parameter. The specific techniques of these analysis methods are known in the art (http://www.ncbi.nl.m.nih.gov.).

Further, the described, polynucleotide encodes an amino acid sequence represented by SEQ ID NO: 66. Such a polynucleotide may be used in the production of said protein. In addition, since a polynucleotide having the nucleotide sequence represented by SEQ ID NO: 2 or a sequence complementary thereto is overexpressed in a cancer cell, it can be used as a probe for diagnosing cancer by detecting such a polynucleotide.

The described polynucleotide or the oligonucleotide can be produced by a method known to those skilled in the art. For example, it can be synthesized with a commercially available DNA synthesizer (e.g., 394 synthesizer, manufactured by Applied Biosystems) using a protocol known in the art. Alternatively, it can be produced based on the sequence information disclosed in the instant application by the PCR amplification technique well known in the art using a suitable template and primers in combination.

Further, the described polynucleotide or the oligonucleotide can be prepared by constructing a cDNA library from cells expressing the disclosed polypeptide and performing hybridization using a probe having a part of the sequence of the described polynucleotide.

The cDNA library may be prepared by, for example, a method described in the document (Sambrook, J. et al., Molecular Cloning, Cold Spring Harbor Laboratory Press (1989)), or a commercially available DNA library may be used. Alternatively, it can be prepared by preparing RNA from cells expressing the disclosed polypeptide, synthesizing cDNA with a reverse transcriptase, synthesizing oligo-DNA based on the disclosed DNA. sequence (i.e. SEQ ID NO: 2), and amplifying the cDNA encoding the polypeptide of the present invention by PCR using the synthesized oligo-DNA as a primer.

Further, by determining the nucleotide sequence of the obtained cDNA, one can determine the translated region of the cDNA and the amino acid sequence of the disclosed protein.

Further, the obtained cDNA can also be used as a probe for screening a genomic DNA library to isolate genomic DNA.

More specifically, mRNA is first isolated from a cell, tissue (e.g., a lung cancer cell, large bowel cancer cell, liver cancer cell or stomach cancer cell) or the like in which the disclosed protein is expressed. The isolation of mRNA is carried out by a known method. For example, total RNA is prepared by using guanidine ultracentrifugation (Chirgwin J. M. et al. Biochemistry (1979) 18, 5294-5299), or AGPC method (Chomczynski P. and Sacchi N. Anal..Biochem. (1987) 162, 156-159), and mRNA is purified from the total RNA using an mRNA Purification Kit (Pharmacia). Alternatively, mRNA can be directly prepared by using a QuickPrep mRNA Purification Kit (Pharmacia).

From the obtained mRNA, cDNA is synthesized using a reverse transcriptase. The synthesis of cDNA can also be carried out by using AMV Reverse Transcriptase First-strand cDNA Synthesis Kit (Seikagaku Kogyo). Alternatively, cDNA can be synthesized and amplified according to the 5'-RACE method (Frohman M. A. et al. Proc. Natl. Acad. Sci. U.S.A. (1988) 85, 8998-9002; Belyavsky A. et al. Nucleic Acids Res. (1989) 17, 2919-2932), using a 5' -Ampli FINDER RACE Kit (manufactured by Clontech) and polymerase chain reaction (PCR).

A desired DNA fragment is prepared from the obtained PCR products and ligated to a vector DNA, whereby recombinant vectors are prepared. These recombinant vectors are introduced into E. coli or the like, and a desired recombinant vector is prepared by selecting a colony. The nucleotide sequence of the desired DNA can be determined by a known method such as the dideoxynucleotide chain termination method.

By taking into account the frequency of codon usage in the host to be used for expression, the nucleotide sequence of the disclosed RNA can be designed to be expressed more efficiently (Grantham R. et al. Nucleic Acids Research (1981) 9, r43-74). The described DNA may be modified by a commercially available kit or by a known method. Examples of the modification include, for example, digestion with a restriction enzyme, insertion of a synthetic oligonucleotide or an appropriate DNA fragment, addition of a linker, insertion of the initiation codon (ATG) and/or a stop codon (TAA, TGA, or TAG).

The disclosed oligonucleotide may be used as a nucleic acid probe for detecting the disclosed cancer-associated genein a sample. The probe is selected to have a nucleotides sequence comprising at least 12, 20, 30, 50, 100 or more consecutive nucleotides of the nucleotide sequence represented by SEQ ID NO: 2 or a nucleotide sequence complementary thereto, and to hybridize specifically to a specific region of the cancer-associated gene. DNA is extracted from a sample such as tissue or blood, or mRNA is extracted and cDNA is synthesized. DNA or cDNA is brought into contact with the probe under the conditions that allows hybridization to occur, and the presence or the amount of the probe bound to the sample is detected, whereby the presence or amount or variation of the cancer-associated gene or a transcript thereof in the sample can be detected.

The probe may be immobilized on a solid support. Examples of such a solid support include, but are not limited to, plastic, agarose, sepharose, polyacrylamide, latex beads, and nitrocellulose. Techniques to immobilize the probe on such a solid support are well known in the art. The probe can be visualized by labeling with a standard labeling technique such as radioactive labeling, enzymatic labeling (horseradish peroxidase or alkaline phosphatase), fluorescence labeling, biotin-avidin labeling or chemiluminescence. The described composition can be provided as a kit for detecting the presence of the disclosed cancer-associated gene or a transcript thereof in a sample. Such a kit may contain, in addition to the above-mentioned probe, a washing reagent, a reagent that can detect the presence of a bound probe, and a usage guideline.

Alternatively, the disclosed diagnostic composition may contain a pair of primers that can amplify the nucleotide sequence represented by SEQ ID NO: 2. With the use of these primers, the desired sequence is amplified by polymerase chain reaction (PCR) by using an appropriate cDNA library as a template. The PCR products are analyzed by a technique such as hybridization or nucleotide sequencing, whereby the presence or amount or variation of the cancer-associated gene or a transcript thereof in a sample can be detected. Such a PCR technique is well known in the art, and described in, for example, "PCR Protocols, A Guide to Methods and Applications", Academic Press, Michael, et al., eds. 1990.

For use as a primer, it is preferred that the oligonucleotide has a sequence comprising at least 12, preferably 12 to 50, more preferably 12 to 20 consecutive nucleotides of the nucleotide sequence represented by SEQ ID NO: 2. or a nucleotide sequence complementary thereto.

### Detection method

The present invention provides a method of detecting cancer comprising the step of measuring the expression level of the gene or the protein as described herein. Specific embodiments of the detection method will be described below, however, the defection method of the present invention is not limited to such a method.

In one embodiment of the detection method of the present invention, an RNA sample is first prepared from a subject. Subsequently, the level of RNA encoding the disclosed protein contained in the RNA sample is measured. Then, the measured RNA level is compared with that of a control. In another embodiment, a cDNA sample is first prepared from a subject. Subsequently, the level of cDNA encoding the protein as disclosed herein contained in the cDNA sample is measured. Then, the measured cDNA level is compared with that of a control.

Such a method includes any of the methods well known to those skilled in the art, for example, Northern blotting, RT-PCR, DNA array analysis.

In the DNA array analysis, a cDNA sample is prepared by using RNA from a subject as a template, and is brought into contact with a substrate on which the disclosed oligonucleotide has been immobilized. The intensity of hybridization of the cDNA sample with the nucleotide probe immobilized on the substrate is detected to determine the expression level of the disclosed gene contained in the cDNA sample. Subsequently, the measured expression level of the disclosed gene is compared with that of a control.

The cDNA sample may be prepared from a subject by a method well known to those skilled in the art. In a preferred embodiment for preparing the cDNA sample, total RNA is first extracted from cells or tissue (e.g., lung, large bowel, stomach, liver, etc.) of a subject. Total RNA may be extracted by a method well known to those skilled in the art. Total RNA may be extracted by a conventional method or kit that provides highly pure total RNA. For example, a sample is pretreated with "RNA later" (Ambion) and total RNA is extracted using "Isogen" (Nippon Gene). Specific procedures may follow the attached protocols.

Subsequently, cDNA is synthesized with a reverse transcriptase using the extracted total RNA as a template to prepare a cDNA sample. The synthesis of cDNA from total RNA can be carried out by a method well known to those skilled in the art. The prepared cDNA sample is detectably labeled as needed. The labeling substance is not particularly limited as long as it can be detected, and may include fluorescent substances and radioisotopes. cDNA may be labeled by a method generally used by those skilled in the art (L. Luo et al., Gene expression profiles of laser-captured adjacent neuronal subtypes, Nat. Med., 1999,117-122).

Those skilled in the art can appropriately determine the intensity of hybridization between cDNA and a nucleotide probe, depending on the type of substance used to label the cDNA sample. For example, when the cDNA is labeled with a fluorescent substance, it can be detected by reading the fluorescent signal with a scanner.

In another embodiment of the detection method of the present invention, a protein sample is first prepared from cells or tissue of a subject. The level of the disclosed protein contained in the protein sample is measured. Then, the measured protein level is compared with that of a control. Examples of a method for measurement include SDS polyacrylamide electrophoresis, and methods utilizing the antibody of the invention, such as Western blotting, dot-blotting, immunoprecipitation, enzyme-linked immunosorbent assay (ELISA), and immunofluorescence. Alternatively, it is possible to diagnose cancer by measuring the expression level of the disclosed protein rather than measuring the expression level of the disclosed gene.

In the above-mentioned method, the subject is diagnosed to have cancer or to have a high possibility of developing cancer when the expression level of the disclosed gene or protein increased significantly compared with that of a control,

Further, the present invention discloses a diagnostic drug to be used for detecting cancer. Examples of such a test drug include a test drug comprising the disclosed oligonucleotide (including a substrate on which the oligonucleotide is immobilized), and a diagnostic drug comprising the disclosed antibody. Any type of antibodies may be used as long as it is suitable for use in a diagnostic test. The antibody is labeled as needed.

The above-mentioned test drug may contain, in addition to the oligonucleotide or the antibody as an active ingredient, sterilized water, physiological saline, a vegetable oil, a surfactant, a lipid, a solubilizing agent, a buffer, a protein stabilizer (such as BSA or gelatin), a preservative or the like.

### Detection of C20orf102

In particular, the present invention provides a method of diagnosing cancer by detecting C20orf102 protein. The method of the present invention is characterized by detecting C20orf102 protein. C20orf102 is a secretory protein with a secretory signal at the N-terminus, and its amino acid sequence and the gene sequence encoding this sequence are described in GenBank Accession No. NM_080607 (SEQ ID NOs: 2 and 66). In the present invention, C20orf102 protein encompasses both full-length protein and a fragment thereof. The fragment is a polypeptide containing a given domain of C20orf102 protein and it may not have a function of natural C20orf102 protein. The secretory signal of C20orf102 protein corresponds to 1 to 24 amino acids in the amino acid sequence represented by SEQ ID NO: 66 (Psort Prediction: http://psort.nibb.ac.jp/).

In the present invention, it was found that the expression of C20orf102 is elevated at the protein level with a very high frequency in a cancer cell, particularly in lung cancer, liver cancer (e.g., moderately differentiated liver cancer) or pancreatic cancer. In addition, it was shown that immunohistological diagnosis may be carried out by using a monoclonal antibody specific for C20orf102.

The C20orf102 protein to be detected in the present invention is preferably human C20orf102 protein, however, it may be any C20orf102 such as dog C20orf102, cat C20orf102, mouse C20orf102 or hamster C20orf102.

C20orf102 to be detected in the present invention may be C20orf102 of pre-secreted type, however, it is preferably C20orf102 of post-secreted type. C20orf102 is a secretory protein with a secretory signal at the N-terminus, and is secreted to the outside of a cell after being produced in then cell. C20orf102 of post-secreted type means those C20orf102 proteins present outside the cell.

In the present invention, detection includes quantitative or non-quantitative detection. For example, non-quantitative detection includes simple determination of whether or not C20orf102 protein is present, determination of whether or not a predetermined amount or more of C20orf102 protein is present, comparison of the amount of C20orf102 protein with that in another sample (e.g., a control sample, etc.). Quantitative detection includes determination of the concentration of C20orf102 protein, determination of the amount of C20orf102 protein and the like.

The test sample is not particularly limited as long as it may contain C20orf102 protein, but is preferably those collected from the body of an organism such as a mammal, more preferably those collected from human. Specific examples of the test sample include cells, cell homogenate, blood, interstitial fluid, plasma, extravascular fluid, cerebrospinal fluid, synovial fluid, pleural fluid, serum, lymph fluid, saliva, urine and the like, preferably blood, serum or plasma. Further, a sample derived from a test sample, such as a culture solution of cells collected from the body of an organism, is also included in the test sample of the present invention.

Specific examples of the cancer to be diagnosed according to the invention include, but not limited to, liver cancer, pancreatic cancer, lung cancer, large bowel cancer, breast cancer, renal cancer, brain tumor, uterine cancer, lung cancer, stomach cancer, prostate cancer, leukemia, lymphoma and the like. Preferred are lung cancer, liver cancer and pancreatic cancer.

Liver cancer is classified into poorly differentiated liver cancer, moderately differentiated liver cancer, well differentiated liver cancer and the like. Any liver cancer may be detected according to the present invention. Preferably, moderately differentiated liver cancer is detected.

Lung cancer is further classified into lung adenocarcinoma, lung squamous cell carcinoma, lung small cell cancer, lung large cell cancer and the like. Any lung cancer may be detected according to the present invention. Preferably, lung adenocarcinoma is detected.

In the present invention, the subject is diagnosed to have cancer or to have a high possibility of developing cancer when C20orf102 protein is detected in a test sample, or when it is determined that the amount of C20orf102 protein detected in a test sample is higher compared with that of a negative control or a normal healthy subject.

In a preferred embodiment of the diagnostic method of the present invention, the diagnostic method is characterized by detecting C20orf102 protein released from a cell and present in the blood. Particularly preferably, C20orf102 protein or a fragment thereof present in the blood is detected.

C20orf102 protein contained in a test sample may be detected by any methods, but is preferably detected by an immunological method using an anti-C20orf102 antibody. Examples of the immunological method include radioimmunoassay, enzyme immunoassay, fluorescence immunoassay, luminescent immunoassay, immunoprecipitation, immunonephelometry, Western blotting, immunostaining, immunodiffusion method and the like. Preferred is an enzyme immunoassay, and particularly preferred is an enzyme-linked immunosorbent assay (ELISA) (e.g., sandwich ELISA). The above-mentioned immunological methods such as ELISA may be carried out by a method known to those skilled in the art.

For example, a general detection method for detecting C20orf102 protein in a test sample using an anti-C20orf102 antibody may comprise the step of immobilizing the anti-C20orf102 antibody on a support, adding a test sample thereto, incubating the sample to allow for binding the anti-C20orf102 antibody to C20orf102 protein, washing, and detecting C20orf102 protein bound to the support via the anti-C20orf102 antibody.

The support to be used for immobilizing the anti-C20orf102 antibody in the present invention may include an insoluble support made of an insoluble polysaccharide such as agarose or cellulose, a synthetic resin such as a silicon resin, polystyrene resin, a polyacrylamide resin a nylon resin or a polycarbonate resin, glass or the like. Such a support may be used in the form of beads, a plate or the like. In the case of beads, a column may be filled with these beads. A plate may include a multiwell plate (96-multiwell plate or the like), a biosensor chip or the like. The binding of the anti-C20orf102 antibody to the support may be effected by a commonly used method such as via a chemical bond or physical adsorption. All these supports are commercially available.

Usually, the anti-C20orf102 antibody may be bound to C20orf102 protein in a buffer. The buffer may include, for example, a phosphate buffer, a Tris buffer, a citrate buffer, a borate buffer, a carbonate buffer or the like. Incubation may be carried out under commonly used conditions, for example at 4°C to room temperature for 1 hour to 24 hours. The washing step after the incubation can be carried out in any way as long as it does not inhibit the binding of C20orf102 protein to the anti-C20orf102 antibody, for example, with a buffer containing a surfactant such as Tween 20.

In the method of the present invention of detecting C20orf102 protein, a control sample may be prepared besides the test sample to be analyzed for C20orf102 protein. The control sample includes a negative control sample that does not contain C20orf102 protein and a positive control sample that contains C20orf102 protein. In this case, C20orf102 protein in a test sample can be detected by comparing the result with the result obtained from the negative control sample that does not contain C20orf102 protein, or the result from the positive control sample that contains C20orf102 protein. In addition, a series of control samples having incremental concentrations are prepared, and the results from the respective control samples are obtained as numerical values to create a standard curve. C20orf102 protein contained in a test sample can be quantitatively detected from the numerical value of the result from the test sample based on the standard curve.

In a preferred embodiment, C20orf102 protein bound to the support via the anti-C20orf102 antibody may be detected using an anti-C20orf102 antibody labeled with a labeling substance. For example, a test sample is brought into contact with an anti-C20orf102 antibody immobilized on a support, washed and detected using a labeled antibody that specifically recognizes C20orf102 protein.

The labeling of the anti-C20orf102 antibody may be carried out by a commonly known method. Any of the labeling substances known to those skilled in the art may be used in the invention, such as a fluorescent dye, an enzyme, a coenzyme, a chemiluminescent substance or a radioactive substance. Specific examples include radioisotopes (such as ³²P, ¹⁴C, ¹²⁵I, ³H and ¹³¹I), fluorescein, rhodamine, dansyl chloride, umbelliferone, luciferase, peroxidase, alkaline phosphatase, β-galactosidase, β-glucosidase, horseradish peroxidase, glucoamylase, lysozyme, saccharide oxidases, microperoxidase, biotin and the like. When biotin is used as a labeling substance, it is preferred that a biotin-labeled antibody is added and then avidin conjugated with an enzyme such as alkaline phosphatase is added. The labeling substance may be bound to the anti-C20orf102 antibody by a known method such as a glutaraldehyde method, a maleimide method, a pyridyl disulfide method or a periodate method.

Specifically, a solution containing an anti-C20orf102 antibody is added to a support such as a plate, and the anti-C20orf102 antibody is immobilized on the support. After the plate is washed, the plate is blocked with, for example, BSA, gelatin, albumin or the like in order to prevent unspecific binding of proteins. Then, the plate is washed again and a test sample is added to the plate. After incubation, the plate is washed a labeled anti-C20orf102 antibody is added. After appropriate incubation, the plate is washed, and the labeled anti-C20orf102 antibody remaining on the plate is detected. The detection can be carried out by a method known to those skilled in the art. For example, in the case where the labeling is carried out with a radioisotope, the protein can be detected by liquid scintillation or an RIA method. In the case where the labeling is carried out with an enzyme, a substrate is added, and the enzymatic change of the substrate such as chromogenic change can be detected with an absorption spectrometer. Specific examples of the substrate include 2,2'-azinobis(3-ethylbenzothiazoline-6-sulfonic acid), diammonium salt (ABTS), 1,2-phenylenediamine (o-phenylenediamine), 3,3',5,5'-tetramethylbenzidine (TMB) and the like. In the case of a fluorescent substance, the change can be detected with a spectrofluorometer.

In a particularly preferred embodiment of the method of the present invention, C20orf102 protein can be detected with a biotin-labeled anti-C20orf102 antibody and avidin.

Specifically, a solution containing an anti-C20orf102 antibody is added to a support such as a plate, and the anti-C20orf102 antibody is immobilized thereon. After the plate is washed, the plate is blocked with, for example BSA in order to prevent unspecific binding of proteins. Then, the plate is washed again and a test sample is added to the plate. After incubation, the plate is washed and a biotin-labeled anti-C20orf102 antibody is added. After appropriate incubation, the plate is washed, and avidin bound to an enzyme such as alkaline phosphatase or peroxidase is added to the plate. After incubation, the plate is washed and a substrate corresponding to the enzyme bound to avidin is added, and then C20orf102 protein is detected through the enzymatic change of the substrate as an indicator.

In another embodiment of the method of the present invention, C20orf102 protein can be detected using one or more types of primary antibodies specifically recognizing C20orf102 protein and one or more types of secondary antibodies specifically recognizing the primary antibodies.

For example, a test sample is brought into contact with one or more types of anti-C20orf102 antibodies immobilized on a support, the support is incubated and washed, and then C20orf102 protein bound after washing is detected by the primary anti-C20orf102 antibodies and one or more types of secondary antibodies specifically recognizing the primary antibodies. In this case, the secondary antibodies are preferably labeled with a labeling substance.

In another embodiment of the method of the present invention, C20orf102 protein can be detected by an agglutination reaction. In this method, C20orf102 can be detected using a support sensitized with an anti-C20orf102 antibody. The support sensitized with an anti-C20orf102 antibody may be of any types as long as it is insoluble, does not cause a non-specific reaction, and is stable. Such a support include, for example, latex particles, bentonite, collodion, kaolin, fixed sheep erythrocytes or the like. It is preferred to use latex particles. The latex particles may include polystyrene latex particles, styrene-butadiene copolymer latex particles, polyvinyltoluene latex particles or the like. It is preferred to use polystyrene latex particles. Sensitized particles are mixed with a sample, and stirred for a certain period of time. The degree of agglutination of the particles becomes higher as the concentration of anti-C20orf102 antibody contained in the sample is higher, therefore, C20orf102 can be detected by observing the agglutination with the naked eye. In addition, the protein can be detected by measuring the turbidity due to agglutination with a spectrophotometer.

In another embodiment of the method of the present invention, C20orf102 protein can be detected with a biosensor based on the surface plasmon resonance phenomenon. With the biosensor based on the surface plasmon resonance phenomenon, the protein-protein interaction can be observed with a small amount of unlabeled protein in real time as indicated by a surface plasmon resonance signal. For example, binding of the anti-C20orf102 antibody to C20orf102 protein can be detected with a biosensor such as BIAcore (manufactured by Amersham Biosciences). Specifically, a test sample is brought into contact with a sensor chip on which an anti-C20orf102 antibody has been immobilized, and then C20orf102 protein bound to the anti-C20orf102 antibody can be detected as a change in the resonance signal.

The detection method of the present invention can be automated using various automatic testing device, where a large number of samples can be tested at once.

Also disclosed is a diagnostic drug or kit for detecting C20orf102 protein in a test sample for diagnosis of cancer, where the diagnostic drug or kit contains at least the anti-C20orf102 antibody. In the case where the diagnostic drug or kit is based on an EIA method such as an ELISA method, the drug or kit may contain a support for immobilizing the antibody, or the antibody may be immobilized on the support in advance. In the case where the diagnostic drug or kit is based on the agglutination method using a support such as latex, the drug or kit may contain a support to which the antibody has been adsorbed. In addition, the kit may contain a blocking solution, a reaction solution, a reaction termination solution, a reagent for treating a sample or the like as needed.

### Production of anti-C20orf102 antibody

The anti-C20orf102 antibody to be used in the present invention may be of any origin, of any type (monoclonal or polyclonal), and of any form, as long as it specifically binds to C20orf102 protein. Specifically, any of known antibodies can be used in the invention, such as a mouse antibody, a rat antibody, a human antibody, a chimeric antibody or a humanized antibody. The antibody may be a polyclonal antibody, but is preferably a monoclonal antibody.

Further, an anti-C20orf102 antibody to be immobilized on a support and an anti-C20orf102 antibody to be labeled with a labeling substance may recognize the same epitope of the C20orf102 molecule, but preferably recognize different epitopes. The sites to be recognized are not particularly limited.

The anti-C20orf102 antibody to be used in the present invention can be obtained as a polyclonal antibody or a monoclonal antibody by a known technique. A monoclonal antibody derived from a mammal is particularly preferred as the anti-C20orf102 antibody to be used in the present invention. Examples of the monoclonal antibody derived from a mammal include those produced by a hybridoma and those produced by a host transformed with an expression vector containing a genetically engineered antibody gene.

A monoclonal antibody-producing hybridoma can be principally produced using a known technique as follows. An animal is immunized with C20orf102 as a sensitizing antigen according to a common immunization method. The immunocyte is obtained and fused with a known parent cell by a common cell fusion method. A cell producing a monoclonal antibody is screened by a common screening method.

Specifically, a monoclonal antibody can be produced as follows.

First, C20orf102 to be used as a sensitizing antigen for obtaining an antibody is prepared by expressing C20orf102 gene/amino acid sequence described in GenBank Accession No. NM_080607. More specifically, the gene sequence encoding C20orf102 is inserted into a known vector system, an appropriate host cell is transformed with the vector, and then, a desired human C20orf102 protein of interest is purified by a known method from the host cell or the culture supernatant thereof. Alternatively, C20orf102 protein may be purified from a natural source.

Subsequently, the purified C20orf102 protein is used as a sensitizing antigen. Alternatively, a partial peptide of C20orf102 can be used as a sensitizing antigen. In this case, the partial peptide can be obtained by chemical synthesis based on the an amino acid sequence of human C20orf102, or by introducing a portion of C20orf102 gene into an expression vector, or by digesting native C20orf102 with a protease. Any site and size of C20orf102 may be used as a partial peptide.

Any types of mammal may be immunized with the sensitizing antigen, but is preferably selected in consideration of its compatibility with the parent cell to be used in cell fusion. Generally a mammal include a rodent such as a mouse, a rat or a hamster, or a rabbit, a monkey or the like.

An animal is immunized with a sensitizing antigen using a known method. In a commonly used method, the sensitizing antigen is injected into the mammal intraperitoneally or subcutaneously. Specifically, a sensitizing antigen is diluted and suspended in an appropriate amount of phosphate buffered saline (PBS), physiological saline or the like, and mixed with an appropriate amount of a common adjuvant such as Freund's complete adjuvant as needed. After being emulsified, it is administered to a mammal for several times every 4 to 21 days. Additionally a suitable carrier may be used upon immunization of the sensitizing antigen. In particular, when a partial peptide with a small molecular weight is used as a sensitizing antigen, it is preferred to bind it to a carrier protein such as albumin or keyhole limpet hemocyanin for immunization.

After a mammal is immunized as described above and the increase in the desired antibody level in the serum is observed, the immunocytes are taken out from the mammal and are subjected to cell fusion. Preferred immunocytes include, in particular, the spleen cells.

A mammalian myeloma cell may also be used as a parent cell for cell fusion with the above-mentioned immunocyte. Preferably, known variety cell lines are used as the myeloma cell such as P3 (P3x63Ag8.653) (J. Immunol. (1979) 123, 1548-1550), P3x63Ag8U.1 (Current Topics in Microbiology and Immunology (1978) 81, 1-7), NS-1 (Kohler, G. and Milstein, C. , Eur. J. Immunol. (1976) 6, 511-519), MPC-11 (Margulies, D. H. et al., Cell (1976) 8, 405-415), SP2/0 (Shulman, M. et al., Nature (1978) 276, 269-270), FO (de St. Groth, S. F. et al., J. Immunol. Methods (1980) 35, 1-21) , S194 (Trowbridge, I. S., J. Exp. Med. (1978) 148, 313-323) , and R210 (Galfre, G. et al., Nature (1979) 277, 131-133).

The cell fusion between the immunocyte and the myeloma cell may be carried out principally according to a known method such as a method of Kohler and Milstein et al. (Kohler, G. and Milstein, C., Methods Enzymol. (1981) 73, 3-46).

More specifically, the above-mentioned cell fusion is carried out in a usual nutritional medium in the presence of, for example, a cell fusion-promoting agent. The cell fusion-promoting agent include, for example, polyethyleneglycol (PEG), Sendai virus (HVJ) or the like. An auxiliary agent such as dimethylsulfoxide can also be used to increase the fusion efficiency as needed.

The ratio of the number of the immunocyte to the myeloma cell to be used may be appropriately determined. For example, the number of the immunocyte is preferred to be set at 1 to 10 times that of the myeloma cell. The culture medium to be used in the above-mentioned cell fusion includes culture media suitable for the growth of the above-mentioned myeloma cell line, for example, RPMI 1640 culture medium and MEM culture medium, and a standard culture medium which is used for this type of cell culture. Further, a serum supplement such as fetal calf serum (FCS) may be used in combination.

In cell fusion, predetermined number of above-mentioned immunocytes and myeloma cells are thoroughly mixed in the above-mentioned culture medium, a PEG solution previously heated to about 37°C (for example, an average molecular weight of about 1000 to 6000) is added at a concentration of 30 to 60% (w/v) and mixed to form a desired fusion cell (hybridoma) . Then, the process of sequential addition of an appropriate culture medium, centrifugation and removal of a supernatant is repeated to remove the cell fusion agent and those which are undesirable for the growth of the hybridoma.

The resulting hybridoma is then selected by culturing it in a standard selection culture medium such as HAT culture medium (a culture medium containing hypoxanthine, aminopterin, and thymidine). The cultivation in the above-mentioned HAT culture medium is continued for sufficient time (usually from several days to several weeks) so that cells other than the desired hybridoma (non-fused cells) will die. Then, a hybridoma that produces a desired antibody is screened and monocloned by a standard limiting dilution method.

A desired antibody may be screened and monocloned by a known screening method based on an antigen-antibody reaction. For example, an antigen is bound to a support such as beads made of polystyrene or the like or a commercially available 96-well microtiter plate, then a culture supernatant of hybridoma is added. After the support is washed, an enzyme-labeled secondary antibody or the like is added to determine whether or not a desired antibody reacting with the sensitizing antigen is contained in the culture supernatant. The hybridoma that produces a desired antibody can be cloned by a limiting dilution method or the like. The antigen used for immunization may be used in the screening procedure.

In addition to the above-mentioned method where an animal other than human is immunized with an antigen to obtain a hybridoma, it is also possible to sensitize a human lymphocyte in vitro with C20orf102, and the resulting sensitized lymphocyte is fused with a human myeloma cell having the ability to divide permanently, whereby a desired human antibody having the activity of binding to C20orf102 can be obtained (see JP-B-1-59878). Alternatively, C20orf102 is administered to a transgenic animal having the repertoire of all the genes for human antibody to obtain a cell producing the anti-C20orf102 antibody. The cell is immortalized and a human antibody against C20orf102 may be obtained from the immortalized cell (see International Patent Application Nos. WO 94/25585, WO 93/12227, WO 92/03918 and WO 94/02602).

The thus prepared hybridoma that produces a monoclonal antibody can be subcultured in a standard culture medium, or can be stored for a long period of time in liquid nitrogen.

In order to obtain a monoclonal antibody from the hybridoma, the hybridoma is cultured according to a standard method and an antibody is obtained as the culture supernatant. alternatively, the hybridoma is administered to and grown in a mammal compatible with the hybridoma and an antibody is obtained as the ascites or the like. The former method is suitable for obtaining high-purity antibodies, whereas the latter is suitable for mass production of antibodies.

According to the present invention, a recombinant monoclonal antibody produced by genetic engineering techniques can also be used as a monoclonal antibody. The antibody gene is cloned from the hybridoma, introduced into an appropriate vector and introduced into the host cell to produce a recombinant-type monoclonal antibody (see, for example, Vandamme, A. M. et al., Eur. J. Biochem. (1990) 192, 767-775, 1990). Specifically, mRNA encoding the variable (V) region of the anti-C20orf102 antibody is isolated from the hybridoma producing the anti-C20orf102 antibody. The isolation of mRNA is carried out by a known method such as guanidine ultracentrifugation (Chirgwin, J. M. et al. Biochemistry (1979) 18, 5294-5299) or the AGPC method (Chomczynski, P. et al., Anal. Biochem. (1987) 162, 156-159) to prepare total RNA, and then a desired mRNA is prepared by using an mRNA Purification Kit (manufactured by Pharmacia). Alternatively, mRNA can be directly prepared by using a QuickPrep mRNA Purification Kit (manufactured by Pharmacia).

cDNA coding for the variable (V) region of the antibody is synthesized from the resulting mRNA by using a reverse transcriptase. The synthesis of the cDNA is carried out by using AMV Reverse Transcriptase First-strand cDNA Synthesis Kit (manufactured by Seikagaku Kogyo) or the like. Alternatively, cDNA may be synthesized and amplified by the 5'-RACE method (Frohman, M. A. et al., Proc. Natl. Acad. Sci. USA (1988) 85, 8998-9002, Belyavsky, A. et al., Nucleic Acids Res. (1989) 17, 2919-2932) using a 5'-Ampli FINDER RACE Kit (manufactured by Clontech), PCR and the like.

The desired DNA fragment is purified from the resulting PCR product and ligated with a vector DNA. Then a recombinant vector is constructed therefrom and introduced into E. coli or the like, and a colony is selected, whereby a desired recombinant vector is prepared. The nucleotide sequence of the desired DNA is checked by a known method such as the dideoxy nucleotide chain termination method.

Once the desired DNA encoding the V region of the anti-C20orf102 antibody is obtained, and the DNA is incorporated integrated into an expression vector containing DNA encoding the constant region (C region) of a desired antibody.

In order to produce the anti-C20orf102 antibody to be used in the present invention, the antibody gene is incorporated into an expression vector so as to be expressed under the control of the expression regulatory region, for example, an enhancer or a promoter. Subsequently, a host cell is transformed with the expression vector, and the antibody is expressed in the cell.

The antibody gene may be expressed in the cell by separately introducing DNAs encoding the heavy chain (H chain) and the light chain (L chain) of the antibody into expression vectors and co-transforming a host cell with the vectors; or by introducing DNAs encoding the H chain and the L chain into a single expression vector and transforming a host cell with the vector (see WO 94/11523).

In addition to the above-mentioned host cell, a transgenic animal can be used for the production of a recombinant antibody. For example, an antibody gene is inserted into the middle of a gene encoding a protein produced specifically into milk (such as goat β-casein) to prepare a fusion gene. A DNA fragment containing the fusion gene comprising the antibody gene is injected into a goat's embryo, which is then introduced into a female goat. A desired antibody can be obtained from milk produced by a transgenic goat which is born from the goat that had received the embryo or offspring thereof. To increase the amount of milk containing the desired antibody produced by the transgenic goat, an appropriate hormone may be administered to the transgenic goat (Ebert, K.M. et al., Bio/Technology (1994) 12, 699-702).

In addition to the above-mentioned antibodies, an artificially modified genetic recombinant-type antibody, such as a chimeric antibody or a humanized antibody can be used in the present invention. Such a modified antibody can be produced by using a known method.

The chimeric antibody may be obtained by ligating the DNA encoding the V region of the antibody obtained as described above with DNA encoding the C region of a human antibody, introducing the resulting DNA into an expression vector, and introducing the vector into a host for production of the antibody. By using this known method, a chimeric antibody useful for the present invention can be obtained.

A humanized antibody, also referred to as a "reshaped humane antibody", is obtained by grafting the complementarity determining region (CDR) of an antibody from a non-human mammal, such as a mouse, into the complementarity determining region of a human antibody. A general technique of genetic recombination is also known in the art (see European Patent Application EP125023 and WO 96/02576).

Specifically, a DNA sequence designed to ligate a mouse antibody CDR to the framework region (FR) of a human antibody is synthesized by PCR using as primers several oligonucleotides constructed to have overlapping portions at the ends of both CDR and FR (see the method described in WO 98/13388).

The framework region of the human antibody to be ligated via the CDR is selected such that the complementarity determining region will form a favorable antigen-binding site. As necessary, amino acids in the framework region of an antibody variable region may be substituted, so that the complementarity determining region of a reshaped human antibody forms an appropriate antigen-binding site (Sato, K. et al., Cancer Res. (1993) 53, 851-856).

C regions from the human antibody is used as the C region in the chimeric antibody or the humanized antibody. For example, Cγ1, Cγ2, Cγ3 or Cγ4 can be used for the H chain, and Cκ or Cλ can be used for the L chain. The C region of the human antibody may be modified in order to improve the stability of the antibody itself or the production process.

A chimeric antibody is composed of the variable region of an antibody derived from a non-human mammal and the constant region derived from a human antibody. On the other hand, a humanized antibody is composed of the complementarity determining region of an antibody derived a non-human mammal, and the framework region and the constant region derived from a human antibody. Since the antigenicity of the humanized antibody is expected to be reduced in human body, the humanized antibody is useful as an active ingredient of a therapeutic agent of the present invention.

The antibody to be used in the present invention is not limited to the whole antibody molecule and may be a fragment of the antibody or a modified fragment thereof as long as it binds to C20orf102. It includes a divalent antibody and a monovalent antibody. Examples of the fragment of the antibody include Fab, F(ab')2, Fv, Fab/c having one Fab and a full Fc, and a single chain Fv (scFv) where the Fv of the H chain and the L chain are linked via an appropriate linker. Specifically, an antibody is treated with an enzyme such as papain or pepsin to provide a fragment of the antibody. Alternatively, a gene encoding such an antibody fragment is constructed and introduced into an expression vector, and the antibody fragment is expressed in a suitable host cell (see, for example, Co, M. S. et al., J'. Immunol. (1994) 152, 2968-2976, Better, M. & Horwitz, A. H. Methods in Enzymology (1989) 178, 476-496, Academic Press, Inc., Plueckthun, A. & Skerra, A. Methods in Enzymology (1989) 178, 476-496, Academic Press, Inc., Lamoyi, E., Methods in Enzymology (1989) 121, 652-663, Rousseaux, J. et al., Methods in Enzymology (1989) 121, 663-669, Bird, R. E. et al., TIBTECH (1991) 9, 132-137).

The scFv can be obtained by linking the H chain V region and the L chain V region of an antibody. In the scFv, the H chain V region and the L chain V region are preferably linked via a linker, preferably a peptide linker (Huston, J. S. et al., Proc. Natl. Acad. Sci. U.S.A. (1988) 85, 5879-5883). The H chain V region and the L chain V region in scFv may be derived from any antibody described as an antibody in this specification. For example, any single chain peptide having 12 to 19 amino acid residues may be used as the peptide linker for ligating the V regions.

DNA encoding scFv can be obtained by amplifying a fragment by PCR using as a template a DNA portion encoding all or a desired amino acid sequence of the sequences of DNA encoding the H chain or the H chain V region of the above-mentioned antibody and DNA encoding the L chain or the L chain V region of the above-mentioned antibody with a primer pair that defines the both ends thereof. Then the fragment is amplified with a combination of DNA encoding a peptide linker portion and a primer pair which defines both ends to be ligated to the H chain and the L chain.

Once DNA encoding scFv is prepared, an expression vector containing the DNA and a host cell transformed with the expression vector can be obtained according to a standard method. The scFv can be obtained from such a host according to a standard method.

These antibody fragments can be produced in a host by obtaining the gene thereof in the same manner as described above and by allowing it to be expressed. The term "antibody" in the present invention also encompasses these antibody fragments.

A modified antibody, for example, an anti-C20orf102 antibody conjugated with any of a variety of molecules such as a labeling substance can also be used in the invention. The term "antibody" in the present invention also encompasses such a modified antibody. Such a modified antibody can be obtained by chemically modifying the antibody obtained as above. Methods of modifying an antibody have already been established in the art.

Further, the antibody to be used in the present invention may be a bispecific antibody. The bispecific antibody may have antigen-binding sites that recognize different epitopes on the C20orf102 molecule. Alternatively, one of which may recognize C20orf102, and the other may recognize a labeling substance or the like. The bispecific antibody can also be produced by ligating an HL pair of two types of antibodies, or by fusing hybridomas producing different monoclonal antibodies to provide a fusion cell producing the bispecific antibody. Furthermore, the bispecific antibody can also be produced by genetic engineering techniques.

Antibodies can be expressed from the antibody gene constructed as described above by a known method. In the case of a mammalian cell, the gene can be expressed by operable linking a conventional useful promoter, an antibody gene to be expressed and a poly A signal at the 3'-downstream of the gene. A promoter/enhancer includes, for example, a human cytomegalovirus immediate early promoter/enhancer.

Further, examples of the promoter/enhancer used for expressing antibodies to be used in the present invention include, for example, viral promoter/enhancers such as retrovirus, polyoma virus, adenovirus and simian virus 40 (SV40), mammalian promoter/enhancers such as human elongation factor 1α (HEF1α).

Antibodies can be readily expressed by the method of Mulligan et al. (Nature (1979) 277, 108) when SV40 promoter/enhancer is used, and by the method of Mizushima et al. (Nucleic Acids Res. (1990) 18, 5322) when HEF1α promoter/enhancer is used.

In the case of E. coli, the gene can be expressed by operably linking a conventional useful promoter, a signal sequence for antibody secretion and an antibody gene to be expressed. A promoter includes, for example, lacZ promoter and araB promoter. The gene can be expressed by the method of Ward et al. (Nature (1989) 341, 544-546; FASEB J. (1992) 6, 2422-2427) when the lacZ promoter is used, and by the method of Better et al. (Science (1988) 240, 1041-1043) when the araB promoter is used.

A signal sequence for antibody secretion may be used for producing the antibody in the periplasm of E. coli, such as pelB signal sequence (Lei, S. P. et al., J. Bacteriol. (1987) 169, 4379). After isolating the antibody produced in the periplasm, the antibody is appropriately refolded for use.

A replication origin may be derived from SV40, polyoma virus, adenovirus, bovine papilloma virus (BPV). To amplify the gene copy number in a host cell system, the expression vector may contain as a selection marker the aminoglycoside transferase (APH) gene, the thymidine kinase (TK) gene, the E. coli xanthine guaninephosphoribosyl transferase (Ecogpt) gene, the dihydrofolate reductase (dhfr) gene or the like.

Any expression system, for example, a eukaryotic cell or a prokaryotic cell can be used for producing the antibody to be used in the present invention. Examples of the eukaryotic cell include established animals cells such as mammalian cells, insect cells, filamentous fungus cells, and yeast cells and the like. Examples of the prokaryotic cell include bacteria cells such as E. coli cells.

The antibody to be used in the present invention is preferably expressed in a mammalian cell such as a CHO, COS, myeloma, BHK, Vero, or Hela cell.

Subsequently, the transformed host cell is cultured in vitro or in vivo to produce a desired antibody. The host cell may be cultured according to a known method. For example, DMEM, MEM, RPMI1640 and IMDM can be used as a culture medium, and a serum supplement such as fetal calf serum (FCS) may be used in combination.

The thus expressed and produced antibody can be isolated from the cell or the host animal and purified to homogeneity. The isolation and purification of the antibody to be used in the present invention can be carried out by using an affinity column. Examples of a Protein A column include Hyper D, POROS, Sepharose F. F. (manufactured by Pharmacia). Any other standard methods for isolation and purification of proteins may be used in the invention. For example, the antibody can be isolated and purified by appropriately selecting and combining chromatography columns, besides the above-mentioned affinity columns, filters, ultra filtration, salting-out, dialysis and the like (Antibodies A Laboratory Manual, Ed Harlow, David Lane, Cold Spring Harbor Laboratory, 1988).

### Cancer-associated gene as described herein

The cancer-associated gene identified in the present invention is shown in Table 1 with its name, a cancer tissue in which the expression of the gene is elevated, and the SEQ ID NO of the sequence of the gene and the sequence of a protein encoded by the gene.

TEG1 (SEQ ID NO: 2; SEQ ID NO: 66) encodes C20orf102. The GenBank accession number of the gene is AA206763 (reference sequence ID: NM_080607). It was found that the expression of the gene is elevated in lung cancer, moderately differentiated liver cancer and pancreatic cancer. It is not known that the expression of the gene is associated with cancer.

### Examples

Hereinafter, the present invention will be described in more detail with reference to Examples, however, these Examples are not to be construed to limit the scope of the invention.

### Example 1

### Identification of gene whose expression is elevated in human cancer tissue

In order to identify a gene whose expression is elevated in each type of human cancer tissue (lung adenocarcinoma, stomach cancer, large bowel cancer, hepatocellular carcinoma and brain tumor) compared with that in normal tissue, an expression analysis of mRNA was carried out in each type of excised human cancer tissue using GeneChip (GeneChip^{™} HG-133A, B Target; manufactured by Affymetryx).

### 1.1. Identification of gene whose expression is elevated in human lung adenocarcinoma

In order to identify a gene whose expression is elevated in human lung adenocarcinoma compared with that in normal human lung tissue, an expression analysis of mRNA was carried out as follows.

First, total RNA was prepared from the cancerous part of 12 cases of excised lung adenocarcinoma tissue including different differentiation degrees and stages, and one case of normal lung using ISOGEN (Nippon Gene) according to the attached method. Then, mRNA expression in the lung adenocarcinoma and normal lung was analyzed using a GeneChip^{™} HG-U133A, B (manufactured by Affymetryx). More specifically, 5 µg of a material obtained by mixing the respective total RNA prepared from the 12 cases in an equal amount as for the cancerous part, or 5 µg of the total RNA prepared from the one case of normal lung as a control was analyzed for gene expression according to Expression Analysis Technical Manual (Affymetryx). The mean value of expression scores of all genes in each analysis was defined as 100 and the expression level of each gene was indicated as a relative value.

### 1.2. Identification of gene whose expression is elevated in human stomach cancer

In order to identify a gene whose expression is elevated in human stomach cancer compared with that in normal human stomach tissue, an expression analysis of mRNA was carried out in the same manner as described above.

Total RNA was prepared from 3 cases of excised stomach cancer tissue and one case of normal stomach in the same manner as described above. Five micrograms of a material obtained by mixing the respective total RNA prepared from the 3 cases in an equal amount as for the cancerous part, or 5 µg of the total RNA prepared from the one case of normal stomach as a control was analyzed for mRNA expression using a Genedhip^{™} HG-U133A, B (manufactured by Affymetryx). The mean value of expression scores of all genes in each analysis was defined as 100 and the expression level of each gene was indicated as a relative value.

### 1.3. Identification of gene whose expression is elevated in human large bowel cancer

Identification of a gene whose expression is elevated in human large bowel cancer compared with that in normal human large bowel tissue was carried out in the same manner as described above.

Total RNA was prepared from the cancerous part of 3 cases of excised large bowel cancer tissue and one case of normal large bowel tissue in the same manner as described above. Five micrograms of a material obtained by mixing the respective total RNA prepared from the 3 cases in an equal amount as for the cancerous part, or 5 µg of the total RNA prepared from the one case of normal large bowel as a control was analyzed for mRNA expression using a GeneChip^{™} HG-U133A, B Target (manufactured by Affymetryx). The mean value of expression scores of all genes in each analysis was defined as 100 and the expression level of each gene was indicated as a relative value.

### 1.4. Identification of gene whose expression is elevated in human hepatocellular carcinoma

Identification of a gene whose expression is elevated in human hepatocellular carcinoma compared with that in normal human liver was carried out in the same manner as described above.

Total RNA was prepared from the cancerous part of 3 cases of hepatitis C virus infected moderately differentiated hepatocellular carcinoma, 3 cases of hepatitis C virus infected poorly differentiated hepatocellular carcinoma and one case of normal liver tissue in the same manner as described above. Five micrograms of a material obtained by mixing the respective total RNA prepared from the 3 cases in an equal amount as for each cancerous part of the different differentiation degrees, or 5 µg of the total RNA prepared from the one case of the normal liver as a control was analyzed for mRNA expression using a GeneChip^{™} HG-U133A, B (manufactured by Affymetryx). The mean value of expression scores of all genes in each analysis was defined as 100 and the expression level of each gene was indicated as a relative value.

### 1.5. Identification of gene whose expression is elevated in human glioblastoma

Identification of a gene whose expression is elevated in human glioblastoma compared with that in normal human brain tissue was carried out in the same manner as described above.

Total RNA was prepared from the cancerous part of 5 cases of excised glioblastoma tissue and one case of normal brain tissue in the same manner as described above. Five micrograms of a material obtained by mixing the respective total RNA prepared from the 5 cases in an equal amount as for the cancerous part, or 5 µg of the total RNA prepared from the one case of the normal brain tissue as a control was analyzed for mRNA expression using a GeneChip^{™} HG-U133A, B (manufactured by Affymetryx). The mean value of expression scores of all genes in each analysis was defined as 100 and the expression level of each gene was indicated as a relative value.

From the results of the above analyses, it was found that mRNA expression of the genes shown in Table 2 is elevated compared with that in each of the corresponding normal tissue.

**Table 2**

| No. | Name | Cancer type in which expression is elevated | Gene chip analysis results | | | | | | | | | | |
|---|---|---|---|---|---|---|---|---|---|---|---|---|---|
| | | | Lung | Lung cancer | Stoma ch | Stoma ch cancer | Large bowel | Large bowel cancer | Liver | Moderately differentiated liver cancer | Poorly differentiated liver | Brain | Gliobl astoma |
| TEG1 | C20orf102 | Lung cancer, Moderately differentiated liver cancer | 32 | 299.3 | 98.6 | 50.3 | 95.2 | 18.4 | 39.1 | 104.9 | 13 | 834.4 | 90.8 |
| TEG2 | ASCL2 | Stomach cancer, Large bowel cancer | 66.1 | 27.4 | 6 | 406.9 | 79.1 | 738.8 | 19.5 | 5.9 | 31.2 | 3.6 | 10.7 |
| TEG3 | EST | Stomach cancer Moderately differentiated liver cancer | 65.1 | 74.6 | 92.1 | 440.8 | 112.9 | 107.6 | 142.3 | 216 | 164.4 | 53.2 | 86.9 |
| TEG4 | EST | Stomach cancer, Large bowel cancer | 50.9 | 25.1 | 41.4 | 117.2 | 52.5 | 106.8 | 12 | 38.5 | 12.5 | 31.2 | 61.1 |
| TEG5 | EST | Stomach cancer | 79.7 | 85 | 58.7 | 248.2 | 73.9 | 63.5 | 11.3 | 59.7 | 96.9 | 44 | 87.2 |
| TEG6 | OK/SW-CL.. 30 | Lung cancer, Stomach cancer, Large bowel cancer, Moderately differentiated liver cancer | 84.1 | 118.9 | 55.6 | 537.1 | 98.5 | 734.1 | 157.7 | 1781. 4 | 160.8 | 78.7 | 106 |
| TEG7 | DKFZp686L 1533 | Lung cancer, Stomach cancer, Large bowel cancer, Moderately or poorly differentiated liver | 79.2 | 173.3 | 14.6 | 588.5 | 89.2 | 750.3 | 22.7 | 158 | 309.6 | 15.5 | 87.1 |
| TEG8 | EST | Stomach cancer, Poorly differentiated liver cancer | 59.1 | 50.8 | 37.5 | 260.7 | 36.3 | 22.7 | 58.7 | 26.8 | 120 | 68.7 | 17.4 |
| TEG9 | LOC93082 | Stomach cancer, Poorly differentiated liver cancer | 107.3 | 34.5 | 14.5 | 1030. 1 | 89.2 | 21.9 | 130 | 155.6 | 448.1 | 18.5 | 105.4 |
| TEG10 | EST | Stomach cancer, Moderately or poorly differentiated liver cancer | 38.1 | 37.8 | 32.8 | 385.8 | 20.3 | 20.5 | 28.2 | 103.9 | 356.5 | 60.2 | 63.5 |
| TEG11 | FLJ11041 | Stomach cancer, Large bowel cancer, Moderately differentiated liver cancer | 607.1 | 481.8 | 16.9 | 261.5 | 19.2 | 522.1 | 97.9 | 128.1 | 56.2 | 43.2 | 49.2 |
| TEG12 | EST | Liver cancer | 60.8 | 65.2 | 91 | 38.6 | 44.5 | 62.2 | 16.2 | 194.3 | 527 | 66.2 | 47.7 |
| TEG13 | EST | Liver cancer | 38 | 10.3 | 35.5 | 17 | 16.3 | 4.6 | 26.1 | 493.7 | 177.7 | 4.6 | 14.7 |
| TEG14 | ASPM | Stomach cancer, Large bowel cancer, Liver cancer | 1.3 | 45.1 | 3.8 | 107.3 | 18.2 | 99.6 | 3.6 | 111.3 | 246.1 | 1.5 | 83.8 |
| TEG15 | Sp5 | Stomach cancer, Large bowel cancer, Liver cancer | 8 | 15.8 | 57.9 | 219.2 | 14.5 | 270.1 | 11.2 | 288.7 | 219.2 | 12 | 6.8 |
| TEG16 | IMAGE:2974 03 | Liver cancer | 5.7 | 12.7 | 25 | 11.5 | 20.2 | 17.8 | 34.4 | 273.1 | 159.7 | 16.2 | 66.8 |
| TEG17 | DKFZp434K 2435 | Stomach cancer, Large bowel cancer | 11.1 | 5.9 | 16.1 | 183.1 | 16.6 | 98.1 | 8.4 | 17.3 | 9.5 | 14.5 | 18.8 |
| TEG18 | CBRC7TM_ 249 | Stomach cancer, Large bowel cancer, Moderately or poorly differentiated liver cancer | 13.6 | 86.6 | 45 | 240.4 | 19.6 | 175.4 | 158.8 | 669 | 949 | 13.5 | 47.9 |
| TEG19 | R1 | Lung cancer | 23.6 | 254.4 | 17.1 | 5.3 | 18 | 4.3 | 133.6 | 77.4 | 21.2 | 21.8 | 111.8 |
| TEG20 | C20orf54 | Stomach cancer, Large bowel cancer, Liver cancer | 21.7 | 69.6 | 22.8 | 261.1 | 22.4 | 50.5 | 8.4 | 8.2 | 24.4 | 6.6 | 15.5 |
| TEG21 | RHBG | Liver cancer | 8.7 | 13.4 | 19.1 | 5.4 | 15.6 | 8.6 | 17.4 | 792.6 | 57.1 | 15.5 | 9.3 |
| TEG22 | COPG2 | Large bowel cancer | 77 | 66.9 | 83.1 | 47.5 | 21.7 | 178.4 | 52.8 | 8.7 | 22.6 | 40.9 | 78.7 |
| TEG23 | EST | Poorly differentiated liver cancer | 35.1 | 81.2 | 2 | 21.1 | 28 | 15.9 | 9.3 | 33.7 | 539.9 | 22.9 | 42.2 |
| TEG24 | EST | Stomach cancer | 28.9 | 19.4 | 35.6 | 197.1 | 44.8 | 80.1 | 5.2 | 15.5 | 31.1 | 57.6 | 58.8 |
| TEG25 | GPR49 | Stomach cancer, Large bowel cancer, Moderately differentiated liver cancer | 23.9 | 15.8 | 24.3 | 538.3 | 41.6 | 135.3 | 16.7 | 233.8 | 78.8 | 33.5 | 11.2 |
| TEG26 | MUC17 | Stomach cancer | 73.4 | 59.1 | 89.4 | 565.2 | 113.3 | 102.8 | 34.7 | 67.6 | 113.8 | 100 | 56.3 |
| TEG27 | EphB2 | Stomach cancer, Large bowel cancer | 23.2 | 47.5 | 6.8 | 218.7 | 62.8 | 189.4 | 6.6 | 55.1 | 13.6 | 28.7 | 49 |
| TEG28 | FLJ11856/G PCR41 | Stomach cancer, Large bowel cancer | 22.2 | 35.2 | 9.1 | 229.5 | 63.8 | 197.5 | 2.7 | 5.1 | 67.3 | 4.4 | 78.3 |
| TEG29 | HS6ST2 | Stomach cancer, Large bowel cancer, Poorly differentiated liver cancer | 20.8 | 472.6 | 3.6 | 2.3 | 37.2 | 164.9 | 4.5 | 6.5 | 191.4 | 104 | 69 |
| TEG30 | PCDHB2 | Lung cancer | 11.9 | 228.5 | 55.2 | 37.7 | 32.2 | 58.9 | 14.4 | 13.4 | 27.7 | 80.4 | 78 |
| TEG31 | WFDC3 | Lung cancer | 30.1 | 304.2 | 110.6 | 28.7 | 32.2 | 27.8 | 46.4 | 29.9 | 30.4 | 28.7 | 28.9 |
| TEG32 | C20orf42 | Lung cancer, Stomach cancer, Large bowel cancer | 11.6 | 43.8 | 127.7 | 365.4 | 175.8 | 535.2 | 7 | 17.3 | 44 | 23.5 | 15.4 |
| TEG33 | PIGR | Lung cancer | 63.2 | 382.6 | 129.9 | 149.3 | 520.1 | 423.7 | 102.3 | 101.8 | 96 | 65.2 | 77.7 |
| TEG34 | NFE2L3 | Stomach cancer, Large bowel cancer | 37 | 62.4 | 55.2 | 144.9 | 22 | 216.8 | 27.4 | 18.6 | 37.3 | 13.7 | 27.8 |
| TEG35 | TRAG3 | Stomach cancer | 1.8 | 1.7 | 1.9 | 74.4 | 1.2 | 1.3 | 1.7 | 1.6 | 1.4 | 1.4 | 1.9 |
| TEG36 | TRIM31 | Stomach cancer | 16.9 | 13.2 | 14.6 | 155.2 | 67.3 | 52.7 | 21 | 41.4 | 31 | 4.6 | 26.8 |
| TEG37 | KIAA1359 | Stomach cancer, Lung cancer, Large bowel cancer | 22.8 | 190.3 | 7.5 | 521.1 | 196.8 | 196.7 | 37.9 | 5.7 | 9.1 | 3.5 | 40.6 |
| TEG38 | ubiquitinD | Stomach cancer, Large bowel cancer, Lung cancer, Moderately or poorly differentiated liver cancer | 89.7 | 311.5 | 44.2 | 1172. 8 | 60.1 | 605.7 | 269.2 | 1460. 9 | 2542. 8 | 42.1 | 69 |
| TEG39 | Hephaestin | Stomach cancer | 97.6 | 97.3 | 75.8 | 341.5 | 568.8 | 419.1 | 34.6 | 50.6 | 27 | 126.1 | 91.6 |
| TEG40 | KIAA0152 | Stomach cancer, Large bowel cancer, Glioblastoma | 32.5 | 82.1 | 36.2 | 214.9 | 58.2 | 233.5 | 25.1 | 45.8 | 94 | 22.6 | 109.4 |
| TEG41 | KIAA0703 | Stomach cancer | 84.6 | 46.3 | 20.1 | 214.3 | 195.3 | 77.4 | 13.1 1 | 3.5 | 4.7 | 24.9 | 5.9 |
| TEG42 | MEST/PEG1 | Stomach cancer, Large bowel cancer | 235.9 | 406.2 | 92.6 | 524.3 | 178.4 | 640.8 | 423 | 248.4 | 455.9 | 207.2 | 771.4 |
| TEG43 | KIAA1199 | Stomach cancer, Lung cancer, Large bowel cancer | 53.6 | 162.4 | 26.2 | 80.7 | 28.9 | 185 | 68.5 | 63.5 | 44.3 | 89.1 | 69.4 |
| TEG44 | ELOVL2 | Liver cancer, Glioblastoma | 10.1 | 0.8 | 2.8 | 3 | 15.5 | 1.9 | 68.8 | 224.9 | 233.5 | 76.5 | 121.2 |
| TEG45 | ROBO1 | Liver cancer, Glioblastoma | 58.5 | 49.1 | 32.4 | 38 | 21.4 | 123.2 | 9.1 | 236.4 | 563 | 64.3 | 152.3 |
| TEG46 | FLJ10504/ml sato | Liver cancer | 53.8 | 38.8 | 6.5 | 49.5 | 5.6 | 21.5 | 5.1 | 105.2 | 106.8 | 27.4 | 41.6 |
| TEG47 | cystatin SN | Large bowel cancer | 2.7 | 53.6 | 4.4 | 98.1 | 9.4 | 804.5 | 6.1 | 27.6 | 24.1 | 15.5 | 2.3 |
| TEG48 | LOC116238 | Stomach cancer, Large bowel cancer, Lung cancer, Poorly differentiated liver cancer | 6.9 | 159.3 | 45.6 | 122.8 | 10.1 | 136.9 | 43.3 | 63.2 | 220.2 | 80 | 60.5 |
| TEG49 | MRPL50 | Stomach cancer, Large bowel cancer, Moderately or poorly differentiated liver cancer, Glioblastoma | 77.8 | 86.1 | 98.1 | 191.2 | 43.8 | 256.5 | 72 | 155.3 | 200.8 | 47.7 | 100 |
| TEG50 | TOP1MT | Large bowel cancer, Poorly differentiated liver cancer | 16.5 | 30.8 | 19.1 | 49.7 | 31.3 | 206.4 | 24.9 | 31.9 | 306.2 | 25.5 | 19 |
| TEG51 | FKSG14 | Stomach cancer, Large bowel cancer | 23.1 | 338.1 | 11.1 | 114.8 | 32.2 | 165 | 14 | 37.8 | 31.9 | 2.6 | 82.6 |
| TEG52 | CDH3 | Lung cancer, Stomach cancer, Large bowel cancer | 24.1 | 172.5 | 5.8 | 64.5 | 5.4 | 131.3 . | 4.1 | 3.7 | 2.3 | 14.1 | 5.9 |
| TEG53 | NRP2 | Lung cancer | 26.4 | 171.1 | 40.4 | 25.8 | 88 | 79.1 | 89.9 | 19.1 | 43.6 | 22.4 | 155.2 |
| TEG54 | CLDN3 | Stomach cancer, Lung cancer | 3.2 | 147.6 | 0.8 | 624.4 | 1206. 9 | 738.3 | 40.2 | 42.3 | 4.1 | 1.8 | 0.6 |
| TEG55 | CLDN4 | Stomach cancer, Lung cancer | 70.1 | 193.6 | 3.9 | 364.8 | 258.4 | 325.8 | 7.1 | 37.4 | 45.4 | 3.3 | 2.5 |
| TEG56 | SFRP4 | Lung cancer, Stomach cancer, Glioblastoma | 153.6 | 244.9 | 66.9 | 153.1 | 69.4 | 87.8 | 51.1 | 49.2 | 49.3 | 53.4 | 250.3 |
| TEG57 | ASPSCR1 | Liver cancer | 42.4 | 45.4 | 41.5 | 75.1 | 28.4 | 102.3 | 58.3 | 285.1 | 78.3 | 46.1 | 44.5 |
| TEG58 | GAGEC1 | Liver cancer | 6.1 | 17.9 | 31.7 | 4.2 | 4.8 | 11.6 | 5.8 | 2014. 7 | 45.9 | 8.2 | 12.1 |
| TEG59 | RHAMM | Stomach cancer, Large bowel cancer, Liver | 19.6 | 46.1 | 35.6 | 115.3 | 36.2 | 158.6 | 10.6 | 103.2 | 84.5 | 7.4 | 55.4 |
| TEG60 | PEG10 | Uver cancer, Lung cancer, Hepatoblastoma | 42.9 | 216.9 | 45.7 | 21.4 | 28.6 | 36.7 | 40.6 | 389.8 | 174.7 | 80.9 | 64.5 |
| TEG61 | PAEP | Lung cancer | 4.1 | 96.4 | 9.6 | 7.5 | 6.4 | 5.5 | 4.4 | 6.2 | 6 | 6.5 | 4.4 |
| TEG62 | MGC10981 | Lung cancer | 58.1 | 459 | 59.7 | 44.9 | 91 | 71.6 | 98.6 | 87.7 | 8.1 | 56.8 | 34 |
| TEG63 | DUSP9 | Liver cancer | 20 | 33.7 | 25.9 | 28.9 | 30.9 | 24 | 46.4 | 212.7 | 687 | 49.4 | 24.1 |
| TEG64 | EST1B | Liver cancer | 52.6 | 18.7 | 20.8 | 34.9 | 24.3 | 25.5 | 16 | 82 | 83.2 | 24.2 | 42.3 |

In particular, as for TEG1 to TEG18, an elevation in their expression in any type of cancer cells had not been found so far and it was shown in this study that their expression is elevated in a certain type of cancer. In addition, as for each gene of TEG19 to TEG60, it was now found that their expression is elevated in another type of cancer other than those reported before.

### 1.6. Identification of gene whose expression is elevated in each type of cancer tissue

The expression analysis of each gene of TEG1 to Ten64 in each type of cancer was carried out using a GeneChip^{™} HG-U133A, B (manufactured by Affymetryx) and a GeneChip^{™} HG-U133 plus 2 (manufactured by Affymetryx). More specifically, total RNA was prepared in the same manner as described above for each of the specimens from 10 cases of lung small cell lung cancer, 5 cases of lung squamous cell carcinoma, 5 cases of lung adenocarcinoma, 7 cases of large bowel cancer, 8 cases of metastatic tissue in liver from large bowel cancer, 2 cases of renal cancer and 4 cases of pancreatic cancer. Then, 5 µg of the total RNA was analyzed for mRNA expression with a GeneChip^{™} HG-U133A, B. The lung small cell cancer, large bowel cancer and Some samples from metastatic tissue in liver from large bowel cancer were analyzed only by a U-133A chip. The mean value of expression scores of all genes in each analysis was defined as 100 and the expression level of each gene was indicated as a relative value. In addition, samples from 22 cases of small cell cancer and 27 cases of pancreatic cancer were analyzed in the same manner using a GeneChip^{™} HG-U133 plus 2.

The results are shown in Tables 3 and 4. It was found that the expression of each gene of TEG1 to TEG 64 is elevated also in each type of cancer.

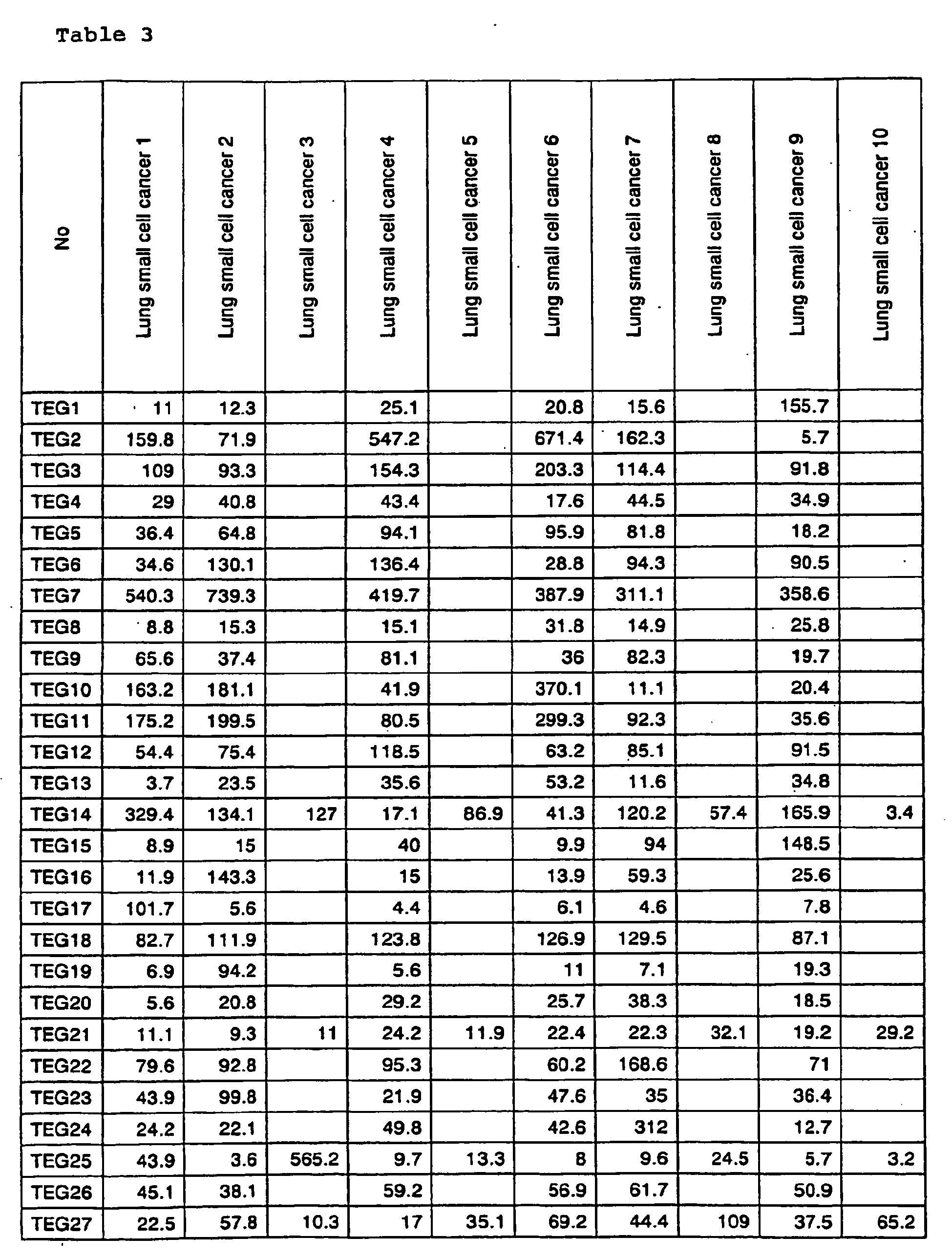

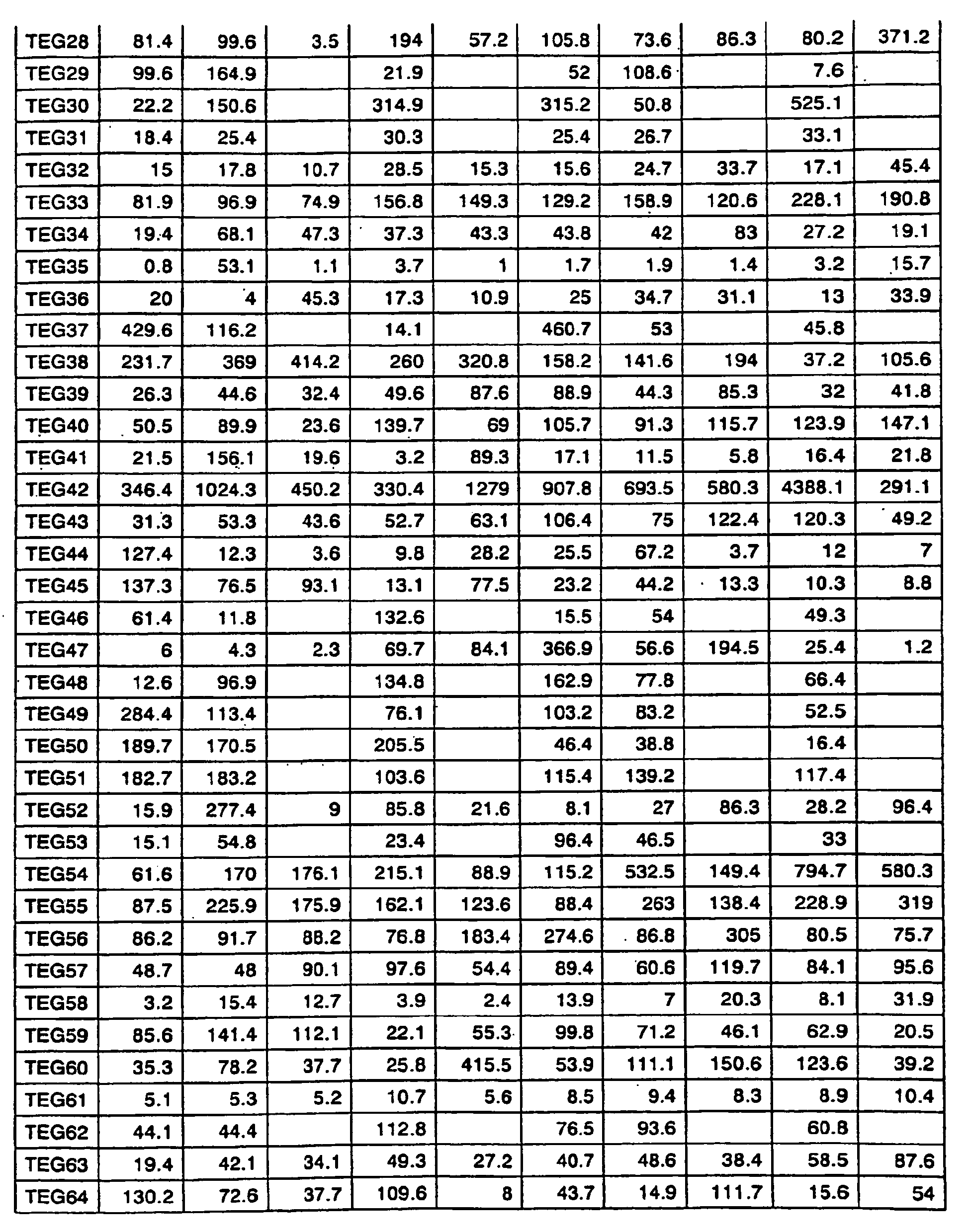

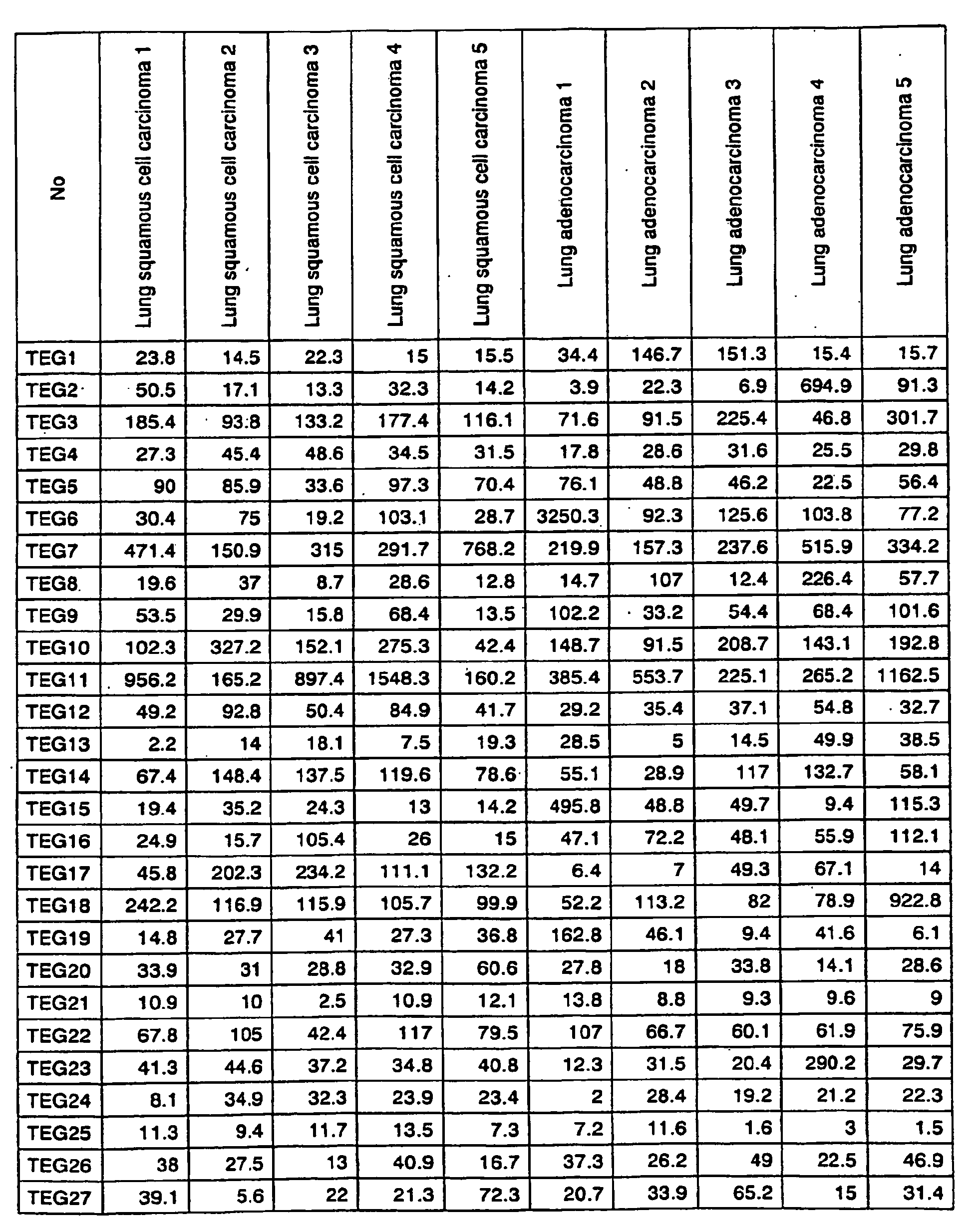

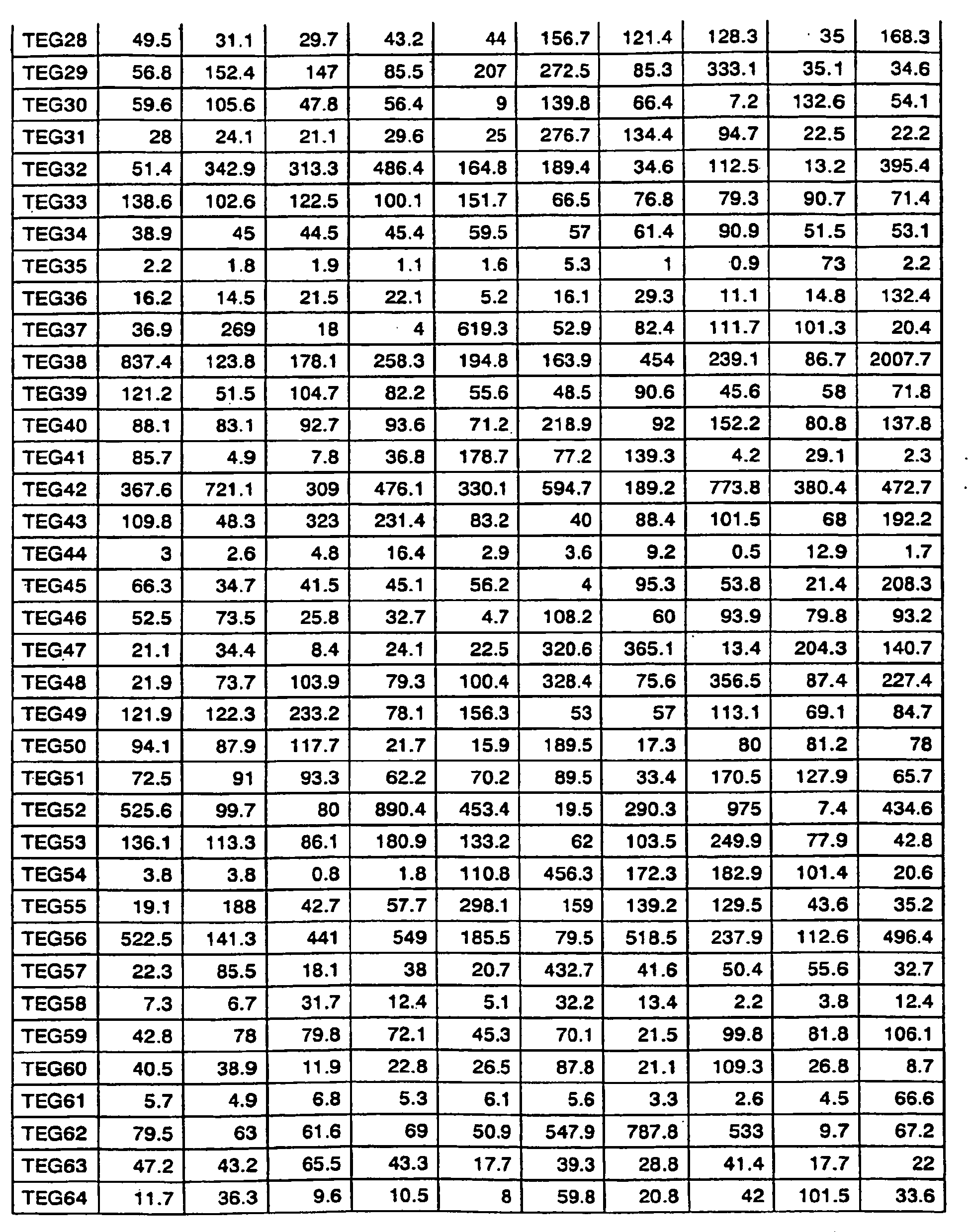

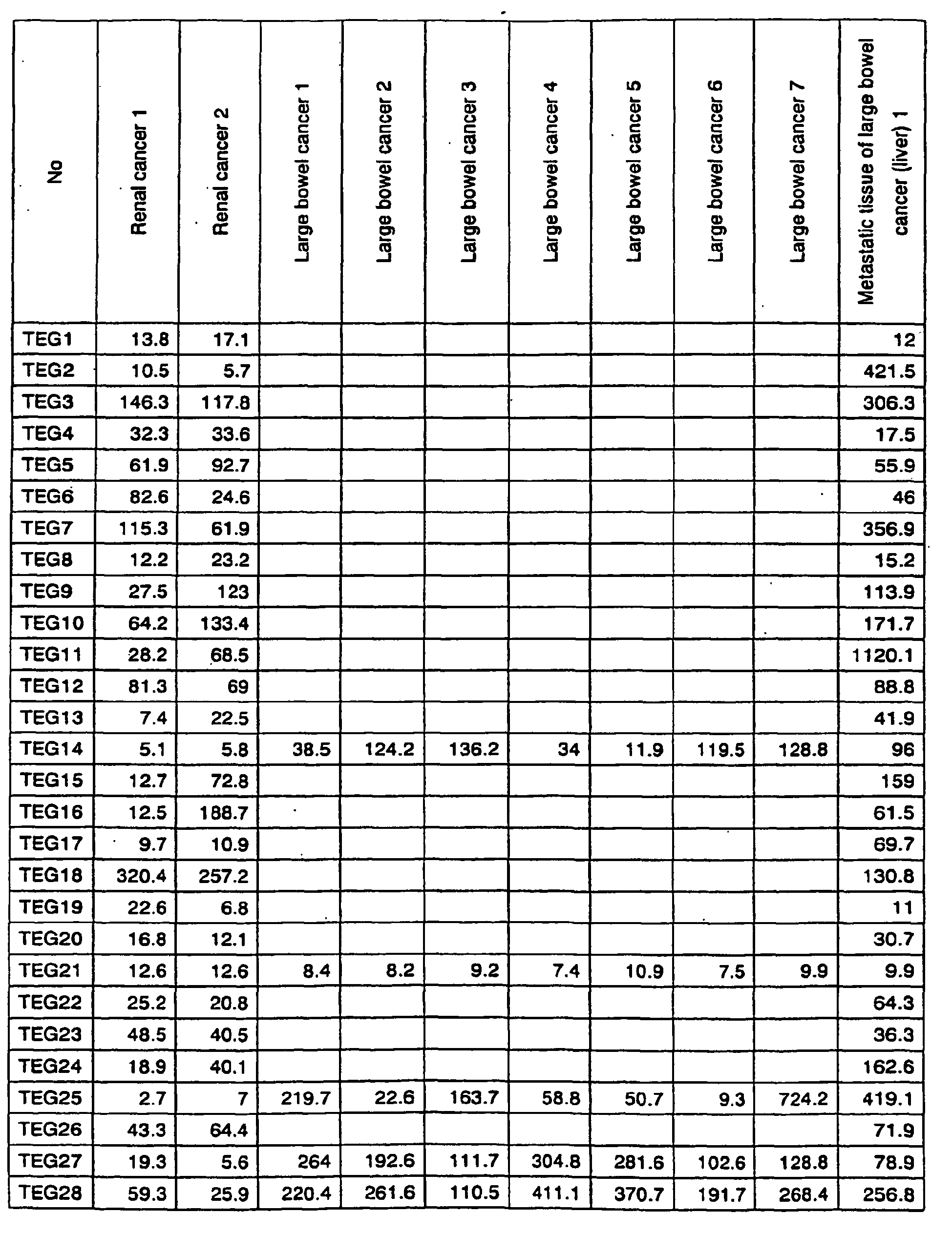

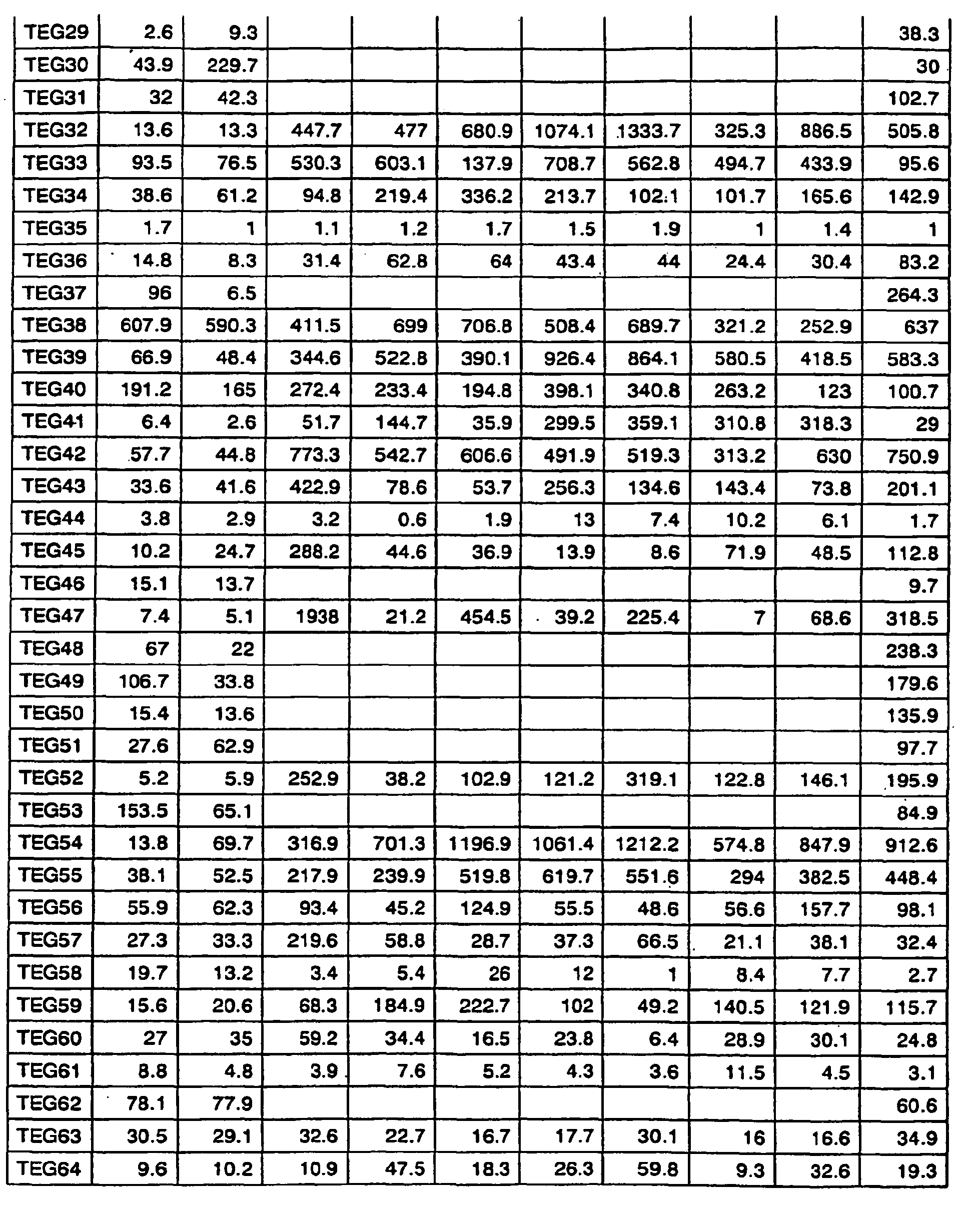

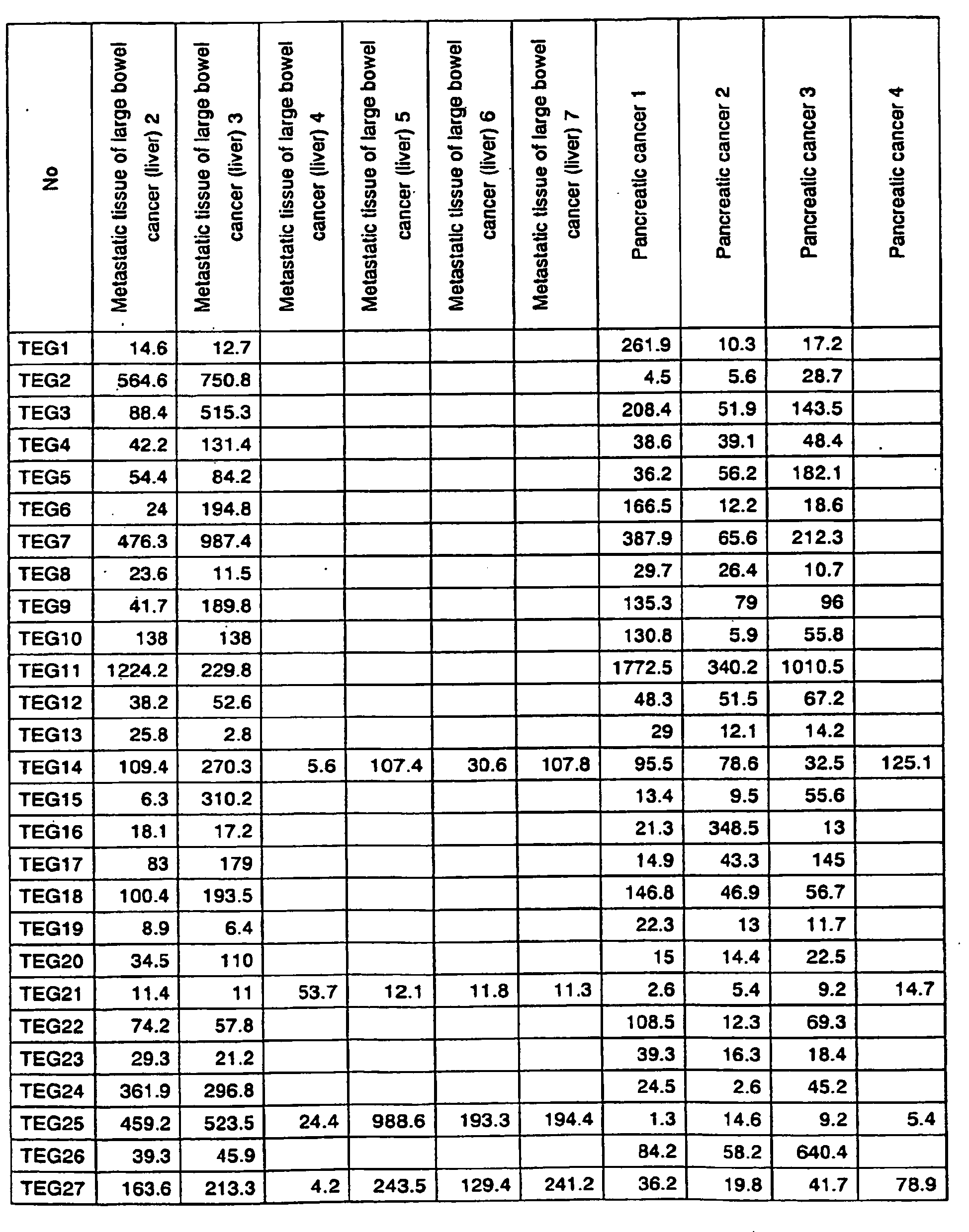

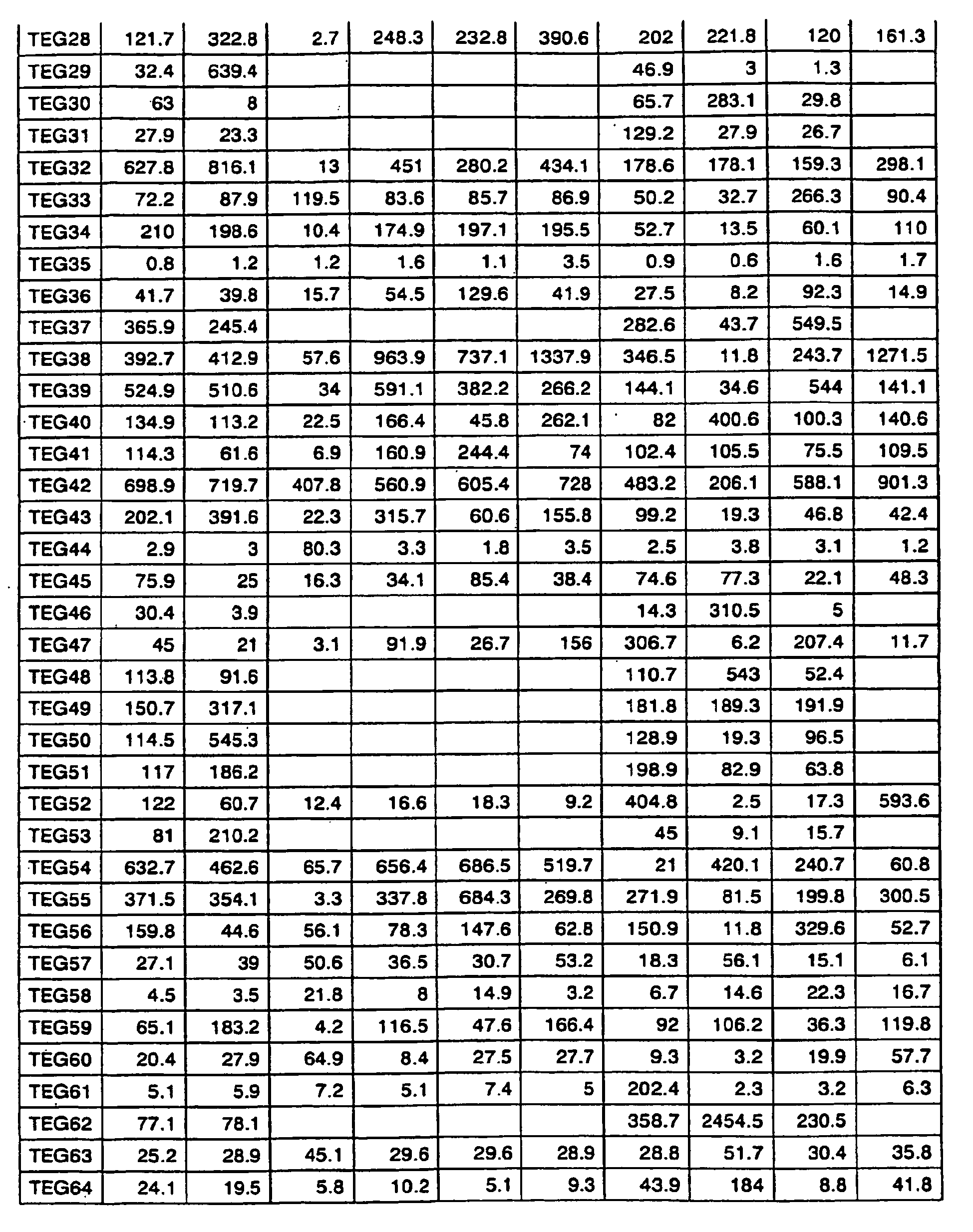

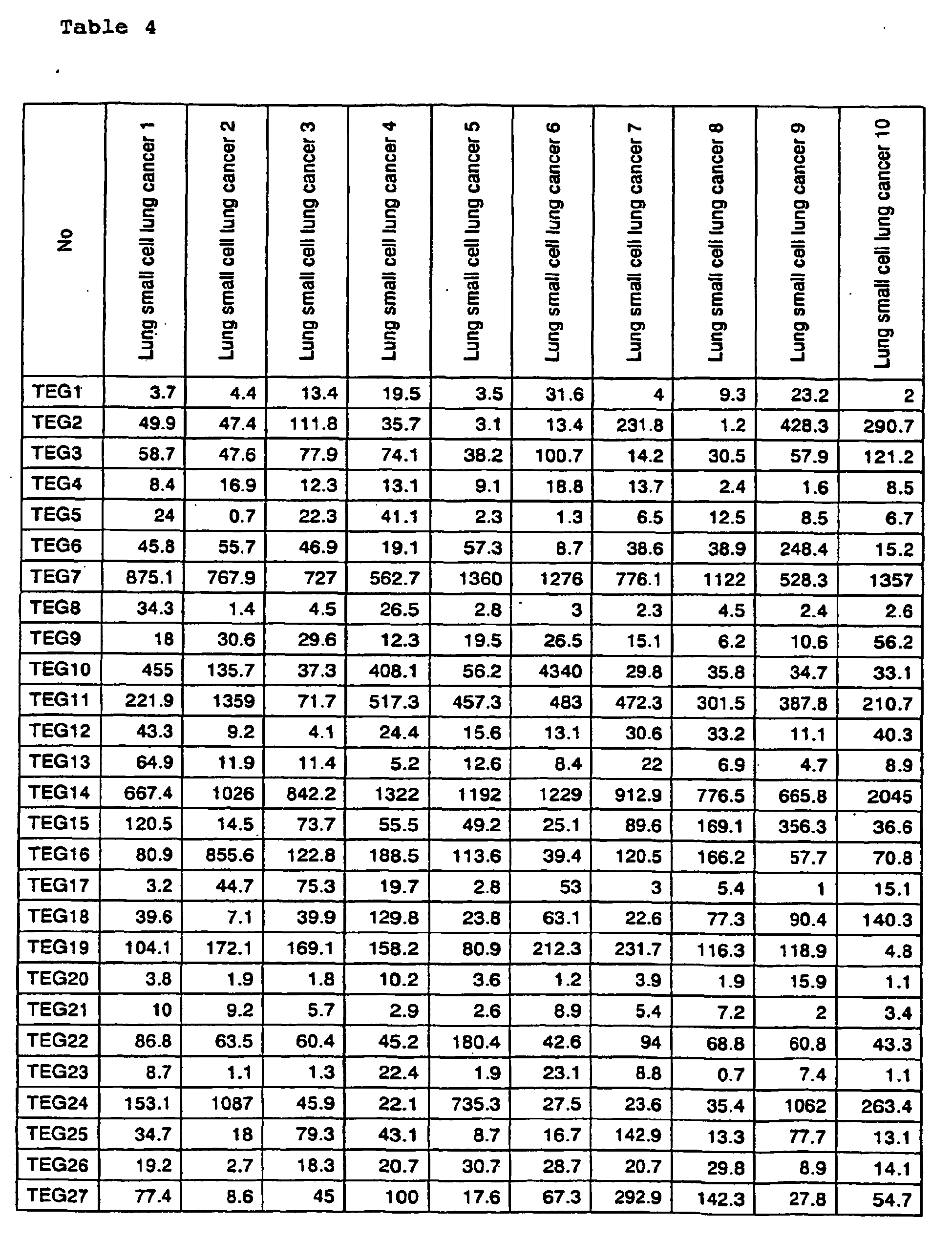

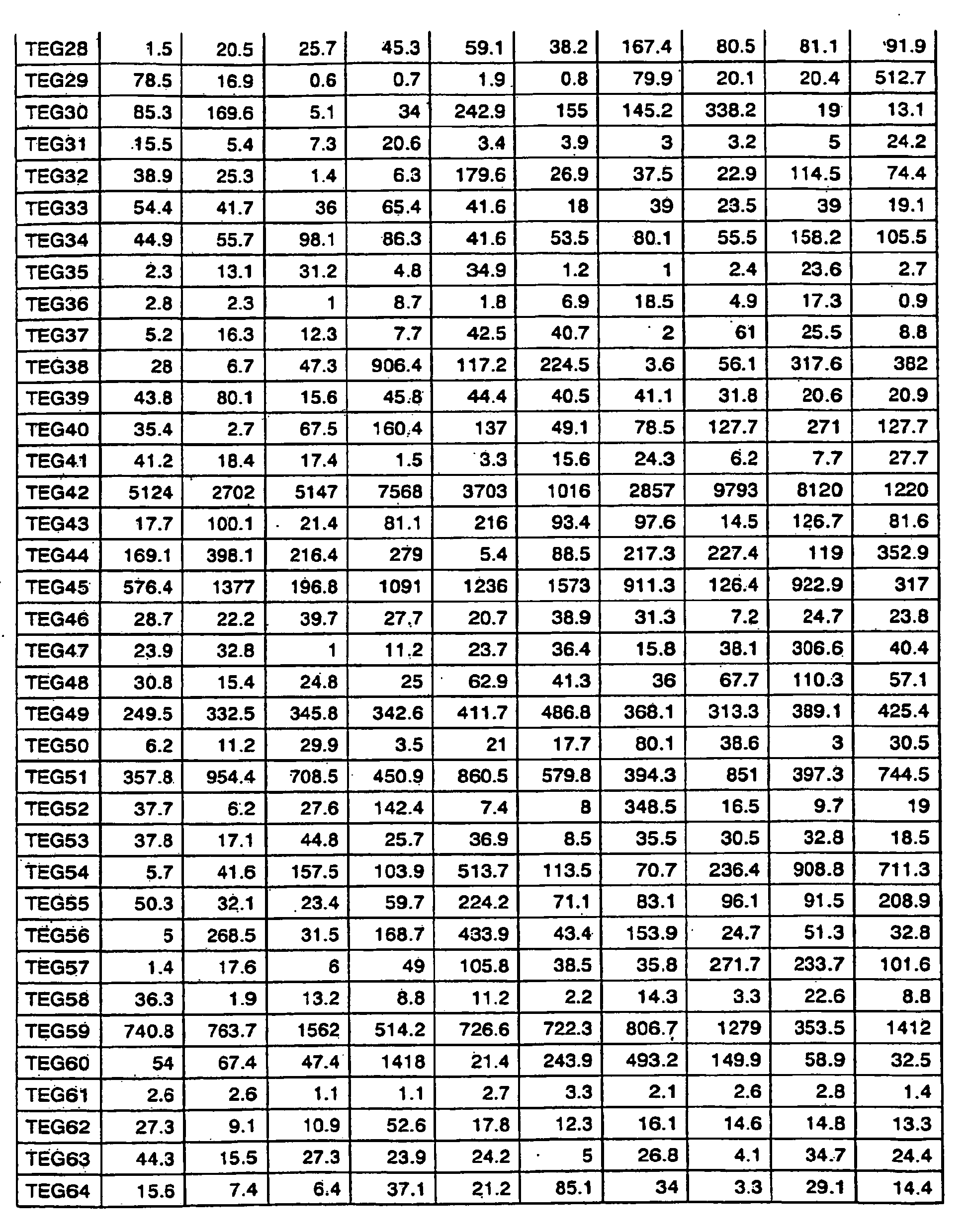

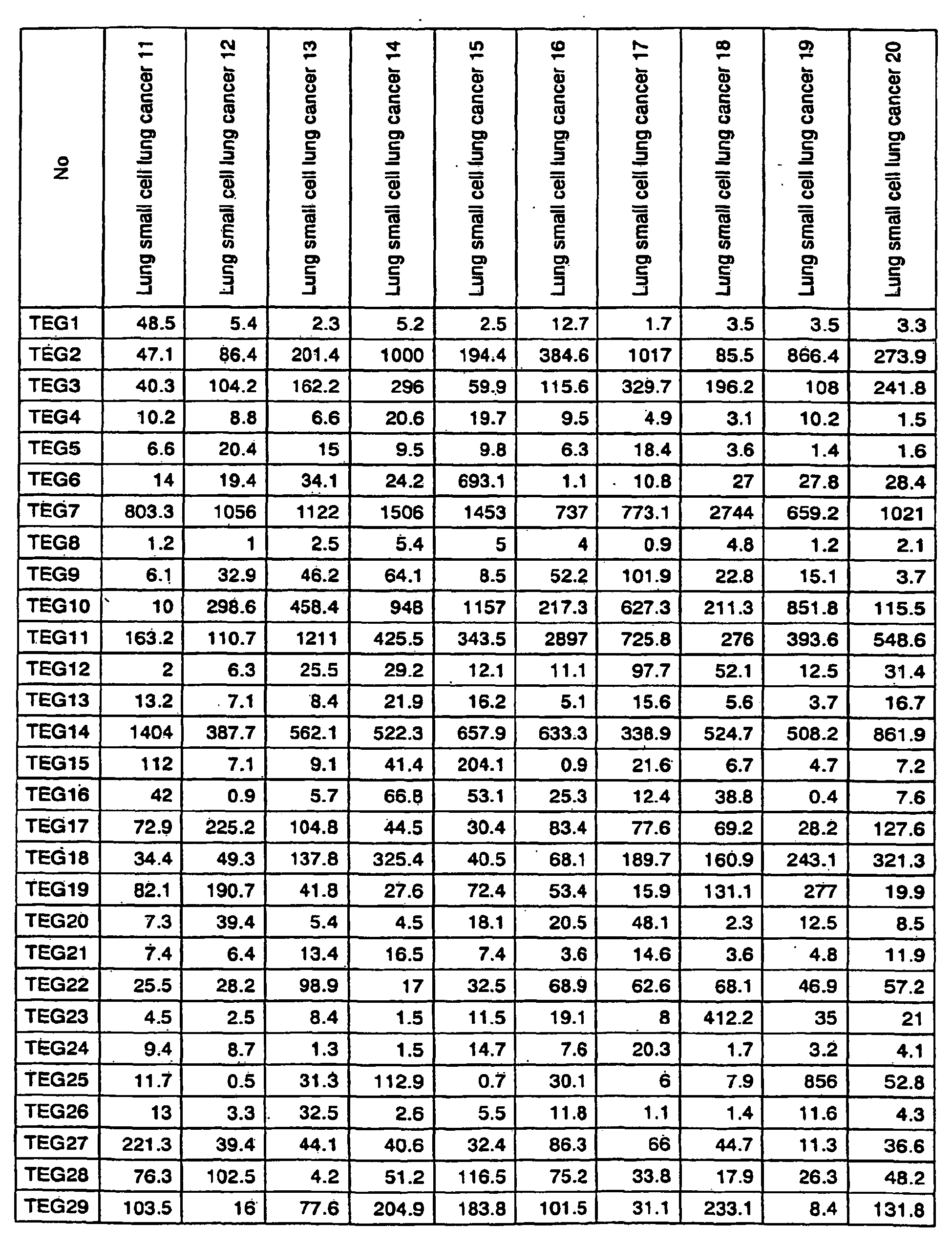

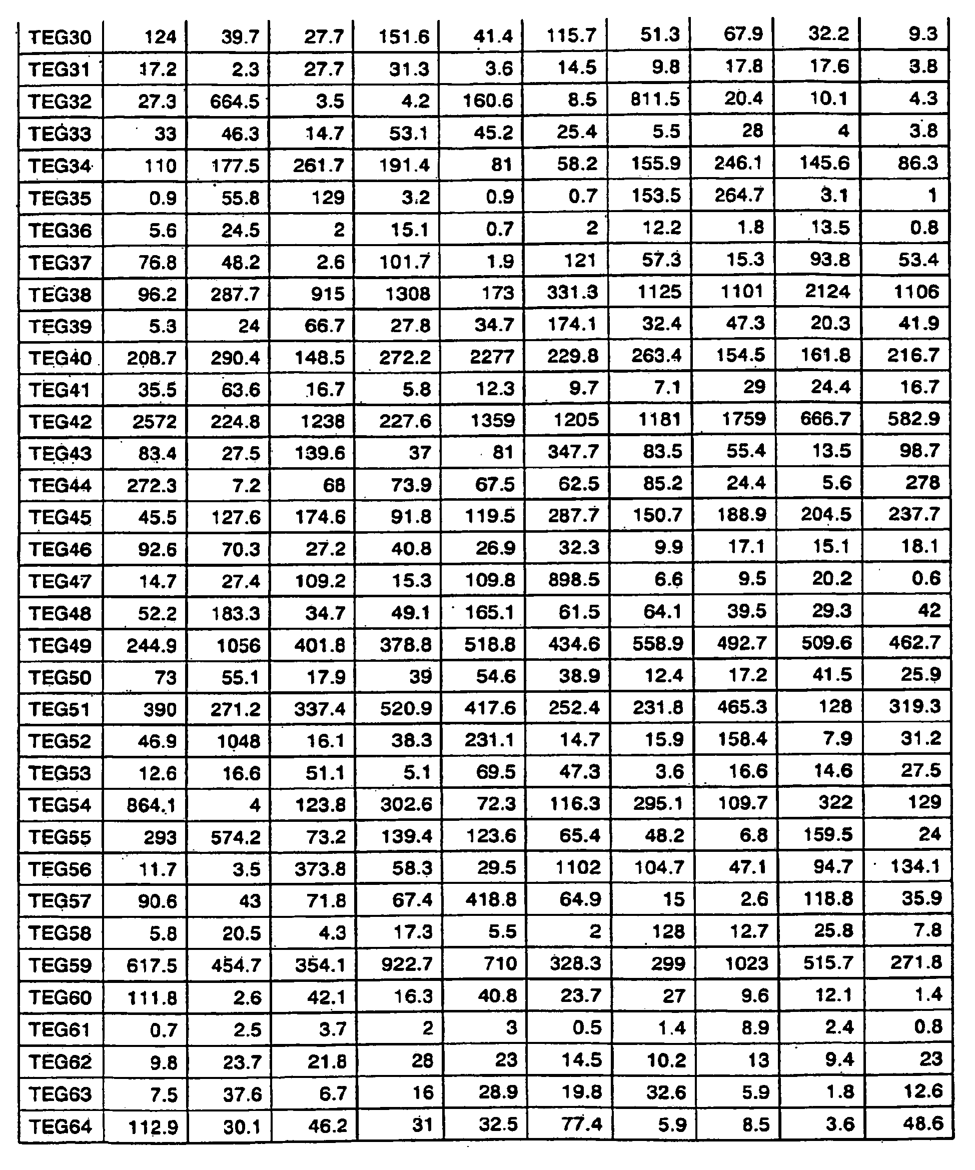

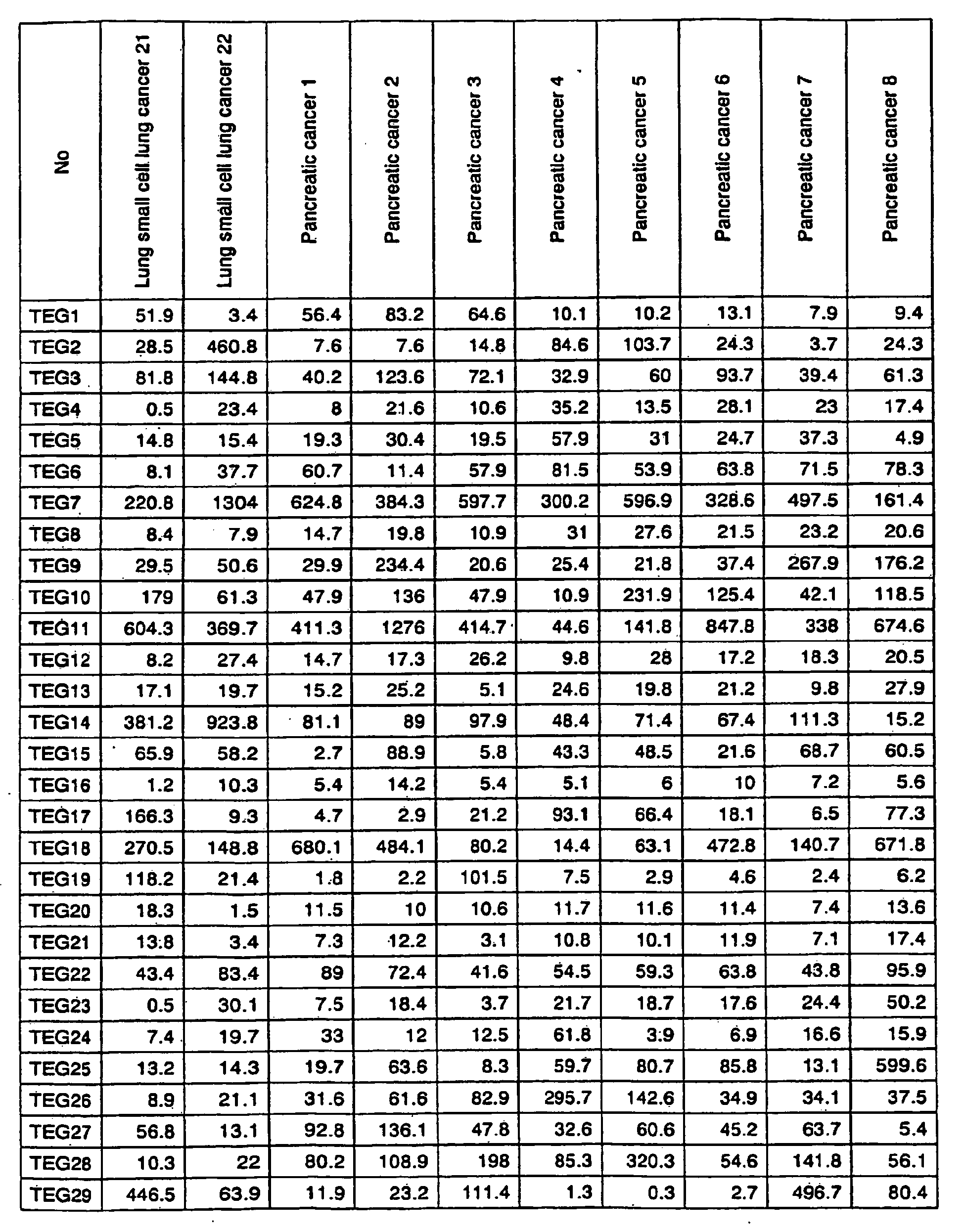

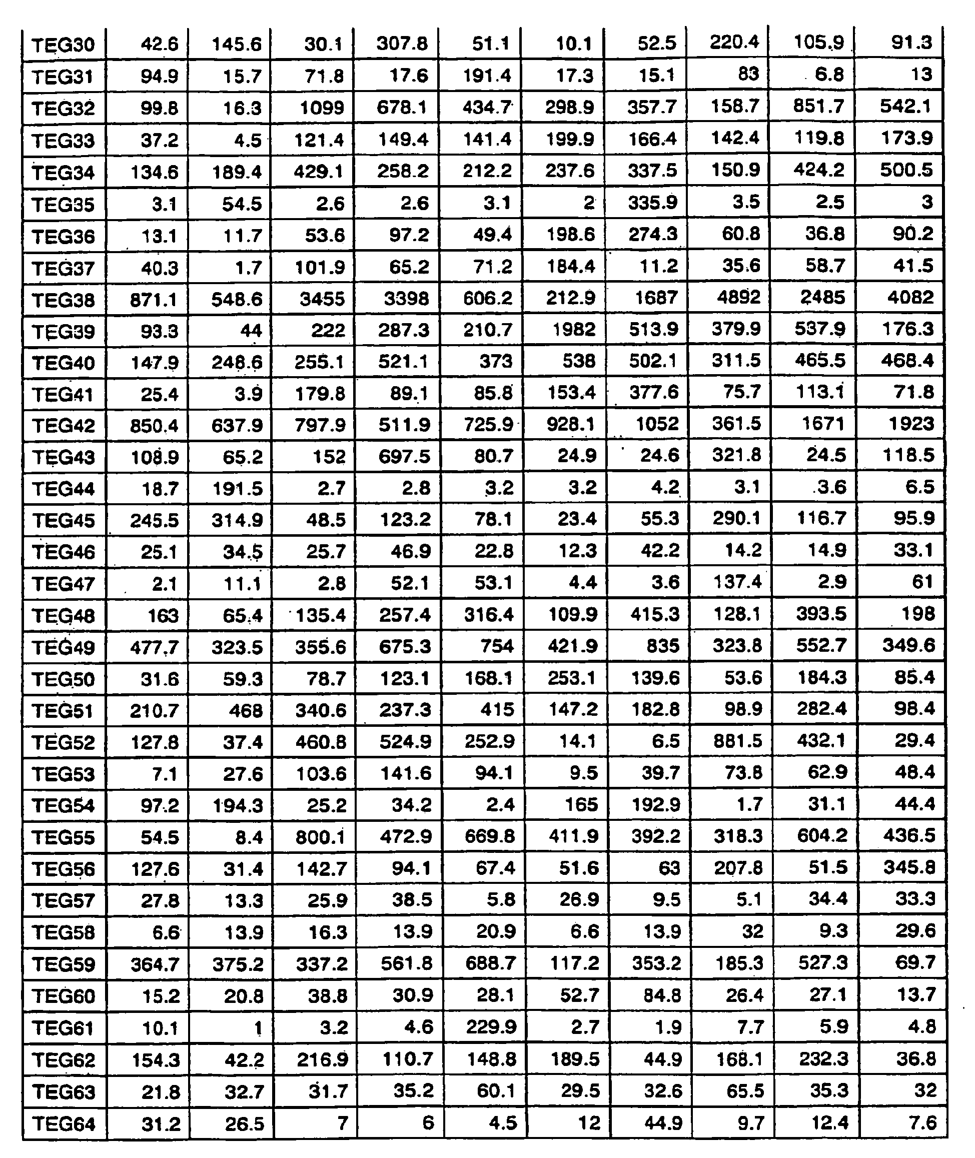

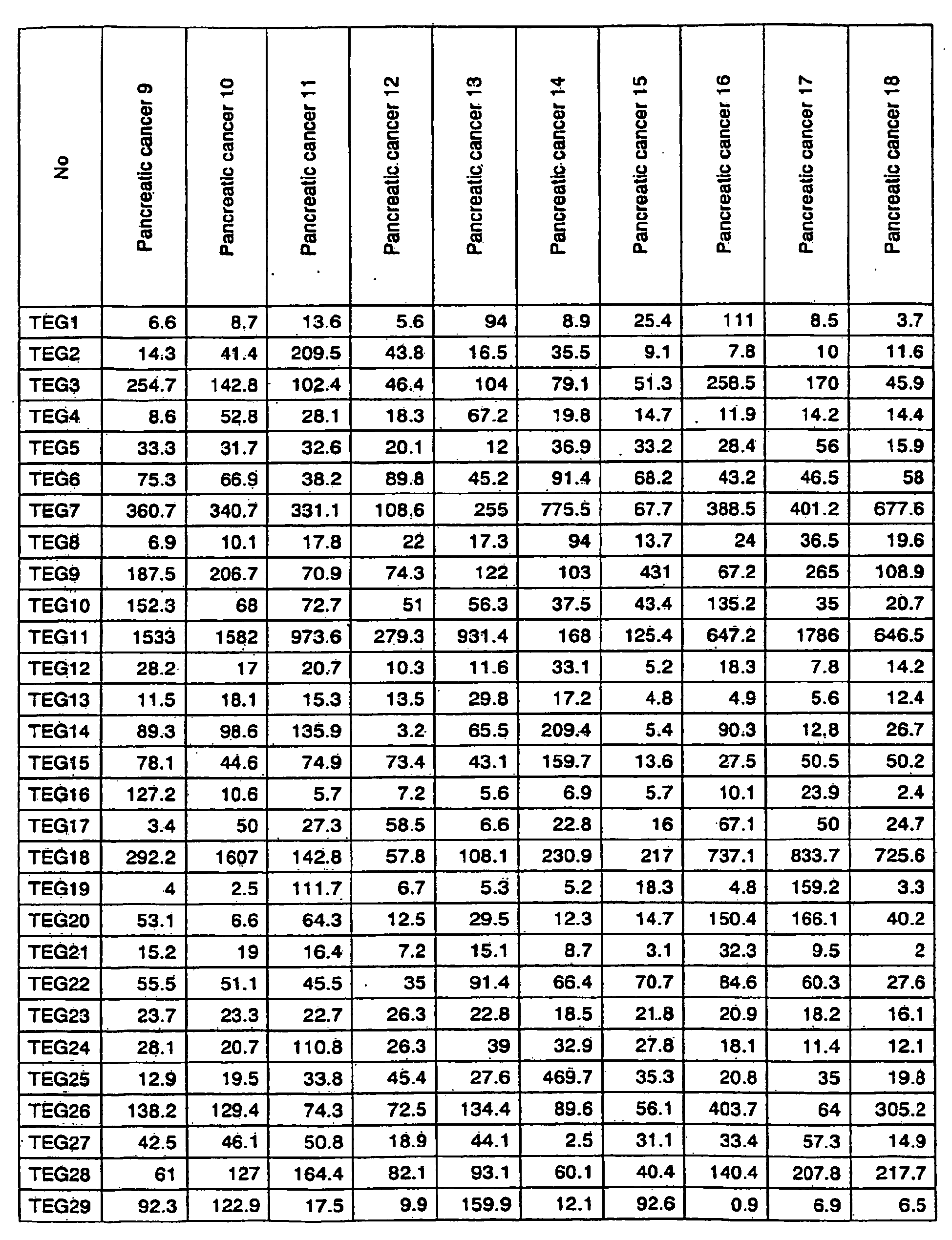

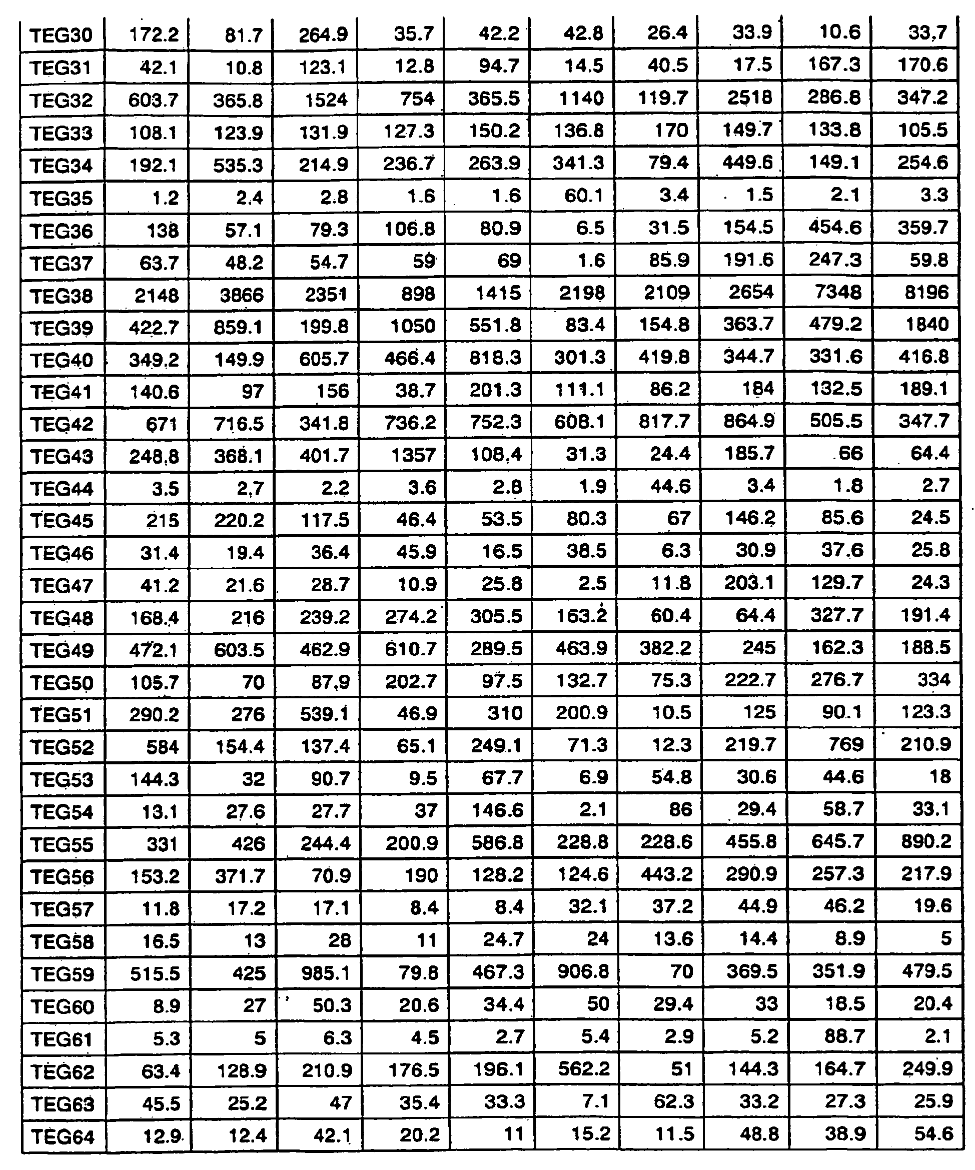

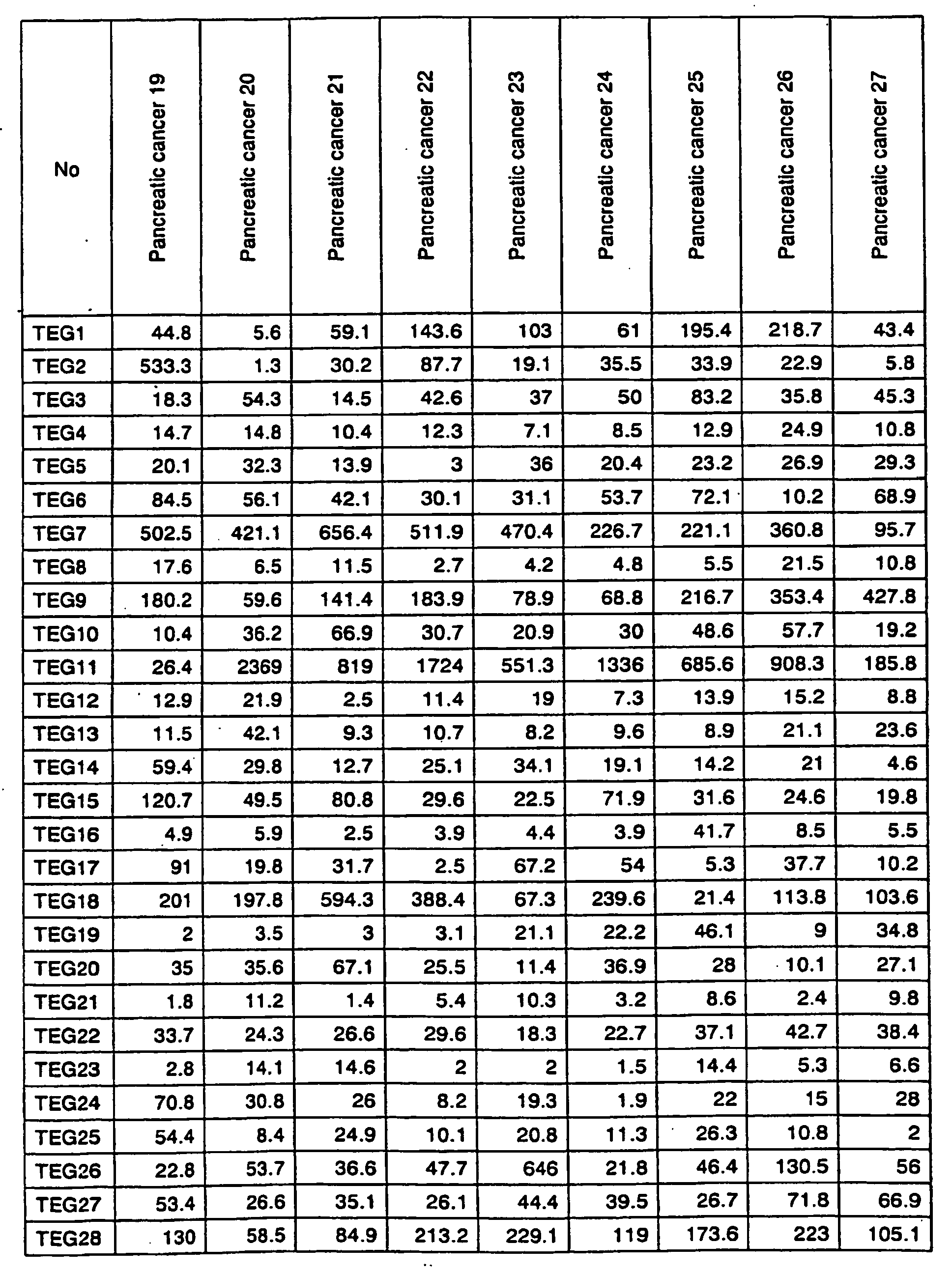

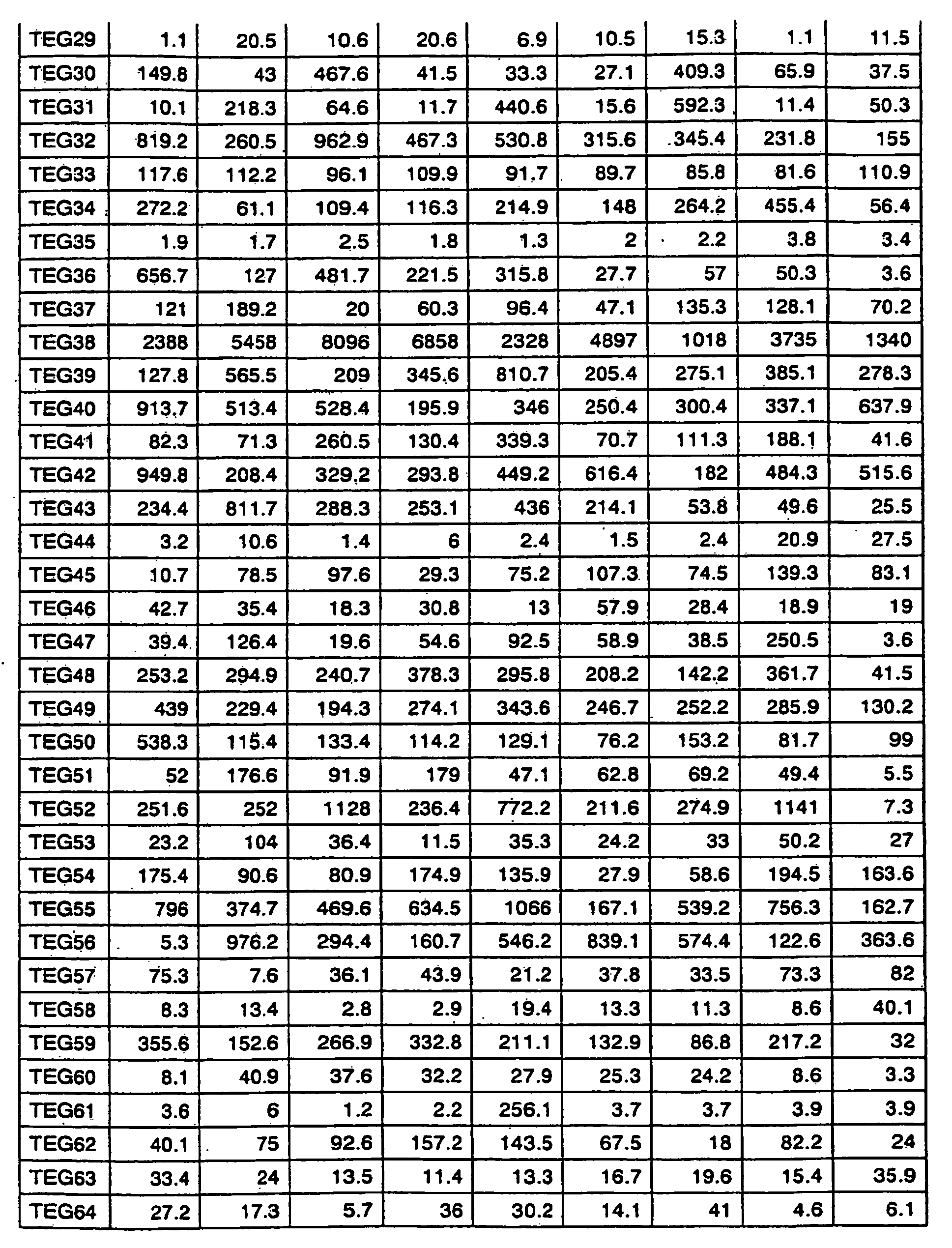

### Example 2

### Study of frequency of expression elevation using RT-PCR

In the above-mentioned Gene chip analysis, RNA prepared from each type of excised cancer tissue was analyzed as a whole. In order to verify the results from the Gene chip analysis, mRNA expression level of each gene in the respective cancer samples and the normal tissue in the non-cancerous part was analyzed by the RT-PCR method to examine the degree of expression elevation and the frequency of expression elevation.

### 2.1. Preparation of single-stranded cDNA from each type of cancer tissue

From each type of human cancer tissue and normal tissue, a single-stranded cDNA to be used as a template DNA in the PCR was prepared as follows.

Total RNA was prepared in the same manner as described above from 12 cases of lung adenocarcinoma tissue and 4 cases of normal lung tissue for lung adenocarcinoma, 10 cases of human large bowel cancer tissue and normal large bowel tissue in non-cancerous part of the same excised tissue for human large bowel cancer, 12 cases of excised human stomach cancer tissue and normal stomach tissue in non-cancerous part of the same excised tissue for human stomach cancer, and 9 cases of excised human liver cancer tissue and non-cancerous part of the same excised tissue for human liver cancer, respectively. Single-stranded cDNA was synthesized from the total RNA using a reverse transcriptase Superscript II (manufactured by GIBCO BRL). The single-stranded cDNA prepared in this manner was used as a template DNA in PCR described below.

### 2.2 Expression analysis by RT-PCR.

Subsequently, the expression level of mRNA corresponding to the genes shown in Table 2 was analyzed by the RT-PCR method. More specifically, 25 µL of a PCR solution was prepared containing 500 mM KCl, 100 mM Tris-HCl (pH 8.3), 20 mM MgCl₂, 0.1% gelatin, 1.25 mM of each dNTPs (dATP, dCTP , dGTP , dTTP), 1 µL of single-stranded cDNA, a set of sense and antisense primers that are specific for each gene in an amount of 5 pmole each, 0.75 µL of SYBR Green I (1000-fold diluted solution, manufactured by TAKARA) and 0.25 µL of recombinant Taq polymerase Mix (FG Pluthero, Rapid purification of high-activity Taq DNA polymerase, Nucl. Acids. Res. 1993 21: 4850-4851). The reaction consisted of initial denaturation at 94°C for 3 minutes, and 30 cycles of 94°C for 15 seconds, 57°C for 15 seconds and 72°C for 30 seconds. RT-PCR primers shown in Table 5 were designed and used in the analysis.

In addition, the expression level of human β-actin gene in each RNA was also analyzed in the same manner as described above using a sense primer (SEQ ID NO: 252: AGAAGGAGATCACTGCCCTGGCACC) and an antisense primer (SEQ ID NO: 253: CCTGCTTGCTGATCCACATCTGCTG) that are specific for human β-actih.

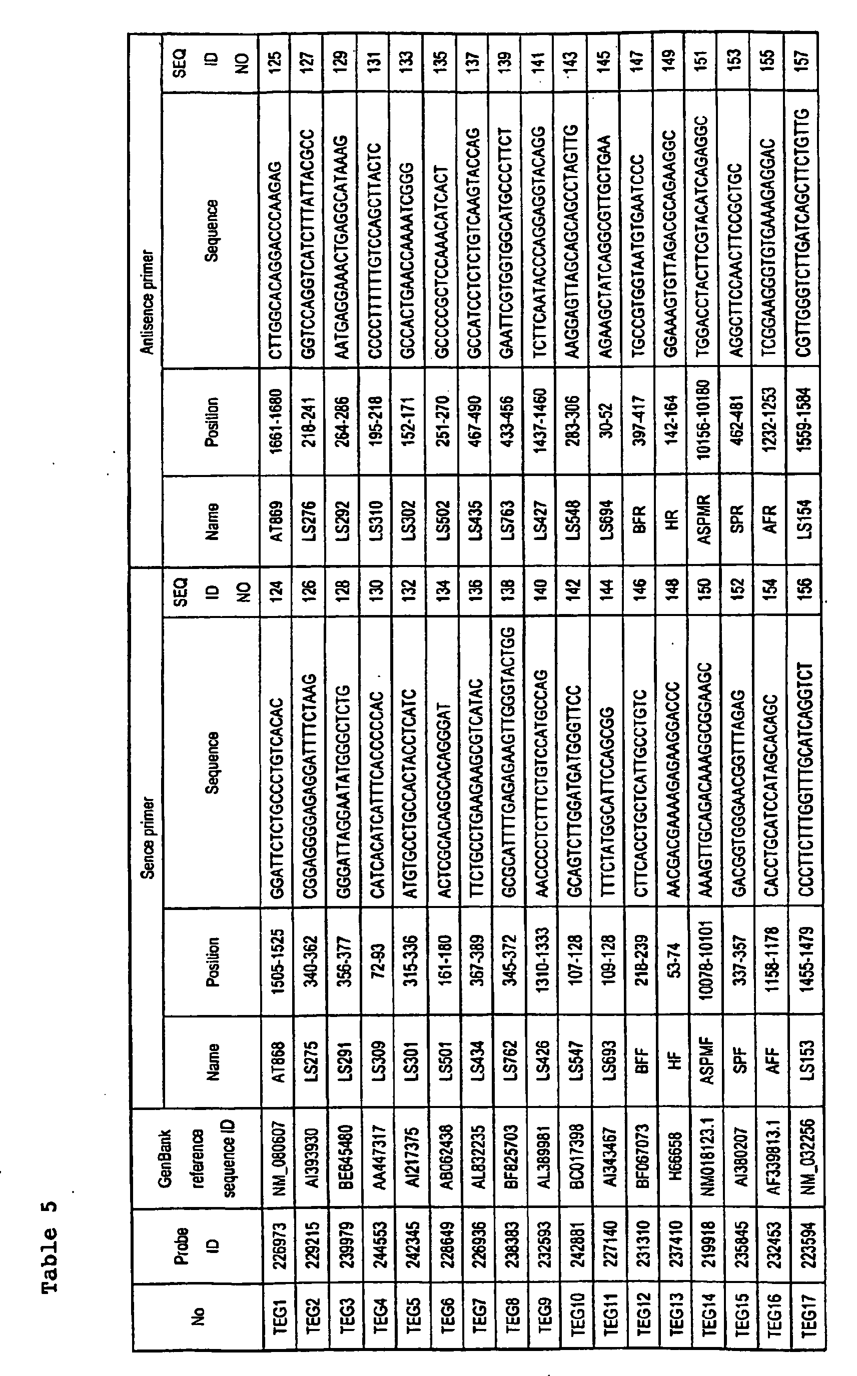

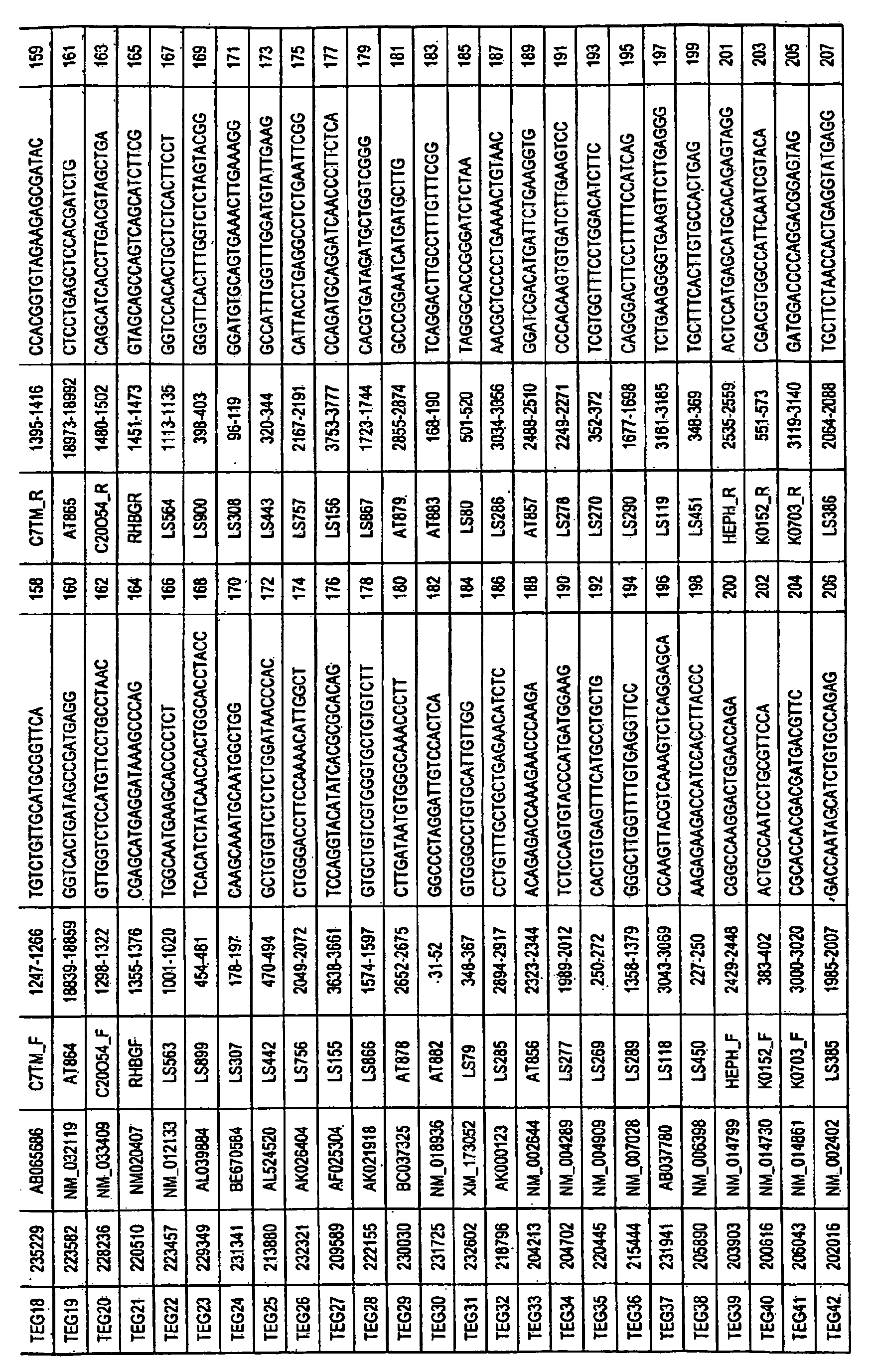

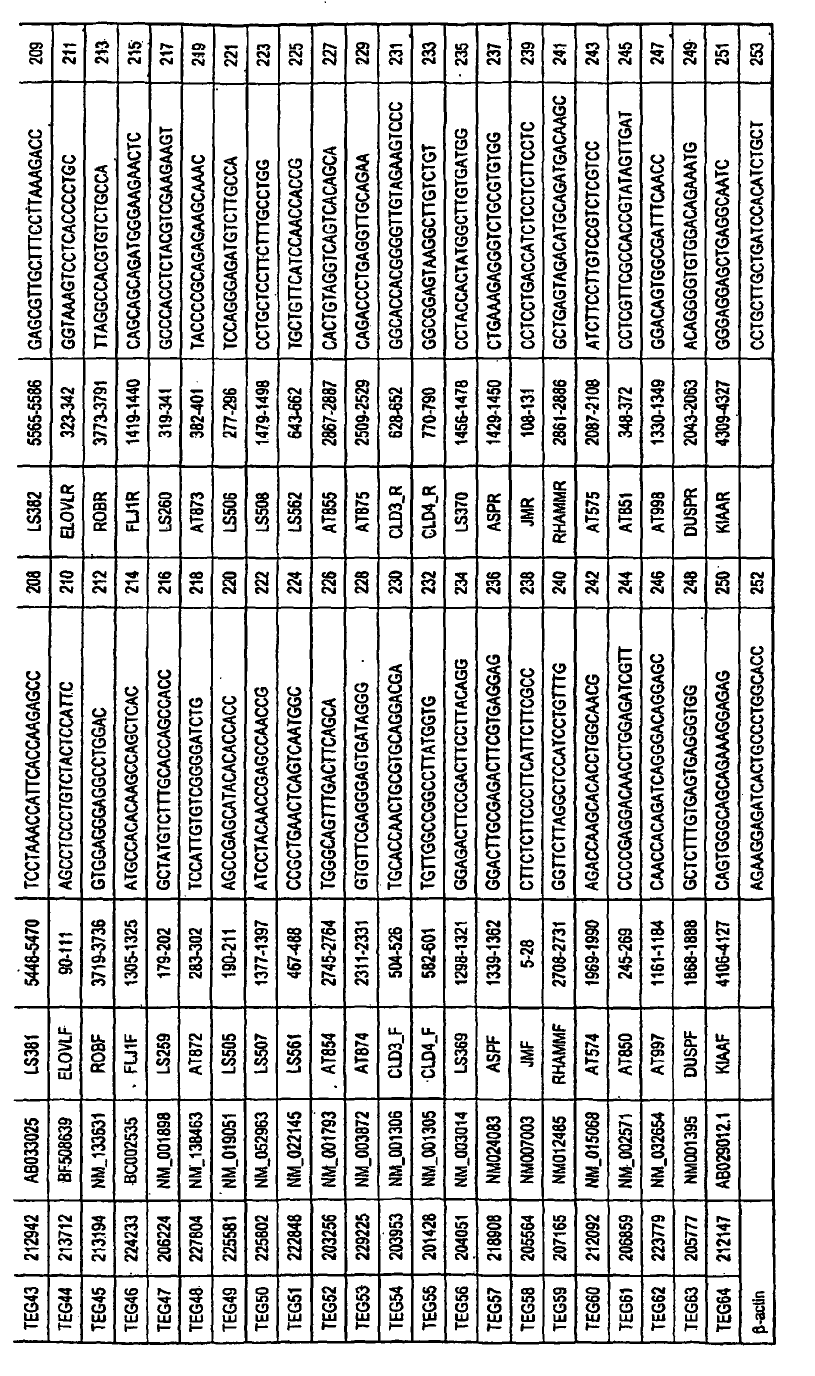

The product amplified by the PCR method was subjected to electrophoresis on 1.0% agarose gel and stained with ethidium bromide to observe the bands. The mRNA level was determined by iCyclerQreal time PCR analysis system (BIO-RAD).

### Expression analysis of TEG1

Gene expression was compared by a quantitative RT-PCR method using RNA prepared from 12 cases of lung adenocarcinoma tissue and 4 cases of normal lung tissue.

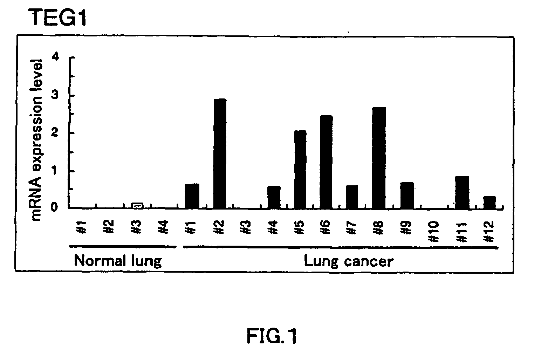

The PCR result showed that, while the expression of mRNA of TEG1 gene was not observed in the normal lung tissue, overexpression of TEG1 gene was observed clearly in 10 cases out of the analyzed 12 cases of lung adenocarcinoma tissue (Fig. 1).

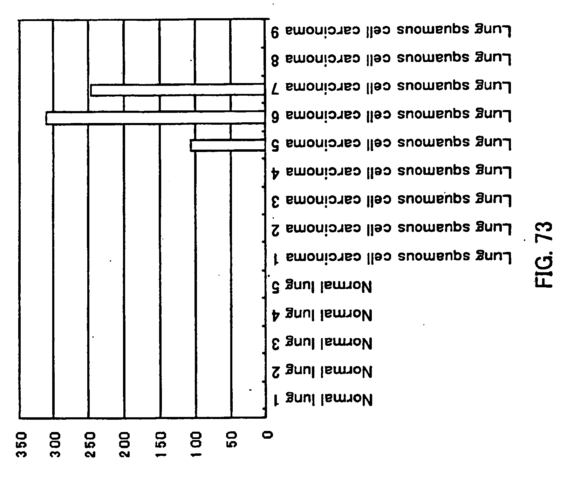

A quantitative PCR analysis was carried out for 5 cases of normal lung and 9 cases of lung squamous cell carcinoma in the same manner. The PCR result showed that, while the expression of mRNA of TEG1 gene was not observed in the normal lung tissue, an elevation in the expression of TEG1 gene was observed in 3 cases out of the analyzed 9 cases of lung squamous cell carcinoma tissue (Fig. 73).

### Example 3

### Production of anti-TEG1: C20orf102 monoclonal antibody

In order to determine whether cancer can be detected using an anti-C20orf102 antibody, an anti-C20orf102 monoclonal antibody was prepared.

### 3-1. Isolation of C20orf102 cDNA

In order to express C20orf102, C20orf102 cDNA was first isolated as follows. A single-stranded cDNA was prepared from lung adenocarcinoma tissue according to the above-mentioned method. Then, PCR was carried out using the single-stranded cDNA as a template and primers F (SEQ ID NO: 269) and R (SEQ ID NO: 270) with a restriction enzyme site for EcoRI or XhoI. A band near about 615 bp was successfully detected, which agrees with that of the predicted sequence of C20orf102. Advantage HF Polymerase Mix (manufactured by Clontech), Advantage HF PCR buffer, 200 µM deoxynucleotide triphosphate and 0.2 µM primer were used as the enzymes and reagents for PCR, and PCR (35 cycles of 94°C for 30 seconds, 68°C for 30 seconds and 72°C for 3 minutes) was carried out using 1 µL of the cDNA as a template. The specifically amplified fragment obtained by PCR was inserted into pGEM-T Easy vector (manufactured by Promega) using a DNA ligation kit (manufactured by Takara). The nucleotide sequence was checked by a standard method, and the isolated cDNA was found to correspond to C20orf102. The primers F and R were designed to hybridize with the 5' end and 3' end of C20orf102 gene (GenBank: NM_080607), respectively.

### SEQ ID NO: 269 (F): CGAATTCATGGGGGCCCCGCTCGCCGTAGC

### SEQ ID NO: 270 (R): CCTCGAGGAGGCTGCAGGCCTCCTGGTCCA

### 3 -2. Preparation of antigen for immunization against C20orf102

The pGEM-T Easy vector incorporated with the PCR product was transformed into a competent cell XL-1 Blue (manufactured by Stratagene), and the vector bearing the PCR product was selected by color selection using 5-bromo-4-chloro-3-indolyl-β-galactopyranoside (X-gal). As for the transformation, 10 µL of a ligation reaction product was added to the competent cells, the mixture was cooled on ice for 30 minutes, and the cells were subjected to heat shock at 42°C for 45 seconds and cooled on ice for 2 minutes thereby inducing transformation. Further, in order to allow an antibiotic resistance gene to be expressed, 900 µL of LB medium without antibiotics was added and the mixture was mildly stirred at 37°C for 30 minutes. Then, the cells were collected by centrifugation and plated on an LB plate containing ampicillin to which 20 µL of 20 mg/mL X-gal had been sprayed, and the cultivation was carried out at 37°C for 16 hours. Among the colonies grown on the plate, 5 colonies without developing color (i. e. the PCR product is expected to be incorporated into the vector) were selected, and the cells were grown in 5 mL of LB medium containing ampicillin at a final concentration of 100 µg/mL at 37°C for 16 hours with vigorous stirring. Plasmid DNA was collected by phenol/chloroform extraction from a portion of the grown cells, then 0.5 µL of EcoRI (8 U/µL), 2 µL of a 10xH buffer and 7.5 µL of distilled water were added thereto, and the plasmid was digested at 37°C for 1 hour. The size of the digested fragment was confirmed to be the same as that of the PCR product by electrophoresis on a 0.8% agarose gel. The plasmid DNA into which C20orf102 gene is considered to have been inserted was collected using a Quantum Prep Plasmid Miniprep Kit (manufactured by BioRad). The plasmid was eluted with distilled water. After the nucleotide sequence was determined by a standard method, DNA was digested with restriction enzymes EcoRI and XhoI. Then the digested fragment was inserted into an E. coli protein expression vector, pET41a vector (manufactured by Novagen). The gene incorporated into pET41 is translated as a GST fusion protein.

pET41 was digested with restriction enzymes (EcoRI and XhoI), subjected to electrophoresis and purified by using a Qiaquick Gel Extraction Kit. A fragment having the sequence of C20orf102 was amplified by pGEM-T Easy and was inserted into pET41 using a DNA ligation kit.

To 4 µL of the C20orf102 fragment purified from pGEM-T Easy, 5 µL of a ligation buffer and 1 µL of pET41 were added and the mixture was incubated at 16°C for 30 minutes.

After finishing the ligation reaction, the plasmid DNA was transformed into XL-1 Blue, the cells were grown with shaking in LB medium containing kanamycin for 16 hours. From the grown E. coli cells, the plasmid was purified using a Quantum Prep Plasmid Miniprep Kit. In order to confirm the insertion of C20orf102 into pET41, the plasmid was sequenced with a primer pair (SEQ ID NOs : 271 and 272) corresponding to the sequence of pET.
SEQ ID NO: 271: TTCGAACGCCAGCACATGGAC
SEQ ID NO: 272: GCTAGTTATTGCTCAGCGGTG

The pET41 vector bearing C20orf102 was transformed into a competent cell of BL21 Codon PLUS RIL (manufactured by Novagen) having T7 promoter.

Transformation was carried out according to the following procedure. To 100 µL of BL21 Codon PLUS RIL, 1 µL of pET-C20orf102-FL was added at a concentration of 1 µg/µL and the mixture was cooled on ice for 5 minutes. Then, the mixture was placed in a thermostat bath at 42°C for 20 seconds, and the cells were subjected to heat shock. Then, the mixture was cooled on ice for 2 minutes, and 900 µL of LB without antibiotics was added. Then, the cells were incubated at 37°C for 10 minutes, and centrifuged (1000 x g, 5 min). After the supernatant was discarded, the competent cells were resuspended and plated on an LB plate containing kanamycin, and then selection culture was carried out at 37°C for 16 hours.

The GST fusion protein of C20orf102 expressed in E. coli was purified by affinity purification utilizing the binding of GST to glutathione. First, the culture solution was centrifuged at 6000 x g for 10 minutes at 4°C to collect E. coli cells. A cell lysis buffer (50 mM sodium chloride, 1 mM EDTA, 1 mM dithiothreitol (DTT), 50 mM tris hydroxyaminomethane hydrochloride, pH 8.0) was added and the mixture was sonicated on ice. Triton X-100 was added at a final concentration of 1%, and centrifuged at 13400 x g for 45 minutes at 4°C to collect the supernatant. To the supernatant, 500 µL of glutathione sepharose (manufactured by Amersham Biosciences) was added, and end-over-end mixing was carried out at 4°C for 1 hour to allow the GST-C20orf102 fusion protein to be adsorbed.

Glutathione sepharose was collected by centrifugation (3000 x g, 4°C, 5 min) and washed with 10 mL of PBS-T (PBS containing 0.5% Triton X-100). An elution buffer (50 mM reduced glutathione, 200 mM sodium chloride, 1 mM EDTA, 1 mM DTT, 200 mM Tris-HCl, pH 8.0) was added and mixed end-over-end at 4°C for 1 hour to elute the GST fusion protein. The glutathione sepharose was removed by centrifugation (3000 x g, 4°C, 5 min) to obtain a purified protein of GST-fused C20orf102. A PBS solution of the fusion protein was prepared using a PD-10 column (manufactured by Amersham Biosciences), and the protein concentration was determined by the Bradford method. The purity of the protein was assayed by SDS-PAGE to confirm that the obtained protein satisfies the amount and purity required for immunization. The protein was used as an immunogen to produce a monoclonal antibody as described below.

### 3-3. Production of C20orf102 monoclonal antibody

A purified product (E. coli-expressed protein) of the GST fusion protein of the full-length human C20orf102 was used as an immunogen. Mice (BALB/c female at 6 weeks of age) were immunized three times with the immunogen at 50 µg/mouse, and the antibody titer in the serum was assayed. The antibody titer assay was carried out by an immunogen solid-phase ELISA method. A dilution series of the immunized mouse serum pretreated with GST protein to absorb the anti-GST antibody was reacted with the immunogen immobilized on an ELISA plate in an amount of 0.5 µg/well. It was reacted with an HRP-labeled anti-mouse antibody, and then the absorbance at 450 nm was measured for the developed color upon addition of a substrate.

Mice showing an increase in the antibody titer received a final immunization with the antigen at 25 µg/mouse. Spleen cells were collected at 72 hours after the final immunization, and fused with myeloma cells (P3/NSI-1-Ag4-1) (Kohler, G, Milstein, C: Nature, 256: 495 (1975)). The cells were cultured in HAT selection medium to obtain hybridomas. The culture supernatant of the hybridomas was pre-absorbed with GST protein and assayed by an immunogen solid-phase ELISA to primarily select those reacting with C20orf102 (actual product). The immunogen ELISA positive hybridoma was assayed for the specificity of the antibody by Western blotting using a protein extract solution from COS7 cells forcibly expressing C20orf102 . A positive hybridoma was cloned by a limiting dilution method, whereby a monoclonal antibody-producing cell line was established. The antibody- producing hybridoma was inoculated into BALB/c mice, and mouse ascites was obtained. The monoclonal antibody in the ascites was purified by an ammonium sulfate precipitation method to obtain a purified antibody preparation. In this way, an anti-C20orf102 antibody H9615 was prepared.

### Example 4

### Detection of C20orf102 protein molecule using anti- C20orf102 monoclonal antibody

In order to test the reactivity of the anti-C20orf102 monoclonal antibody H9615 prepared as described above, C20orf102 protein was measured in cell lysates of a cell line forcible expressing C206rf102 and cell lysates of a variety of cancer cell lines.

First, the reactivity of the anti-C200rf102 monoclonal antibody H9615 was examined by Western blot analysis using a COS7 cell line forcibly expressing C20orf102. A C20orf102 gene expression vector was constructed by inserting the C20orf102-encoding cDNA into pcDNA4Mys-His (manufactured by Invitrogen) and used as an animal cell expression vector. More specifically, 1 µg of the expression vector was introduced into 5 x 10⁴ COS7 cells using FuGene 6 reagent (manufactured by Roche Diagnostics) and the cells were made to transiently express C20orf102. Three days after introduction of the expression vector, the cells were collected and were solubilized with RIPA buffer (150 mM sodium chloride, 1% NP-40. 0.5% deoxycholic acid, 0.1% SDS, 50 mM tris hydroxyaminomethane hydrochloride (pH 8.0)) to prepare a cell lysate. Each lysate in an amount corresponding to 10 µg of proteins was loaded on a SDS-polyacrylamide gel and the protein was separated by SDS-PAGE and transferred to Hybond-P (manufactured by Amersham Biosciences). The protein was detected by ECL plus (manufactured by Amersham Biosciences) using the anti-C20orf102 monoclonal antibody H9615 (1 µg/mL) and HRP-labeled anti-mouse IgG antibody (manufactured by Jackson) as a secondary antibody. A specific band was detected near the theoretical molecular weight of 22.5 kDa, which is considered to be the C20orf102 protein.

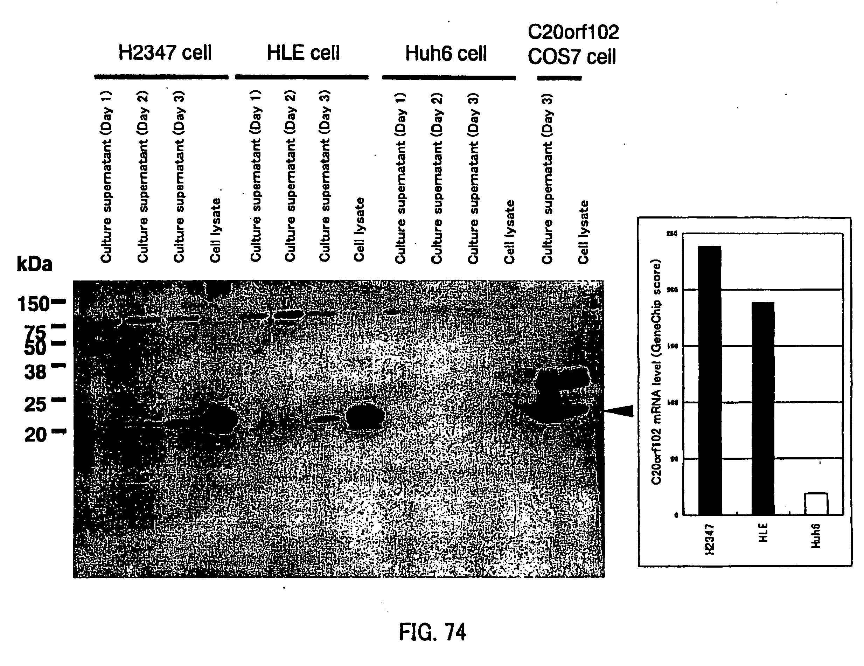

In parallel, the cell lysates from a variety of cancer cell lines were assayed by Western blot analysis in the same way as above. The result was consistent with the analysis results from GeneChip U133, namely, a band which is considered to be the full-length C20orf102 with a molecular weight of about 22.5 kDa was successfully detected only in the cell lines which showed a high mRNA expression score (Fig. 74).

Further, since C20orf102 gene has a secretory signal in the predicted sequence, it was examined whether a secretory form of C20orf102 can be detected in the culture supernatant of a cancer cell line expressing C20orf102. A band with the same Molecular weight as that in the culture supernatant of the cell line forcibly expressing C20orf102 was also detected in the culture supernatant of the cancer cell lines overexpressing C20orf102 by the anti-C20orf102 monoclonal antibody (Fig. 74).

From the above results, it was found that the anti-C20orf102 monoclonal antibody H9615 can specifically detect C20orf102 and that the mRNA expression level from the GeneChip analysis agrees with the C20orf102 protein expression level. Further, from the study using the anti-C20orf102 monoclonal antibody, it was found that secretory C20orf102 is present in the culture supernatant of C20orf102-expressing cells, strongly suggesting that the presence or absence of cancer cells may possibly be determined by detecting the secretory form of C20orf102.

### Example 5

### Expression analysis of C20orf102 protein in lung adenocarcinoma tissue using anti-C20Orf102 monoclonal antibody

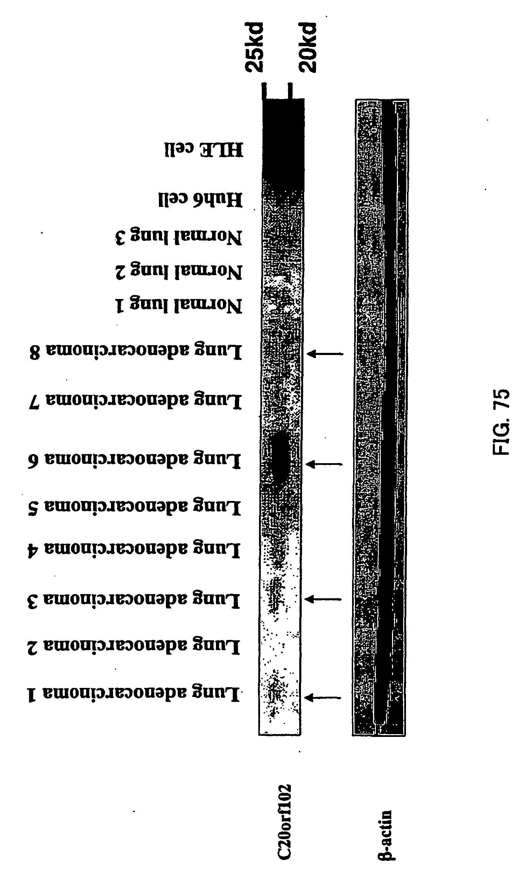

Tissue extract from lung adenocarcinoma was analyzed by Western blot analysis using the anti- C20orf102 monoclonal antibody H9615. Human tissue extract was prepared by adding RIPA buffer (150 mM sodium chloride, 1% NP-40, 0.5% deoxycholic acid, 0.1% SDS, 50 mM tris hydroxyaminomethane hydrochloride (pH 8.0)) to tissue fragments, followed by homogenization by sonication, and then collecting the supernatant fraction by centrifugation. The protein concentration was determined by the Bradford method for each extract sample, and the sample was adjusted at a concentration of 4 mg/mL. Then, the sample was mixed with an equal amount of SDS-sample buffer and heated at 95°C for 5 minutes. Ten µg of each of the extract samples was applied to a 15% polyacrylamide gel and subjected to SDS-PAGE. The sample was analyzed by Western blot analysis with the anti-C20orf 102 monoclonal antibody H9615 in the same manner as described above. A specific band near about 22.5 kDa was detected specifically in the cancerous part (Fig. 75).

From the above results, it was found that the TEG1: C20orf102 molecule is highly expressed specifically in the cancerous part even at the protein level and is secreted in a cancer cell line, suggesting that the molecule is useful for diagnosis of cancer by a monoclonal antibody using tissue and serum specimens.

### Industrial Applicability

The gene, protein, and antibody as described herein can be used in diagnosis of cancer. SEQUENCE LISTING
## Claims

1. An *in vitro* method of diagnosing cancer comprising the step of detecting in a sample collected from a subject a protein having the amino acid sequence as set forth in SEQ ID NO: 66 contained in the sample.

2. The method according to claim 1, wherein the sample collected from the subject is blood, serum or plasma.

3. The method according to claim 1 or 2, wherein an extracellular domain of a protein having the amino acid sequence as set forth in SEQ ID NO: 66 is detected.

4. The method according to claim 1 or 2, wherein a secretory form of the protein having the amino acid sequence as set forth in SEQ ID NO: 66 is detected.

5. The method according to any one of claims 1 to 4, wherein the protein is detected by an antibody recognizing said protein.

6. The method according to claim 5, wherein the antibody is a polyclonal antibody.

7. The method according to claim 5, wherein the antibody is a monoclonal antibody.

8. The method according to any one of claims 1 to 7, wherein the cancer is lung cancer, liver cancer or pancreatic cancer.

9. An *in vitro* method of diagnosing cancer comprising the step of detecting in a sample collected from a subject a polynucleotide, wherein the polynucleotide:
a) comprises or consists of the polynucleotide sequence as set forth in SEQ ID NO: 2 or its RNA equivalent;
b) has a sequence which is a complementary to the sequence of a);
c) has a polynucleotide sequence which codes for the polypeptide, sequence as set forth in SEQ ID NO: 66;
d) has a sequence identity of 70% or more to the sequence shown in SEQ ID NO: 2; or
e) is a fragment of the polynucleotide sequence as set forth in SEQ ID NO: 2.

## Patentansprüche

1. In vitro-Krebsdiagnoseverfahren, umfassend den Schritt des Nachweisens in einer von einem Individuum gewonnenen Probe eines in der Probe enthaltenen Proteins, das die Aminosäuresequenz wie in SEQ ID NO:66 dargestellt aufweist.

2. Verfahren nach Anspruch 1, wobei die von dem Individuum gewonnene Probe Blut, Serum oder Plasma ist.

3. Verfahren nach Anspruch 1 oder 2, wobei eine extrazelluläre Domäne eines Proteins nachgewiesen wird, das die Aminosäuresequenz wie in SEQ ID NO:66 dargestellt aufweist.

4. Verfahren nach Anspruch 1 oder 2, wobei eine sekretorische Form des Proteins nachgewiesen wird, das die Aminosäuresequenz wie in SEQ ID NO:66 dargestellt aufweist.

5. Verfahren nach einem der Ansprüche 1 bis 4, wobei das Protein durch einen Antikörper, der das Protein erkennt, nachgewiesen wird.

6. Verfahren nach Anspruch 5, wobei der Antikörper ein polyclonaler Antikörper ist.

7. Verfahren nach Anspruch 5, wobei der Antikörper ein monoclonaler Antikörper ist.

8. Verfahren nach einem der Ansprüche 1 bis 7, wobei der Krebs Lungenkrebs, Leberkrebs oder Bauchspeicheldrüsenkrebs ist.

9. In vitro-Krebsdiagnoseverfahren, umfassend den Schritt des Nachweisens eines Polynucleotids in einer von einem Individuum gewonnenen Probe, wobei das Polynucleotid:
a) die Polynucleotidsequenz wie in SEQ ID NO:2 dargestellt oder ihr RNA-Äquivalent umfasst oder daraus besteht;
b) eine Sequenz aufweist, die zu der Sequenz von a) komplementär ist;
c) eine Polynucleotidsequenz aufweist, die das Polypeptid wie in SEQ ID NO:66 dargestellt codiert;
d) 70% Sequenzidentität oder mehr zu der in SEQ ID NO:2 gezeigten Sequenz aufweist; oder
e) ein Fragment der Polynucleotidsequenz wie in SEQ ID NO:2 dargestellt ist.

## Revendications

1. Méthode de diagnostic du cancer *in vitro* comprenant l'étape consistant à détecter dans un échantillon prélevé sur un sujet une protéine ayant la séquence d'acides aminés présentée dans SEQ ID N°: 66 contenue dans l'échantillon.

2. Méthode selon la revendication 1, dans laquelle l'échantillon prélevé sur le sujet est le sang, le sérum ou le plasma.

3. Méthode selon la revendication 1 ou 2, dans laquelle un domaine extracellulaire d'une protéine ayant la séquence d'acides aminés exposée dans SEQ ID N°: 66 est détecté.

4. Méthode selon la revendication 1 ou 2, dans laquelle une forme sécrétoire de la protéine ayant la séquence d'acides aminés présentée dans SEQ ID N°: 66 est détectée.

5. Méthode selon l'une quelconque des revendications 1 à 4, dans laquelle la protéine est détectée par un anticorps reconnaissant ladite protéine.

6. Méthode selon la revendication 5, dans laquelle l'anticorps est un anticorps polyclonal.

7. Méthode selon la revendication 5, dans laquelle l'anticorps est un anticorps monoclonal.

8. Méthode selon l'une quelconque des revendications 1 à 7, dans laquelle le cancer est le cancer du poumon, le cancer du foie ou le cancer du pancréas.

9. Méthode de diagnostic du cancer *in vitro* comprenant l'étape consistant à détecter dans un échantillon prélevé sur un sujet un polynucléotide, dans laquelle le polynucléotide :
a) comprend ou est constitué de la séquence de polynucléotides présentée dans SEQ ID N°: 2 ou son équivalent ARN ;
b) a une séquence qui est complémentaire de la séquence en a) ;
c) a une séquence de polynucléotides qui code pour la séquence polypeptidique présentée dans SEQ ID N°: 66;
d) présente une identité de séquence de 70 % ou plus avec la séquence illustrée dans SEQ ID N°: 2 ; ou
e) est un fragment de la séquence de polynucléotides présentée dans SEQ ID N°: 2.


<110> ABURATANI, Hiroyuki; PERSEUS PROTEOMICS INC.; CHUGAI SEIYAKU KABUSHIKI KAISHA
<120> Genes Highly Expressed in Cancers
<130> PCG9001W0
<150> JP 2003-290704
   <151> 2003-08-08
<160> 8
<170> PatentIn version 3.1
<210> 2
   <211> 1952
   <212> DNA
   <213> homo sapiens
<400> 2
   cttccgcgga agggaagagt cccgcagtcg gaggcggccg gctgggcgtg cgctcgctcc     60
   ccgaagccgg ggctgggccg gagccgggcg agggctggga gctgggccgg gtccggggac    120
   agcgggcgag gggcagctgc cggagccggg cagccaggcc gctcagggca ggggacagct    180
   ggcgccggtt ctgcggtctc cggggcccag atgtgaggcg gcggcgcccc cggcccgaga    240
   gcgcacgatg gggGccccgc tcgccgtagc gctgggcgcc ctccactacc tggcactttt    300
   cctgcaactc ggcggcgcca cgcggcccgc cggccacgcg ccctgggaca accacgtctc    360
   cggccacgcc ctgttcacag agacacccca tgacatgaca gcacggacgg gcgaggacgt    420
   ggagatggcc tgctccttcc gcggcagcgg ctccccctcc tactcgctgg agatccagtg    480
   gtggtatgta cggagccacc gggactggac cgacaagcag gcgtgggcct cgaaccagct    540
   aaaagcatct cagcaggaag acgcagggaa ggaggcaacc aaaataagtg tggtcaaggt    600
   ggtgggcagc aacatctccc acaagctgcg cctgtcccgg gtgaagccca cggacgaagg    660
   cacctacgag tgccgcgtca tcgacttcag cgacggcaag gcccggcacc acaaggtcaa    720
   ggcctacctg cgggtgcagc caggggagaa ctccgtcctg catctgcccg aagcccctcc    780
   cgccgcgccc gccccgccgc cccccaagcc aggcaaggag ctgaggaagc gctcggtgga    840
   ccaggaggcc tgcagcctct agactgatgc cctgcccccc gcccatccgc ccccacgctg    900
   tacagagtgc atgaggagcc gccggaccac cggggaccga ctgcctgcgt ccagccgcgc    960
   cccatccccg aggccgcctg tggccaccat gtcggccctc tttccaccac cccttgctca   1020
   gcatgtaagc cccacccacc cctgcccttt cagacccctg cggtgacctg gctcggagaa 1080
   ggtggccctg ggcaccaagg ggccaaccgc cctgaacact ggggcaggga ccatgctggg 1140
   gcccggggcc acccccttcc tgtcaccagc ttctgtggag tccagtgttt tgctttgctt 1200
   gcttgtcccc catcctgtcc tgagccgggg ccccccagcc tcgcctccct cctcctacca 1260
   tccctcactt ggacctgggg gtgtggacag tgacccctcc ctgaatatgg acttgaatct 1320
   tctgagcaga actagggcct ctcccctggt gaagacccag ggaacccagg agggcccttc 1380
   tggggcagtg gctctgcagg gtcactcatg gaggcctagg ggaacagcga gatgccccac 1440
   cacctcctgg cgagtccttc ctgttcagct ccctgtgcga ccctccaggg atgcagggga 1500
   tccaggattc tctgccctgt cacacggcga gtcagaaggg aggggccttt ccctcggacc 1560
   catggcccca ggcagagttt tgcaccagca ggaccccttt gagggccttc aaggctctcc 1620
   caggagtccc cctctgccgg ccccccaatg ccccagctcc ctcttgggtc ctgtgccaag 1680
   tccgccccag ggcctggggc tgttgggagc caagggcccc ctggtactca gttccctcac    1740
   gattcccgat cacgggcaca cctgccccct ggttatttgt aaatatttct attggaccca    1800
   attctcctcg gaattggctg gcacctctgg ttgccacagc tcagtgatga cgtgggggag 1860
   gtgggagagg ccgagggctt tgcctagggg tgggttgccc tgtatacatg atccagtctg 1920
   tgactaccag ccaacctgaa taaagcggtt tt 1952
<210> 66
   <211> 204
   <212> PRT
   <213> homo sapiens
<400> 66
   Met Gly Ala Pro Leu Ala Val Ala Leu Gly Ala Leu His Tyr Leu Ala
   1 5 10 15
   Leu Phe Leu Gln Leu Gly Gly Ala Thr Arg Pro Ala Gly His Ala Pro
   20 25 30
   Trp Asp Asn His Val Ser Gly His Ala Leu Phe Thr Glu Thr Pro His
   35 40 45
   Asp Met Thr Ala Arg Thr Gly Glu Asp Val Glu Met Ala Cys Ser Phe
   50 55 60
   Arg Gly Ser Gly Ser Pro Ser Tyr Ser Leu Glu Ile Gln Trp Trp Tyr
   65 70 75 80
   Val Arg Ser His Arg Asp Trp Thr Asp Lys Gln Ala Trp Ala Ser Asn
   85 90 95
   Gln Leu Lys Ala Ser Gln Gln Glu Asp Ala Gly Lys Glu Ala Thr Lys
   100 105 110
   Ile Ser Val Val Lys Val Val Gly Ser Asn Ile Ser His Lys Leu Arg
   115 120 125
   Leu Ser Arg Val Lys Pro Thr Asp Glu Gly Thr Tyr Glu Cys Arg Val
   130 135 140
   Ile Asp Phe Ser Asp Gly Lys Ala Arg His His Lys Val Lys Ala Tyr
   145 150 155 160
   Leu Arg Val Gln Pro Gly Glu Asn Ser Val Leu His Leu Pro Glu Ala
   165 170 175
   Pro Pro Ala Ala Pro Ala Pro Pro Pro Pro Lys Pro Gly Lys Glu Leu
   180 185 190
   Arg Lys Arg Ser Val Asp Gln Glu Ala Cys Ser Leu
   195 200
<210> 252
   <211> 25
   <212> DNA
   <213> homo sapiens
<400> 252
   agaaggagat cactgccctg gcacc 25
<210> 253
   <211> 24
   <212> DNA
   <213> homo sapiens
<400> 253
   cctgcttgct gatccacatc tgct 24
<210> 269
   <211> 30
   <212> DNA
   <213> homo sapiens
<400> 269
   cgaattcatg ggggccccgc tcgccgtagc 30
<210> 270
   <211> 30
   <212> DNA
   <213> homo sapiens
<400> 270
   cctcgaggag gctgcaggcc tcctggtcca 30
<210> 271
   <211> 21
   <212> DNA
   <213> homo sapiens
<400> 271
   ttcgaacgcc agcacatgga c 21
<210> 272
   <211> 21
   <212> DNA
   <213> homo sapiens
<400> 272
   gctagttatt gctcagcggt g 21